(19) Europäisches Patentamt
European Patent Office
Office européen des brevets

(11) Publication number: **0 049 120**
**B1**

(12) # EUROPEAN PATENT SPECIFICATION

(45) Date of publication of patent specification: **16.01.85**

(21) Application number: **81304424.5**

(22) Date of filing: **24.09.81**

(51) Int. Cl.⁴: **C 07 C 43/164,**
C 07 C 43/166, C 07 C 43/184,
C 07 C 41/06, A 61 K 7/46,
A 01 N 31/00, A 24 B 15/18,
C 11 D 3/50, C 11 D 9/44,
C 11 B 9/00

(54) **Phenethylether derivatives, process for preparing same and uses thereof in combatting tobacco beetles and in augmenting or enhancing the aroma of perfumes, colognes and perfumed articles.**

(30) Priority: **30.09.80 US 192238**
**19.02.81 US 235844**
**27.02.81 US 238750**

(43) Date of publication of application:
**07.04.82 Bulletin 82/14**

(45) Publication of the grant of the patent:
**16.01.85 Bulletin 85/03**

(84) Designated Contracting States:
**CH DE FR GB LI NL**

(56) References cited:

**Toxic Substances Control Act, Chemical Substance Inventory, Vol. II, May 1979, page 163**

(73) Proprietor: **INTERNATIONAL FLAVORS & FRAGRANCES INC.**
**521 West 57th Street**
**New York New York 10019 (US)**

(72) Inventor: **Kiwala, Jacob**
**938 East 28th Street**
**Brooklyn New York 11219 (US)**
Inventor: **Tokarzewski, Richard J.**
**251 Atlantic Street**
**Keyport New Jersey 07735 (US)**
Inventor: **Schmitt, Frederick L.**
**49 McCampbell Road**
**Holmdel New Jersey 07735 (US)**
Inventor: **Sprecker, Mark A.**
**6 Sandpiper Lane**
**Sea Bright New Jersey 07760 (US)**

(74) Representative: **Brown, John David et al**
**FORRESTER & BOEHMERT Widenmayerstrasse 4/I**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 049 120 B1

**Description**

The present invention relates to phenethylether derivatives, process for preparing same and uses thereof in combatting tobacco beetles and in augmenting or enhancing the aroma of perfumes, colognes and perfumed articles.

According to the first aspect of the present invention there is provided a phenalkylether derivative characterised in that it is at least one phenethylether having the structure:

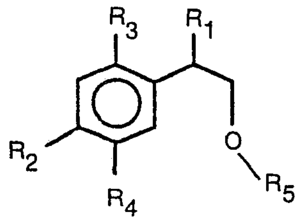

wherein $R_5$ represents $C_6$ secondary alkyl; $C_4$ alkenyl or cyclohexyl or substituted cyclohexyl defined according to the moiety:

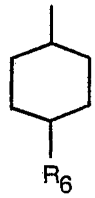

wherein $R_1$, $R_2$, $R_3$, $R_4$ and $R_6$ are the same or different and each represents methyl or hydrogen with the proviso that when one of $R_2$, $R_3$ and $R_4$ is methyl, the other two of $R_2$, $R_3$ and $R_4$ are hydrogen.

According to the second aspect of the present invention there is provided a process for augmenting or enhancing the aroma of a consumable material selected from the group consisting of pheromones, insecticides, perfumes, solid or liquid anionic, cationic, nonionic or zwitterionic detergents, fabric softener compositions, dryer-added fabric softener articles, cosmetic powders, and colognes comprising the step of adding to an appropriate base an aroma-augmenting or enhancing quantity of at least one compound in accordance with the first aspect of the present invention.

According to a third aspect of the present invention there is provided a method of attracting and destroying *Lasioderma serricorne (F.)* comprising applying to an area contaminated with said *Lasioderma serricorne (F.)* at least one compound in accordance with the first aspect of the present invention modified in that $R_5$ may represent $C_3$ secondary alkyl, said attractant being applied to said area in an amount sufficient to attract said *Lasioderma serricorne (F.).*

According to a fourth aspect of the present invention there is provided a perfume composition comprising an aroma augmenting quantity of at least one cyclohexyl phenethylether derivative defined according to the structure:

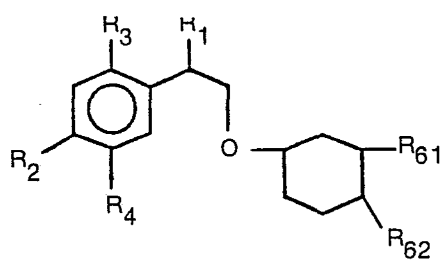

wherein $R_1$, $R_2$, $R_3$ and $R_4$ are the same or different and each represents methyl or hydrogen with the proviso that when one of $R_2$, $R_3$ and $R_4$ is methyl, the other of $R_2$, $R_3$ and $R_4$ is hydrogen and wherein $R_{61}$ and $R_{62}$ represent hydrogen or methyl with at least one of $R_{61}$ and $R_{62}$ being hydrogen; and intimately admixed therewith at least one adjuvant compatible with said cyclohexyl phenethylether derivative from an organoleptic standpoint which is selected from the group consisting of alcohols, aldehydes, ketones, nitriles, ethers other than said cyclohexyl phenethylether derivative, lactones, natural essential oils, synthetic essential oils and hydrocarbons, as well as a cologne comprising said cyclohexyl phenethylether intimately admixed with ethanol and water.

The invention also provides specific uses of specific aforesaid phenalkylether derivatives.

In this regard, attention is directed to Toxic Substances Control Act (TSCA), Chemical Substance Inventory; Volume II, May 1979 at page 163 where there is a reference to benzene, (2-(1-methylethoxy)ethyl) — (68039-47-4) $C_{11}H_{16}O$.

The present invention proposes the use of phenethylether derivatives defined according to the structure:

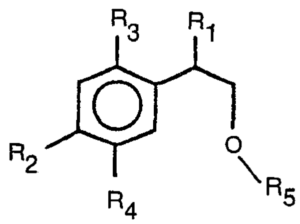

wherein $R_5$ represents $C_3$ or $C_6$ secondary alkyl; $C_4$ alkenyl or cyclohexyl or substituted cyclohexyl defined according to the moiety:

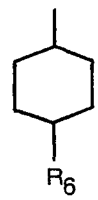

wherein $R_6$ represents hydrogen or methyl; with the proviso that when one of $R_2$, $R_3$ and $R_4$ is methyl, the other two of $R_2$, $R_3$ and $R_4$ are hydrogen for combatting beetles of the order *Lasioderma serricorne (F.)* in such a manner that one or more of said cyclohexyl phenethylether derivatives not only act as a pheromone or ectohormone but also act as aroma augmenting or enhancing agents and, in addition, act as insecticides. Notwithstanding the pheromone and insecticide properties of said cyclohexyl phenethylether derivatives, the instant invention also provides cyclohexyl phenethylether derivatives (in which $R_5$ does not represent $C_3$ secondary alkyl) as fragrances capable of augmenting or enhancing the fragrance of perfume compositions, colognes and perfumed articles (e.g., solid or liquid anionic, cationic, nonionic or zwitterionic detergents, fabric softeners, dryer-added fabric softener articles, hair conditioners, deodorants and cosmetic powders). The present invention also proposes a new process for preparing cyclohexyl phenethylether derivatives according to the reaction:

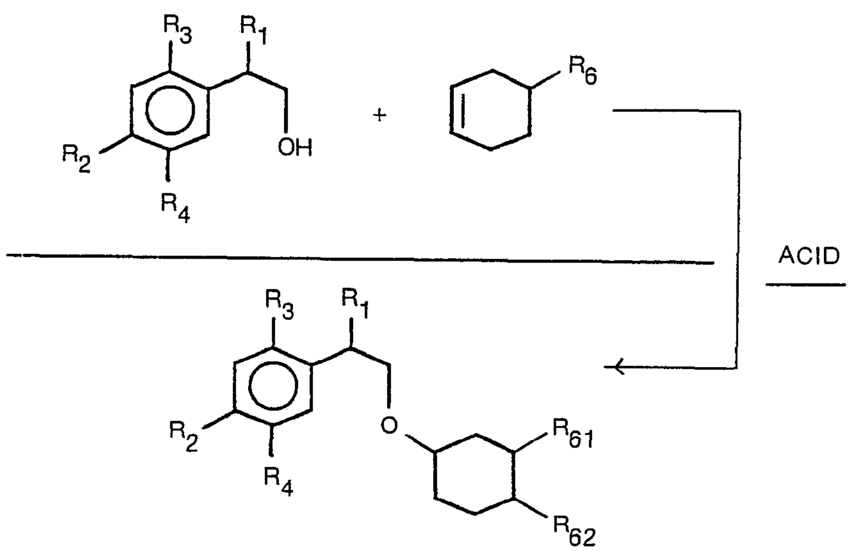


wherein $R_1$, $R_2$, $R_3$, $R_4$ and $R_6$ are the same or different hydrogen or methyl; wherein one of $R_{61}$ or $R_{62}$ is $R_6$ and the other of $R_{61}$ or $R_{62}$ is hydrogen; with the proviso that when one of $R_2$, $R_3$ and $R_4$ is methyl the other two of $R_2$, $R_3$ and $R_4$ are hydrogen which reaction is carried out under specific conditions as indicated infra.

The phenethylether derivatives (in which $R_5$ does not represent $C_3$ secondary alkyl) of our invention are capable of augmenting or enhancing the dry green, hyacinth, rose, fruity and galbanum aromas of perfumes, perfumed articles and colognes of our invention. Of course, as part of the perfumed articles of our invention, there are the "perfumed insecticide-pheromone" compositions of our invention.

The phenethylether derivatives of our invention (in which $R_5$ does not represent $C_3$ secondary alkyl) are capable of augmenting or enhancing the sweet, honey-like, rich, slightly fruity, hay, tobacco-like aromas and tastes both prior to and on smoking and Virginia tobacco-like aroma on smoking in smoking tobaccos and in cigarette articles containing such smoking tobaccos.

The destruction of the *Lasioderma serricorne (F.)* insects can be achieved by distributing the phenethylether pheromonal attractants in the contaminated area at separate individual places, namely, by means of catch trees. These are impregnated with the attractants which may, if desired, act as insecticides too; or one or more of the phenethylether derivatives may be augmented by one or more additional insecticides whereupon the catch trees are sprayed with another insecticide either before or after the insects have gathered at the catch tree (whatever insects are still alive after contact with said phenethylether derivatives). Instead of using one or more phenethylether derivatives taken alone or taken together with another insecticide, one may also use a chemical sterilizing compound. Further, the catch tree may be treated with other chemicals which can be burned. Another possible method for destroying insects with one or more of the phenethylether derivatives of our invention makes use of the disturbance or perturbance theory. Instead of physically destroying the insects with either high concentrations of one or more of the phenethylether derivatives of our invention or by using one or more of the phenethylethers of our invention followed by additional insecticide, it is also possible to combine one or more of the phenethylether derivatives of our invention physically with one or more additional stronger insecticides before using. Thus, it is possible now to spray a combination of one or more phenethylethers which have pleasant aromas in combination with insecticides whose original aroma(s) are covered using one or more of the phenethylether(s) in certain centrally located areas or in the form of rows in the contaminated area. Furthermore, one or more of the phenethylethers can be mixed with the usual solid or liquid carriers or with biocides such as stronger insecticides, pesticides or herbicides. The mixture may contain surface active agents to obtain a better distribution or adherence to the plants.

The cyclohexyl phenethylether derivatives of our invention may be prepared according to a conventional method by reacting cyclohexanol with beta phenylethyl alcohol derivatives such as beta phenylethyl alcohol in the presence of an acid such as sulfuric acid according to the reaction:

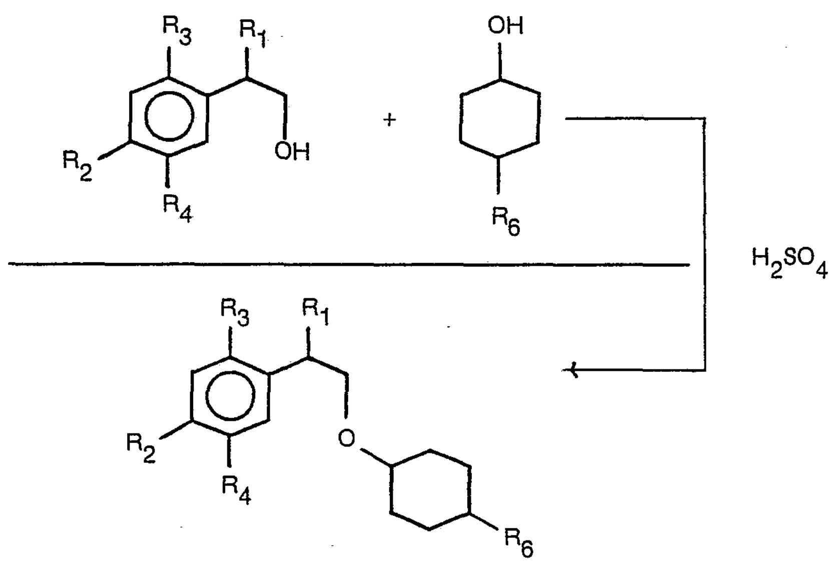


wherein $R_1$, $R_2$, $R_3$, $R_4$ and $R_6$ represent the same or different hydrogen or methyl with the proviso that when one of $R_2$, $R_3$ and $R_4$ is methyl, the other two of $R_2$, $R_3$ and $R_4$ are hydrogen. Thus, for example, the reaction:

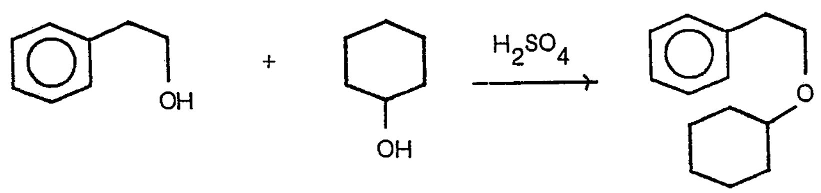


is typical of such a reaction. Alternatively, an alkali metal phenethyl alcoholate derivative may be reacted with a cyclohexyl halide such as cyclohexyl bromide or cyclohexyl chloride or an alkali metal cyclohexyl alcoholate may be reacted with beta phenylethyl chloride or beta phenylethyl bromide by means of a "Williamson" synthesis, conventional in the organic chemistry art.

Furthermore, more efficiently and of lower cost is the carrying out of a process of reacting a beta phenylethyl alcohol derivative with cyclohexene in the presence of specific acid catalysts under specific conditions according to the reaction:

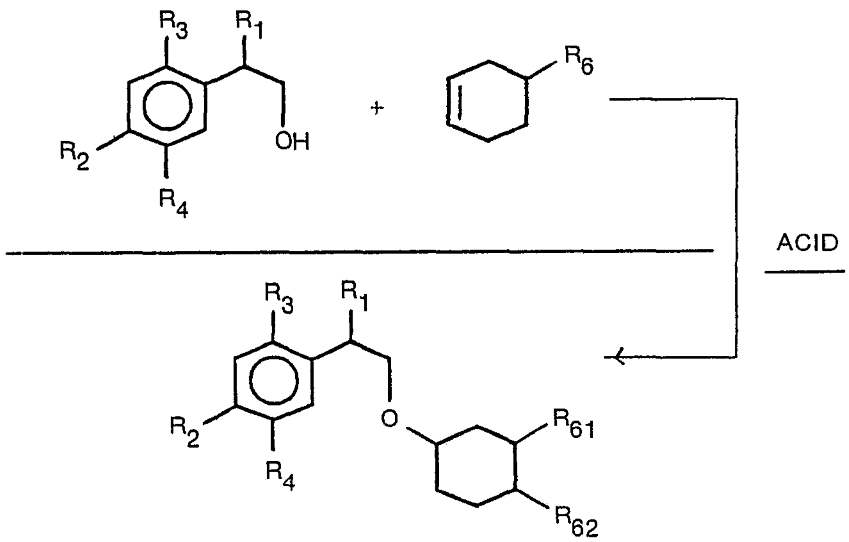


wherein $R_1$, $R_2$, $R_3$, $R_4$ and $R_6$ are the same or different and each represents methyl or hydrogen with the proviso that when one of $R_2$, $R_3$ and $R_4$ is methyl and the other two of $R_2$, $R_3$ and $R_4$ are hydrogen; and wherein one of $R_{61}$ or $R_{62}$ is $R_6$ and the other is hydrogen.

The temperature of reaction may vary from 90°C up to about 180°C at from 1 atmosphere pressure up to 100 atmospheres pressure. Higher temperatures of reaction require higher pressures of reaction.

The mole ratio of phenethyl alcohol derivative to cyclohexene may vary from 1:1 up to about 1:2.

Various acid catalysts can be used including mineral acids such as sulfuric acid or methane sulfonic acid or para-toluene sulfonic acid; or the acid catalyst may be a solid acid catalyst such as a sulfonated copolymer of styrene and divinyl benzene (e.g., "Amberlyst® 15" manufactured by the Rohm & Haas Corporation of Philadelphia, Pennsylvania) or an acid clay such as an activated clay adsorbent such as Filtrol® 105 having the following properties:

Particle size analysis

| | |
|---|---|
| By Roller (10 liters/min. air rate) | |
| 0—5 Microns, Wt. % | 8 |
| 0—20 Microns, wt. % | 43 |
| By Taylor Standard Sieve | |
| Through 100 Mesh, wt.% | 100 |
| Through 200 Mesh, wt.% | 95 |
| Through 325 Mesh, wt.% | 78 |
| Apparent Bulk density, lb/cu. ft. | 42 |
| Free moisture, wt.% | 15 |
| Free and combined moisture, wt.% (loss at 1700°F) | 21 |
| Surface area (BET Method), Sq.M/gm | 300 |
| Acidity, phenolphthalein, mg. KOH/gm | 4.8 |
| Filter rate, cc/min. | 38 |
| Oil retention, wt.% | 35 |

The weight ratio of acid catalyst to phenylethyl alcohol derivative may vary from about 2 wt.% up to about 20 wt.%.

The time of reaction is primarily a function of four variables:

1. temperature of reaction
2. conversion desired
3. particular acid catalyst utilized and
4. concentration of acid catalyst in the reaction mass.

In general, higher temperatures of reaction give rise to a shorter required time of reaction, but too high a temperature of reaction (e.g., greater than about 180°C) and/or too long a time of reaction causes a diminution of conversion due to product decomposition. In general, higher concentration of acid catalyst in the reaction mass gives rise to shorter time periods of reaction for a given conversion to the desired cyclohexyl phenethylether derivative. Thus, in general, a time of reaction may vary from about 1 hour up to about 48 hours.

Notwithstanding the pheromonal and insecticidal activity of the cyclohexyl phenethylether derivatives of our invention, the cyclohexyl phenethylethers of our invention can be used to contribute long lasting dry green, hyacinth, rose, fruity and galbanum aromas which are unexpectedly full and rich for a very long period of time to perfumes, perfumed articles and colognes. As olfactory agents, the cyclohexyl phenethylether derivatives of our invention can be formulated into or used as components of a "perfume composition" or can be used as components of a "perfumed article" or the perfume composition may be added to "perfumed articles".

Examples of the compounds prepared according to the process of our invention, the phenylethyl alcohol derivatives reactant for producing such compound and the olfactory properties of such compounds are set forth in Table I below:

TABLE I

| Product Structure | Reactant | Olfactory Properties |
|---|---|---|
| 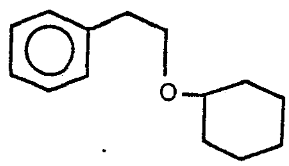 | 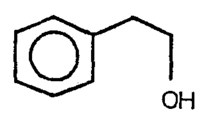 | A long lasting dry green, hyacinth, rose and galbanum aroma. |
| 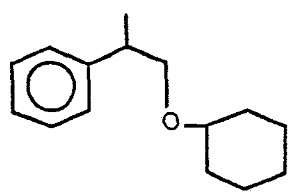 | 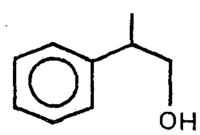 | A floral, green, galbanum-like aroma. |
| 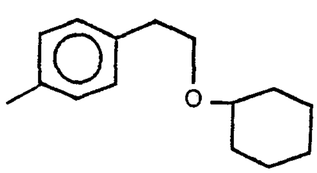 | 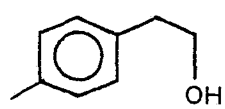 | An interesting low-keyed burnt fruity aroma. |

The alkyl and/or alkenyl phenethylethers of our invention may be prepared by reacting β-phenylethyl alcohol with an appropriate $C_3$ or $C_6$ lower secondary alkanol or a $C_4$ alkenol in the presence of an acid such as sulfuric acid according to the reaction:

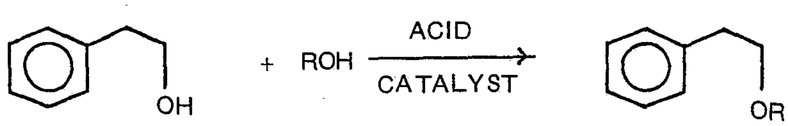


Alternatively, sodium phenethyl alcoholate may be reacted with a $C_3$ or $C_6$ secondary alkyl chloride or a $C_4$ alkenyl chloride or a sodium alkyl alcoholate or a sodium alkenyl alcoholate may be

reacted with β-phenylethyl chloride or β-phenylethyl bromide by means of a "Williamson" synthesis, conventional in the organic chemistry art.

A novel aspect of our invention lies in the preparation of the isopropyl phenethylether of our invention having the structure:

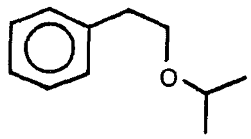

When isopropyl alcohol is reacted with phenylethyl alcohol, not all of the phenylethyl alcohol will react and thus, a mixture of phenylethyl alcohol having the structure:

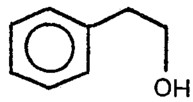

and isopropyl phenethylether having the structure:

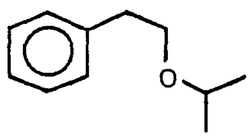

is formed.

Another aspect of our invention involves the production of the isopropyl phenethylether. In reacting the phenylethyl alcohol having the structure:

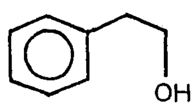

with the isopropanol in the presence of an acid catalyst, for example, sulfuric acid, a mixture of two compounds is formed: the isopropyl phenethylether and unreacted phenylethyl alcohol. It is impossible to separate this mixture whereby the isopropyl phenethylether is produced in pure form and, accordingly, we have found that the mixture of isopropyl phenethylether and phenylethyl alcohol may be easily reacted with propionic anhydride to form a mixture of isopropyl phenethylether having the structure:

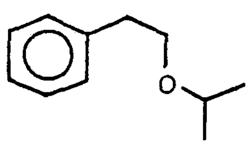

and the phenylethyl alcohol propionate having the structure:

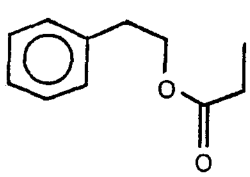

The resulting mixture of isopropyl phenethylether and phenylethyl alcohol propionate may then be easily separated by fractional separation to yield substantially pure isopropyl phenethylether in high yields. In forming the mixture of isopropyl phenethylether and phenylethyl alcohol propionate from the mixture of isopropyl phenethylether and phenylethyl alcohol with propionic anhydride, it is preferred to carry out the reaction at reflux conditions in the presence of an easily separatable solvent which is inert to the reaction mass such as toluene. The foregoing reaction sequence is illustrated as follows:

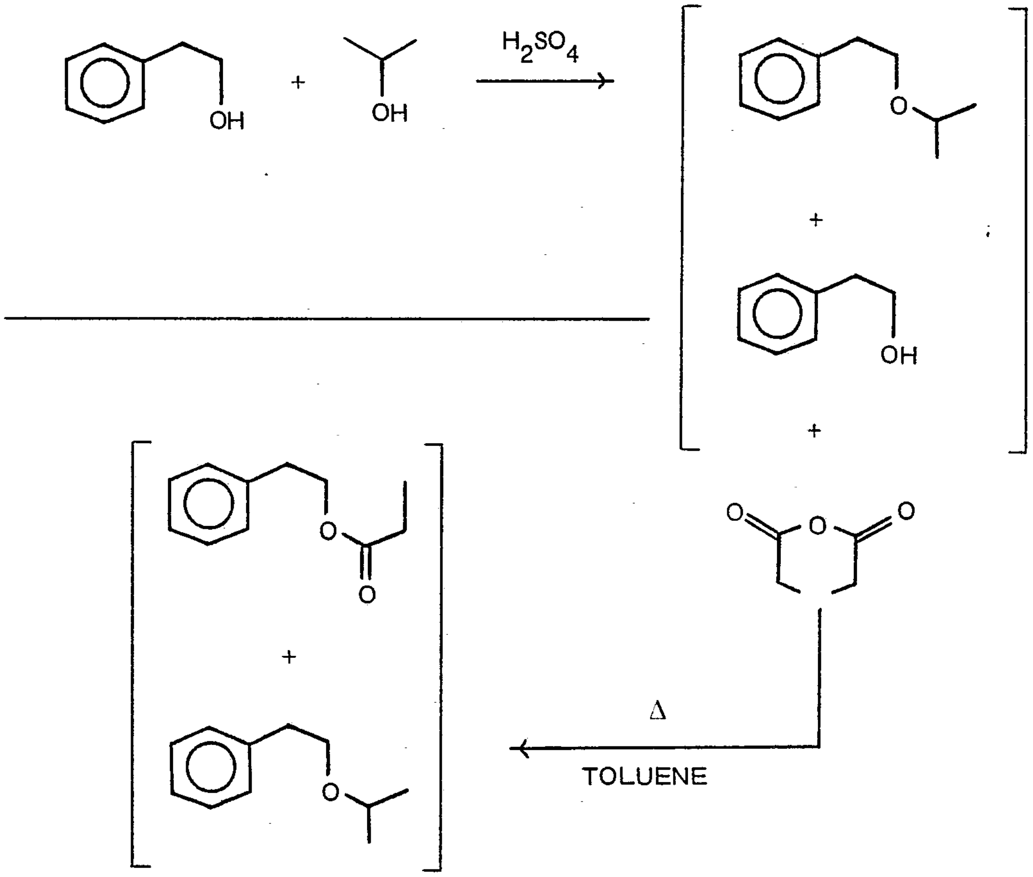


and is more specifically illustrated in Example II, infra.

Notwithstanding the pheromonal and insecticidal activity of the alkyl and/or alkenyl phenethylethers of our invention, the alkyl and/or alkenyl phenethylethers of our invention can be used to contribute long-lasting green, fruity, floral, hyacinth-like rosy, rose-hyacinth-like; galbanum-like, cassis-like and narcissus-like aromas with hyacinth/honey aromas on dry-out and with peppery and mushroomy undertones which are unexpectedly full and rich for very long periods of time, to perfumes, perfumed articles and colognes.

As olfactory agents, the alkyl and/or alkenyl phenethylethers of our invention can be formulated into or used as components of a "perfume composition" or can be used as components of a "perfumed article" or the perfume composition may be added to "perfumed articles".

The term "perfume composition" is used herein to mean a mixture of organic compounds including, for example, alcohols, aldehydes, ketones, nitriles, ethers in addition to and other than the alkyl and alkenyl phenethylethers of our invention, lactones, natural essential oils, synthetic essential oils and frequently hydrocarbons which are admixed so that the combined odors of the individual components produce a pleasant or desired fragrance. Such perfume compositions usually contain (a) the main note or the "bouquet" or foundation stone of the composition; (b) modifiers which round off and accompany the main note; (c) fixatives which include odorous substances which lend a particular note to the perfume throughout all states of evaporation, and substances which retard evaporation; and (d) top notes which are usually low-boiling, fresh-smelling materials.

In perfume compositions, the individual component will contribute its particular olfactory characteristics, but the overall effect of the perfume composition will be the sum of the effects of each of the ingredients. Thus, the cyclohexyl and/or alkyl and/or alkenyl phenethylethers of our invention can be used to alter the aroma characteristics of a perfume composition, for example, by highlighting or moderating the olfactory reaction contributed by another ingredient in the composition. Furthermore, the alkyl and/or alkenyl phenethylethers of our invention can be used in combination with the cyclohexyl phenethylethers. The amount of alkyl and/or alkenyl phenethylethers that may be used in conjunction with the cyclohexyl phenethylethers may vary from about 1:99 up to about 99:1 whereby very interesting hyacinth-like, rosy, rose-hyacinth-like, galbanum-like aromas with cassis-like, narcissus-like peppery and mushroomy undertones are achieved.

The amount of cyclohexyl and/or alkyl and/or alkenyl phenethylether of our invention which will be effective in perfume compositions depends upon many factors, including the other ingredients, their amounts and the effects which are desired. It has been found that perfume compositions containing as

little as 0.05% of the alkyl and/or alkenyl phenethylether of our invention taken alone or in combination with the cyclohexyl phenethylether, or even less can be used to impart long lasting, interesting, very strong, green, fruity, floral, hyacinth-like, rosy, rose-hyacinth-like, galbanum-like, cassis-like and narcissus aromas to soaps, liquid and solid cationic, nonionic, anionic and zwitterionic detergents, cosmetic powders, liquid and solid fabric softeners, dryer-added fabric softener articles, optical brightener compositions and other products. The amount employed can range up to 50% or more and will depend upon considerations of cost, nature of the end product and the effect desired on the finished product and particular fragrance sought.

The alkyl and/or alkenyl phenethylethers of our invention can be used alone or in a perfume composition as an olfactory component in detergents and soaps, space odorants and deodorants; perfumes; colognes, toilet waters; bath salts; hair preparations such as lacquers, brilliantines, pomades and shampoos; cosmetic preparations such as creams, deodorants, hand lotions and sun screens; powders such as talcs, dusting powders, face powder and the like. When used as an olfactory component of a perfumed article, as little as 0.01% of the alkyl and/or alkenyl phenethylether taken alone or in combination with cyclohexyl phenethylether will suffice to impart an interesting, long-lasting, green, fruity, floral, hyacinth-like, rosy, rose-hyacinth-like, galbanum-like, cassis and narcissus-like aroma. Generally no more than 0.5% is required in the perfumed article.

It has also been found that perfume compositions containing as little as 0.05% of one or a mixture of the cyclohexyl phenethylether derivatives of our invention or even less can be used to impart long lasting, interesting, very strong, dry green, hyacinth, rose, fruity and galbanum aromas to soaps, liquid and solid cationic, non-ionic, anionic and zwitterionic detergents, cosmetic powders, liquid and solid fabric softeners, dryer-added fabric softener articles, optical brightener compositions and other products. The amount employed can range up to 50% or more and will depend upon considerations of cost, nature of the end product and the effect desired on the finished product and particular fragrance sought.

One or more of the cyclohexyl phenethylether derivatives of our invention can be used alone or in combination with one another or in a perfume composition as an olfactory component in detergents and soaps, space odorants and deodorants; perfumes, colognes, toilet water; bath salts; hair preparations such as lacquers, brilliantines, pomades and shampoos; cosmetic preparations such as creams, deodorants, hand lotions and sun screens; powders such as talcs, dusting powders, face powder and the like. When used as an olfactory component of a perfumed article, as little as 0.01% of one or a mixture of cyclohexyl phenethylether derivatives will suffice to impart an interesting long-lasting dry green, hyacinth, rose, fruity and/or galbanum aroma. Generally no more than 0.5% is required in the perfumed article.

In addition, the perfume composition can contain a vehicle or carrier for the alkyl and/or alkenyl phenethylether alone or in combination with the cyclohexyl phenethylether in combination with still other ingredients. The vehicle can be a liquid such as an alcohol such as ethanol, a glycol such as propylene glycol or the like. The carrier can be an adsorbent solid such as a gum or components for encapsulating the composition such as gelatin which can be used to form a capsule wall surrounding the perfume oil by means of coacervation.

In addition, the perfume composition can contain a vehicle or carrier for the phenethylether derivatives taken alone or taken in combination with other ingredients. The vehicle can be a liquid such as an alcohol such as ethanol, a glycol such as propylene glycol, or the like. The carrier can be an absorbent solid such as a gum or components for encapsulating the composition such as gelatin which can be used to form a capsule wall surrounding the perfume oil by means of coacervation.

This invention further provides improved tobacco additives and methods whereby various desirable sweet, honey-like, rich, slightly fruity, Virginia tobacco-like and hay tobacco-like flavor and aroma characteristics may be imparted to smoking tobacco products and may be readily varied and controlled to produce the desired uniform flavoring characteristics.

In carrying out this aspect of our invention, we add to smoking tobacco materials or a suitable substitute therefor (e.g., dried lettuce leaves) an aroma and flavor additive containing as an active ingredient one or more of the phenethylether derivatives of our invention.

In addition to the phenethylether derivatives of our invention, other flavoring and aroma additives may be added to the smoking tobacco material or substitute therefor either separately or in mixture with the phenethylether derivatives as follows:

I. Synthetic materials:
Beta-ethyl-cinnamaldehyde;
Eugenol;
Dipentene;
β-damascone;
β-damascenone;
Maltol;
Ethyl maltol;
Delta undecalactone;

Delta decalactone;
Benzaldehyde;
Amyl acetate;
Ethyl butyrate;
Ethyl valerate;
Ethyl acetate;
2-hexenol-1,2-methyl-5-isopropyl-1,3-nonadiene-8-one;
2-methyl-5-isopropyl acetophenone;
2-hydroxy-2,5,5,8a-tetramethyl-1-(2-hydroxyethyl)-decahydronaphthalone;
Dodecahydro-3a,6,6,9a-tetramethyl naphtho-(2,1-b)-furan;
4-hydroxy hexanoic acid, gamma lactone;
Polyisoprenoid hydrocarbons defined in Example V of U.S. Patent No. 3,589,372, issued on June 29, 1971.

II. Natural oils
Celery seed oil;
Coffee extract;
Bergamot oil;
Cocoa extract;
Nutmeg oil; and
Origanum oil.

An aroma and flavoring concentrate containing one or more of the phenethylether derivatives of our invention and if desired, one or more of the above indicated additional flavoring additives may be added to smoking tobacco material, to the filter or to the leaf or paper wrapper. The smoking tobacco material may be shredded, cured, cased and blended tobacco material or reconstituted tobacco material or tobacco substitutes (e.g., lettuce leaves) or mixtures thereof. The proportions of flavoring additives may be varied in accordance with taste but insofar as the augmentation or the enhancement or the imparting of the sweet, honey-like, fruity, Virginia tobacco-like notes are concerned, we have found that satisfactory results are obtained if the proportion by weight of the sum total of phenethylether derivatives to smoking tobacco material is between 250 ppm and 1,500 ppm (0.025%—0.15%) of the active ingredients to the smoking tobacco material. We have further found that satisfactory results are obtained if the proportion by weight of the sum total of phenethylether derivatives used to flavoring material is between 2,500 and 10,000 ppm (0.25%—1.0%).

Any convenient method for incorporating the phenethylether derivatives in the tobacco product may be employed. Thus, the phenethylether derivatives taken alone or along with other flavoring additives may be dissolved in a suitable solvent such as ethanol, n-pentane, diethyl ether and/or other volatile organic solvents and the resulting solution may be either sprayed on the cured, cased and blended tobacco material or the tobacco material may be dipped into such solution. Under certain circumstances, a solution of the phenethylether derivatives taken alone or taken further together with other flavoring additives as set forth above may be applied by means of a suitable applicator such as a brush or roller on the paper or leaf wrapper for the smoking product, or it may be applied to the filter by either spraying or dipping or coating.

Furthermore, it will be apparent that only a portion of the tobacco or substitute therefor need be treated and the thus treated tobacco may be blended with other tobaccos before the ultimate tobacco product is formed. In such cases, the tobacco treated may have the phenethylether derivative in excess of the amounts or concentrations above indicated so that when blended with other tobaccos, the final product will have the percentage within the indicated range.

In accordance with one specific example of our invention, an aged, cured and shredded domestic burley tobacco is spread with a 20% ethyl alcohol solution of phenethyl cyclohexylether in an amount to provide a tobacco composition containing 800 ppm by weight of cyclohexyl phenethylether on a dry basis. Thereafter, the alcohol is removed by evaporation and the tobacco is manufactured into cigarettes by the usual techniques. The cigarette, when treated as indicated has a desired and pleasing aroma which is detectable in the main and side streams when the cigarette is smoked. This aroma is described as being sweet, rich, floral, fruity, honey-like and Virginia tobacco-like.

While our invention is particularly useful in the manufacture of smoking tobacco, such as cigarette tobacco, cigar tobacco and pipe tobacco, other tobacco products formed from sheeted tobacco dust or fines may also be used. Likewise, the phenethylether derivatives of our invention can be incorporated with materials such as filter tip materials, seam paste, packaging materials and the like which are used along with tobacco to form a product adapted for smoking. Furthermore, the phenethylether derivatives of our invention can be added to certain tobacco substitutes of natural or synthetic origin (e.g., dried lettuce leaves and cellulose derivatives) and, accordingly, by the term "tobacco" as used throughout this specification is meant any composition intended for human consumption by smoking or otherwise, whether composed of tobacco plant parts or substitute materials, or both.

It will thus be apparent that one or more of the phenethylether derivatives of our invention can be

utilized to alter, modify, augment or enhance the aroma of a wide variety of consumable materials including fragrance formulations, colognes, pheromones, smoking tobaccos, smoking tobacco articles, insecticides and perfumed articles in general.

The following examples serve to illustrate our invention and this invention is to be considered restricted thereto only as indicated in the appended claims.

All parts and percentages given herein are by weight unless otherwise specified.

The words "Amberlyst", 'Goodloe", "Neodol" and "Adogen" are Trade Marks.

Example I(A)

Preparation of cyclohexyl phenethylether

Reaction:

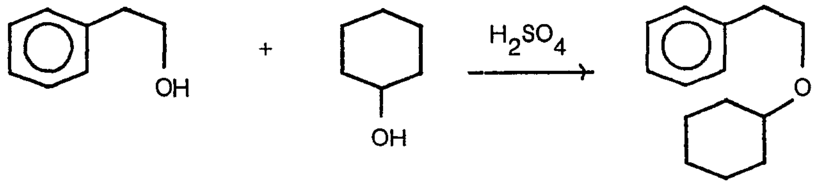


Into a 2 liter reaction vessel equipped with stirrer, thermometer, heating mantle and overhead condenser with azeotrope takeoff apparatus, is placed 488 grams of beta-phenylethyl alcohol; 440 grams of cyclohexanol and 100 grams of concentrated sulfuric acid. The reaction mass is then heated to a temperature of 115°C and maintained at a temperature in the range of 104—127°C at reflux while azeotropically removing 80 ml water, for a period of 3 hours. At the end of the 3 hour period, a sample is analyzed by means of GLC analysis (conditions: 180°C isothermal using an SE—30 packed column). Figure 1(A) is the GLC profile of the reaction product at this point.

The resulting reaction mass is then distilled on a 1' x 29/42 distillation column yielding the following fractions:

| Fraction Number | Vapor Temp. (°C) | Liquid Temp. (°C) | Pressure mm/Hg. | Reflux Ratio |
|---|---|---|---|---|
| 1 | 27 | 40 | 1.2 | 1/4 |
| 2 | 70 | 100 | 1.0 | 4/1 |
| 3 | 67 | 100 | 1.0 | 4/1 |
| 4 | 67 | 100 | | 4/1 |
| 5 | 70 | 105 | 0.7 | 4/1 |
| 6 | | | | 4/1 |
| 7 | 70 | 130 | 0.7 | 4/1 |
| 8 | 88 | 140 | | 4/1 |
| 9 | 100 | 132 | 0.55 | 4/1 |
| 10 | 100 | 160 | | 4/1 |
| 11 | 100 | 165 | | 4/1 |
| 12 | 102 | 185 | 0.4 | 4/1 |
| 13 | 107 | 205 | 0.5 | 4/1 |

Figure 1(B) is the GLC profile for fraction 8 of the distillation product of the reaction product of this example which contains phenethyl cyclohexylether having the structure:

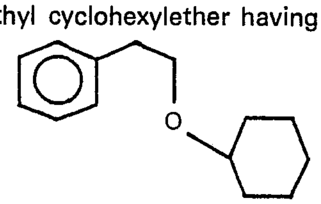

Figure 1(C) is the GLC profile for fraction 10 of the distillation product of the reaction product of this example containing the compound having the structure:

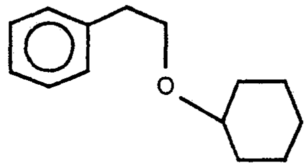

Figure 1(D) is the GLC profile for fraction 12 of the distillation product of the reaction product of this example containing the compound having the structure:

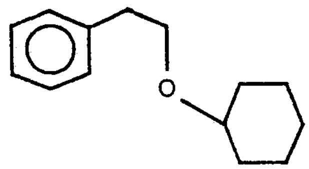

Figure 2 is the NMR spectrum for peak "A" of the GLC profile of Figure 1(A) which consists of the compound having the structure:

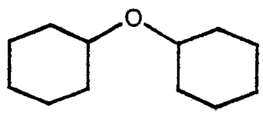

Figure 3 is the NMR spectrum for peak "B" of the GLC profile of Figure 1(A) containing the compound having the structure:

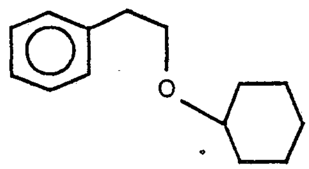

Figure 4 is the infra-red spectrum for peak "B" of the GLC profile of Figure 1(A) containing the compound having the structure:

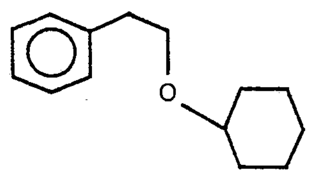

Example 1(B)

Reaction:

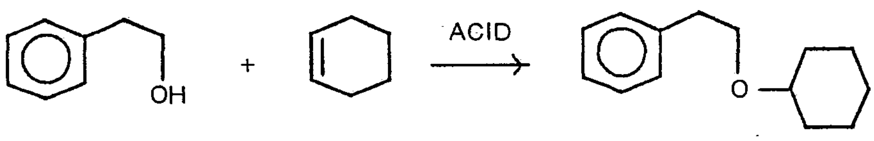


Phenethyl alcohol (1487 grams; 12.2 moles) and Amberlyst® 15 catalyst (a sulfonated phenyl polyethylene ion exchange resin manufactured by the Rohm & Haas Corporation of Philadelphia, Pennsylvania (85.4 grams)) are heated to 110°C. 1000 grams (12.2 moles) of cyclohexene is added drop-wise to the reaction mass over a period of 4.5 hours while maintaining the reaction mass at 110°C. During the addition of the cyclohexene, residual water (23 ml) is removed by azeotropic distillation through a water separating trap. The reaction mass is stirred at 110°C for an additional 5.25 hours. At this point in time, the reaction mass is cooled and the catalyst is removed by filtration. The organic solution is washed with 400 grams of 30% sodium hydroxide and then distilled through a 1½" x 12" Goodloe column yielding the following fractions:

| Fraction Number | Vapor Temp. (°C) | Liquid Temp. (°C) | Pressure mm/Hg. | Reflux Ratio | Weight of Fraction (g.) |
|---|---|---|---|---|---|
| 1 | 35/30 | 75/130 | 300 | 100 | 236.7 |
| 2 | 25/74 | 121/105 | 3 | 9:1 | 77g |
| 3 | 71 | 96 | 5.8 | 9:1 | 35.4 |
| 4 | 72 | 121 | 3.0 | 9:1 | 120.9 |
| 5 | 74 | 137 | 3.0 | 9:1 | 225.9 |
| 6 | 105 | 140 | 3.0 | 9:1 | 61.9 |
| 7 | 105 | 140 | 3.0 | 9:1 | 44.1 |
| 8 | 105 | 140 | 3.0 | 9:1 | 34.2 |
| 9 | 105 | 140 | 3.0 | 9:1 | 113g |
| 10 | 105 | 142 | 2.6 | 1:1 | 82g |
| 11 | 105 | 145 | 2.6 | 1:1 | 101.7 |
| 12 | 105 | 145 | 2.6 | 1:1 | 83g |
| 13 | 105 | 139 | 2.6 | 1:1 | 92.7 |
| 14 | 105 | 140 | 2.6 | 1:1 | 116.3 |
| 15 | 105 | 140 | 2.6 | 1:1 | 84.7 |
| 16 | 105 | 137 | 2.6 | 1:1 | 100.8 |
| 17 | 105 | 138 | 2.6 | 1:1 | 107.3 |
| 18 | 105 | 140 | 2.6 | 1:1 | 90.8 |
| 19 | 105 | 142 | 2.6 | 1:1 | 97g |
| 20 | 108 | 146 | 3mm | 9:1 | 820g |
| 21 | 108 | 157 | 3 | 9:1 | 111.5 |
| 22 | 108 | 163 | 3 | 9:1 | 79g |
| 23 | 117 | 199 | 3 | 9:1 | 54.4 |
| 24 | 129 | 250 | 3 | 9:1 | 95.1g |

Fractions 11—22 contain phenylethyl cyclohexyl ether having a purity of greater than 99%.

Figure 5 is the GLC profile for the reaction product immediately after the sodium hydroxide wash and prior to distillation.

Figure 6(A) is the GLC profile for fraction 7 of the distillation product of the reaction product of Example I(B) containing the compound having the structure:

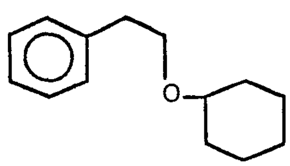

Figure 6(B) is the GLC profile for fraction 10 of the distillation product of the reaction product of Example I(B) containing the compound having the structure:

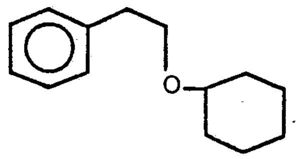

Figure 6(C) is the GLC profile for fraction 22 of the distillation product of the reaction product of Example I(B) containing the compound having the structure:

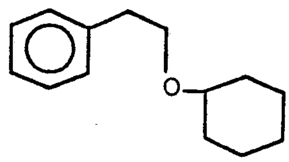

## Example II

*Preparation of cyclohexyl hydrotropyl ether*

Reaction:

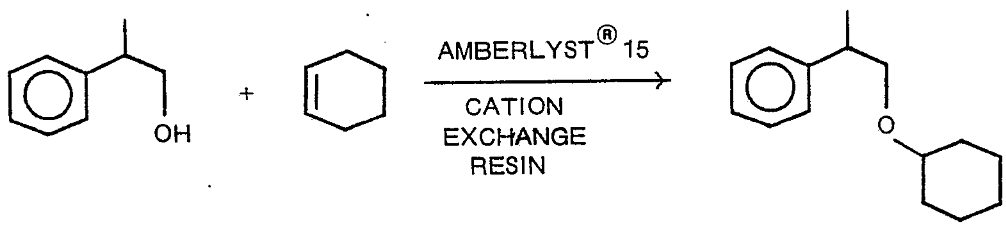


Into a stirred slurry of 408 grams of betamethylphenylethyl alcohol and 25 grams of Amberlyst® 15 (a polysulfonated polystyrene cation exchange resin manufactured by the Rohm & Haas Company of Philadelphia, Pennsylvania) maintained at 110°C is added 246 grams of cyclohexene over a 2.75 hour period. The reaction mass is aged at 110°C for an additional two hours whereupon it is cooled and filtered. The solution is then heated at reflux with 80 grams of 30% aqueous sodium hydroxide. The reaction mass is then cooled and washed with one liter of water. The organic layer is then separated from the aqueous layer and distilled and fractions rich in product are then fractionated through a 12″ x 1″ Goodloe packed column to yield the following fractions:

| Fraction Number | Vapor Temp. (°C) | Liquid Temp. (°C) | Pressure mm/Hg. | Reflux Ratio | Weight of Fraction (g.) |
|---|---|---|---|---|---|
| 1 | 47/92 | 111/RO | 1.4 | 9:1 | |
| 2 | 85 | 125 | 1.4 | 9:1 | 17g |
| 3 | 85 | 124 | 1.4 | 9:1 | 18g |
| 4 | 85 | 124 | 1.4 | 3:1 | 24.5 |
| 5 | 97 | 119 | 1.4 | 2:1 | 31.5 |
| 6 | 87 | 125 | 1.4 | 2:1 | 31.6 |
| 7 | 93 | 127 | 1.4 | 2:1 | 27.5 |
| 8 | 86 | 129 | 1.2 | 2:1 | 21.6 |
| 9 | 86 | 136 | 1.2 | 2:1 | 22 |
| 10 | 94 | 145 | 1.2 | 2:1 | 11.3 |
| 11 | 102 | 146 | 1.2 | 2:1 | 13.6 |
| 12 | 108 | 160 | 1.2 | 2:1 | 17.1 |
| 13 | 105 | 185 | 1.2 | 2:1 | 3.6 |

Figure 7(A) is the GLC profile of the crude reaction product (conditions: 1/8" x 10' 10% SE—30 packed column operated at 180°C isothermal).

Figure 7(B) is the GLC profile for fraction 8 of the distillation product (conditions: 1/8" x 10' SE—30 packed column; programmed at 220°C isothermal).

Figure 8 is the NMR spectrum for fraction 6 of the distillation product as set forth above.

Figure 9 is the infra-red spectrum for fraction 6 of the distillation product as set forth above.

The resulting product has a floral, green, galbanum-like fruity aroma.

Example III

*Preparation of p-methyl phenylethyl cyclohexyl ether*

Reaction:

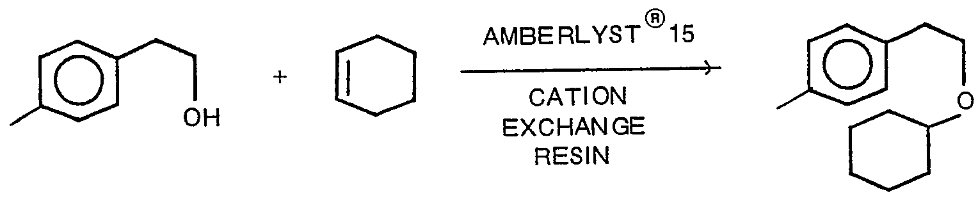


246 grams of cyclohexene is added to a stirred slurry of p-methyl phenyl beta ethanol (408 grams) and 25 grams of Amberlyst® 15 cation exchange resin maintained at 110°C, over a 2 hour period. The reaction mass is then aged at 110°C for an additional 3 hours whereupon it is cooled and filtered. The resulting solution is then heated at reflux with 80 grams of 30% aqueous sodium hydroxide. The reaction mass is then cooled and the resulting organic layer is washed with 1 liter of water. The organic layer is separated from the reaction mass and distilled through a 1½" x 12" Goodloe packed column to afford the following fractions:

| Fraction Number | Vapor Temp. (°C) | Liquid Temp. (°C) | Pressure mm/Hg. | Reflux Ratio | Weight of Fraction (g.) |
|---|---|---|---|---|---|
| 1 | 50/56 | 104/106 | .9 mm | 4:1 | 15.7 |
| 2 | 65 | 124 | | 4:1 | 32.6 |
| 3 | 65 | 126 | | 4:1 | 18.8 |
| 4 | 95 | 128 | | | 18.1 |
| 5 | 92 | 128 | .8 mm | | 3.8 |
| 6 | 95 | 133 | .75 | 9:1 | 12.9 |
| 7 | 95 | 137 | .6 | 9:1 | 16.0 |
| 8 | 95 | 133 | .6 | 1:1 | 31.4 |
| 9 | 95 | 133 | .6 | 1:1 | 27.8 |
| 10 | 92 | 136 | .5 | 1:1 | 22.9 |
| 11 | 92 | 136 | .5 | 1:1 | 29.1 |
| 12 | 96 | 137 | .5 | 1:1 | 30.4 |
| 13 | 94 | 143 | .5 | 1:1 | 29.9 |
| 14 | 94 | 146 | .5 | 1:1 | 28.5 |
| 15 | 94 | 151 | .5 | 1:1 | 30.8 |
| 16 | 98 | 165 | .5 | 1:1 | 30.0 |
| 17 | 95 | 180 | .5 | 1:1 | 27.2 |

Figure 10(A) is the GLC profile of the crude reaction product (conditions: 1/8″ × 10′ 10% SE—30 packed column programmed at 120—220°C at 16°C per minute).

Figure 10(B) is the GLC profile for fraction 8 of the foregoing distillation (conditions: 1/8″ × 10′ 10% SE—30 packed column programmed at 220°C isothermal).

Figure 11 is the NMR spectrum for fraction 10 of the foregoing distillation.

Figure 12 is the infra-red spectrum for fraction 10 of the foregoing distillation.

The resulting product having the structure:

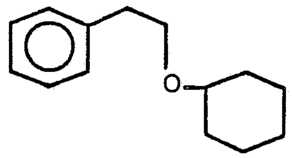

has an interesting intense, burnt fruit aroma.

Example IV(A)

*Preparation of 4-methyl-2-pentanyl phenethylether*

Reaction:

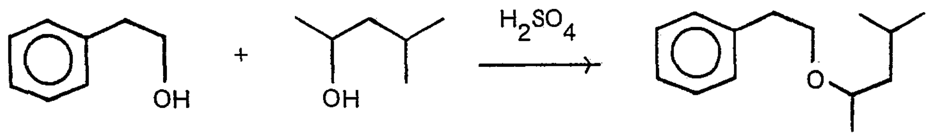


Into a 3 liter reaction vessel equipped with stirrer, thermometer, reflux condenser and heating mantle is placed 732 grams of phenylether alcohol, 1052 grams of 4-methyl-2-pentanol and 100 grams of concentrated (93%) sulfuric acid. The reaction mass is heated to reflux and water is continuously taken off through a Bidwell trap. The reaction mass is refluxed for a period of 14 hours.

At the end of the reaction, the reaction mass is quenched with 3 liters of water, washed with aqueous sodium carbonate (saturated) and then with saturated sodium chloride (aqueous).

The reaction mass is then brushed over followed by fractional distillation to yield the following fractions:

| Fraction Number | Vapor Temp. (°C) | Liquid Temp. (°C) | Pressure mm/Hg. | Reflux Ratio | Weight of Fraction (g.) |
|---|---|---|---|---|---|
| 1 | 40 | 80 | 4 | 9:1 | 52.7 |
| 2 | 64 | 86 | 2 | 9:1 | 48.7 |
| 3 | 68 | 90 | 2 | 9:1 | 29.1 |
| 4 | 68 | 92 | 2 | 9:1 | 27.7 |
| 5 | 68 | 93 | 2 | 9:1 | 18.7 |
| 6 | 70 | 99 | 1.8 | 9:1 | 27.4 |
| 7 | 78 | 100 | 3 | 2:1 | 27.0 |
| 8 | 80 | 105 | 2 | 2:1 | 24.8 |
| 9 | 80 | 105 | 2 | 2:1 | 27.9 |
| 10 | 80 | 105 | 2 | 2:1 | 22.4 |
| 11 | 80 | 105 | 2 | 2:1 | 28.2 |
| 12 | 80 | 105 | 2 | 2:1 | 25.5 |
| 13 | 80 | 108 | 2 | 2:1 | 27.2 |
| 14 | 87 | 114 | 2 | 2:1 | 19.5 |
| 15 | 99 | 172 | 2 | 2:1 | 26.5 |
| 16 | 93 | 220 | 3.5 | 2:1 | 14.5 |

Figure 13 is the GLC profile for the crude reaction product produced according to this example containing the compound having the structure:

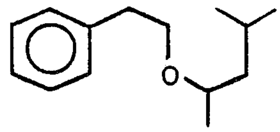

Figure 14 is the NMR spectrum for the reaction product of this example containing the compound having the structure:

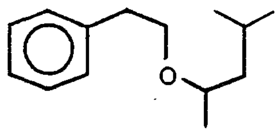

Figure 15 is the infra-red spectrum for the reaction product of this example containing the compound having the structure:

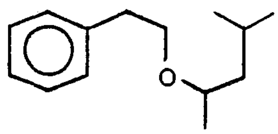

EXAMPLE IV(B)

*Preparation of 4-methyl-2-pentanyl phenethylether*

Reaction:

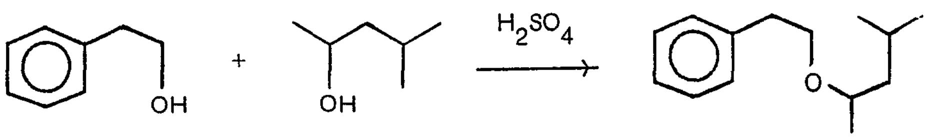


Into a 10 liter reaction vessel equipped with stirrer, thermometer, reflux condenser and heating mantle is placed 1428 grams of 4-methyl-2-pentanol; 915 grams of phenylethyl alcohol and 500 grams of sulfuric acid. The reaction mass is heated to reflux (105°C) and refluxed for a period of 2 hours.

Figure 16 is the GLC profile for the crude reaction mass.

Example V

*Preparation of isopropyl phenethylether*

Reaction:

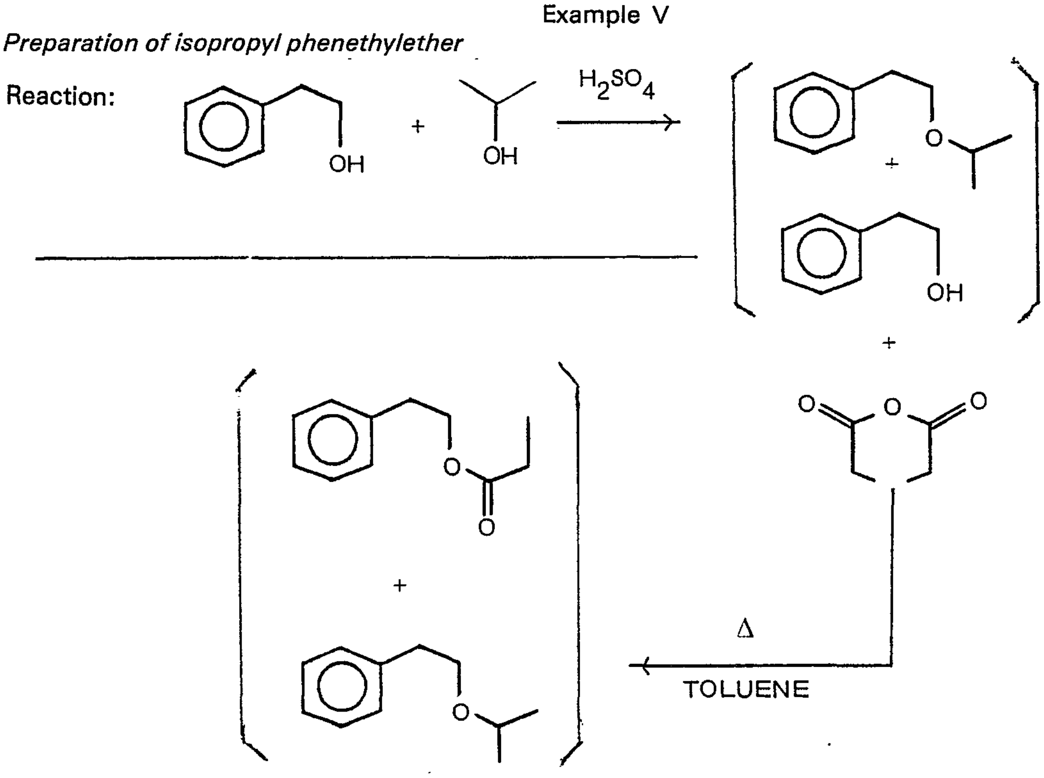

Into a 10 liter reaction flask is placed 1440 grams of isopropyl alcohol and 732 grams of phenylethyl alcohol. Over a period of 1 hour, a solution of 270 grams of sulfuric acid and 30 ml water is added slowly. At the end of the addition of the sulfuric acid solution, the reaction mass is heated to reflux and the reaction mass is refluxed for a period of 4 hours. At the end of the 4 hour period, three liters of water is added to the reaction mass and the reaction mass is neutralized with 50% sodium hydroxide. One liter of toluene is added and the toluene layer is separated from the aqueous layer.

To the toluene mixture is added 260 grams of propionic anhydride and the propionic anhydride/toluene/phenylethyl alcohol/isopropyl phenethylether mixture is then refluxed for a period of 30 minutes. The reaction mass is then cooled down and one liter of water is added thereto. The reaction mass is then washed with 50% caustic followed by saturated sodium chloride and then neutralized with saturated sodium bicarbonate.

The reaction mass is then distilled yielding the following fractions:

| Fraction Number | Vapor Temp. (°C) | Liquid Temp. (°C) | Pressure mm/Hg. | Weight of Fraction (g.) |
|---|---|---|---|---|
| 1 | 24/70 | 24/101 | 1.8/1.8 | 4.2 |
| 2 | 70 | 101 | 1.4 | 12.4 |
| 3 | 60 | 101 | 1.1 | 14.5 |
| 4 | 65 | 102 | 1.1 | 21.0 |
| 5 | 65 | 104 | 1.0 | 27.8 |
| 6 | 60 | 86 | 1.0 | 22.3 |
| 7 | 58 | 110 | 1.3 | 26.3 |
| 8 | 59 | 112 | 1.3 | 26.8 |
| 9 | 64 | 113 | 1.3 | 23.9 |
| 10 | 65 | 113 | 1.4 | 25.8 |
| 11 | 70 | 115 | 1.3 | 28.2 |
| 12 | 72 | 115 | 1.3 | 29.2 |
| 13 | 74 | 115 | 1.3 | 29.6 |
| 14 | 76 | 116 | 1.3 | 30.0 |
| 15 | 77 | 116 | 1.3 | 29.3 |
| 16 | 76 | 115 | 1.2 | 29.1 |
| 17 | 76 | 115 | 1.2 | 27.7 |
| 18 | 76 | 115 | 1.2 | 28.9 |
| 19 | 77 | 116 | 1.2 | 28.9 |
| 20 | 77 | 116 | 1.2 | 29.5 |
| 21 | 78 | 117 | 1.2 | 27.8 |
| 22 | 79 | 117 | 1.2 | 29.6 |
| 23 | 79 | 117 | 1.2 | 25.0 |

| Fraction Number | Vapor Temp. (°C) | Liquid Temp. (°C) | Pressure mm/Hg. | Weight of Fraction (g.) |
|---|---|---|---|---|
| 24 | 82 | 126 | 1.2 | 25.0 |
| 25 | 83 | 128 | 1.2 | 27.1 |
| 26 | 85 | 156 | 1.2 | 29.2 |
| 27 | 106 | 250 | 1.2 | 18.9 |

Figure 17 is the GLC profile for the crude reaction product of this example prior to propionic anhydride reaction containing the compounds having the structures:

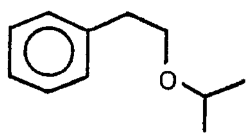

and

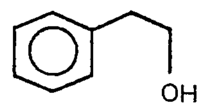

Figure 18 is the GLC profile for fraction 4 of the distillation product of the reaction product of this example after reaction with propionic anhydride, containing the compound having the structure:

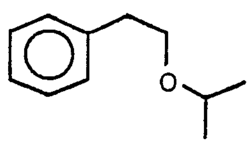

Figure 19 is the NMR spectrum for the reaction product of this example after distillation containing the compound having the structure:

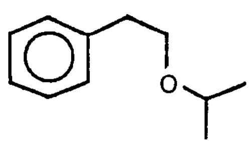

Figure 20 is the infra-red spectrum for the reaction product of this example after distillation after reaction with propionic anhydride containing the compound having the structure:

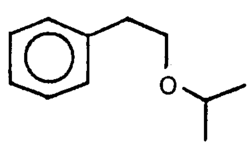

## Example VI

*Preparation of 2-butenyl phenethylether and 1-methallyl phenethylether*

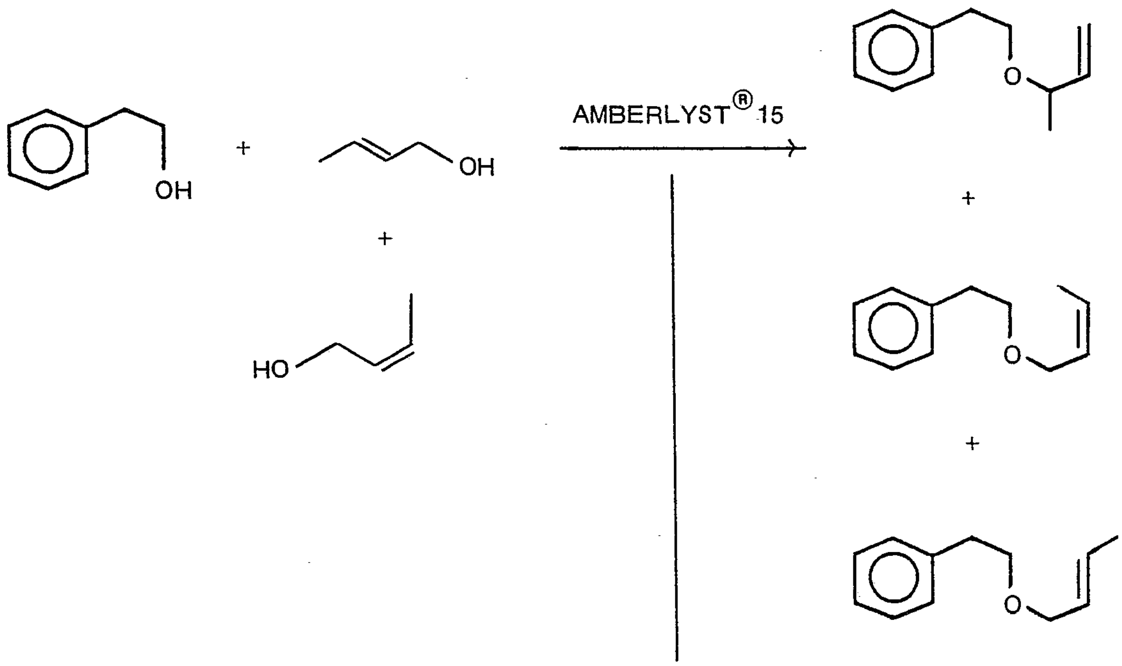


Crotyl alcohol (216 grams) is slowly added to a stirred slurry of phenylethyl alcohol (366 grams) and Amberlyst® 15 (cation exchange resin manufactured by the Rohm & Haas Company, Philadelphia, Pennsylvania; an acid cation exchange resin which is sulfonated polystyrene) at 100°C. The reaction mass is aged at 100°C for a period of 5 hours whereupon the solution is cooled and filtered. The organic solution is washed with 25% aqueous sodium hydroxide. The GLC profile of the crude reaction product is set forth in Figure 21 (conditions: 10% SE-30 packed column programmed at 120—220°C at 8°C per minute). Peak 13 is the peak for phenylethyl alcohol; peak 14 is the peak for the 1-methallyl phenethylether; peak 15 is the peak for 2-butenyl phenethylether (cis and trans isomers).

A portion of the product is isolated by GLC preparative chromatography.

Figure 22(A) represents the NMR spectrum for peak 14 of the GLC profile of figure 21 containing the compound having the structure:

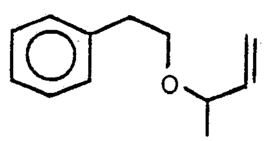

Figure 22(B) is the infra-red spectrum for peak 14 of figure 21 which is the GLC profile for the reaction product of this example containing the compound having the structure:

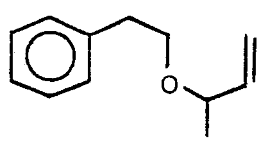

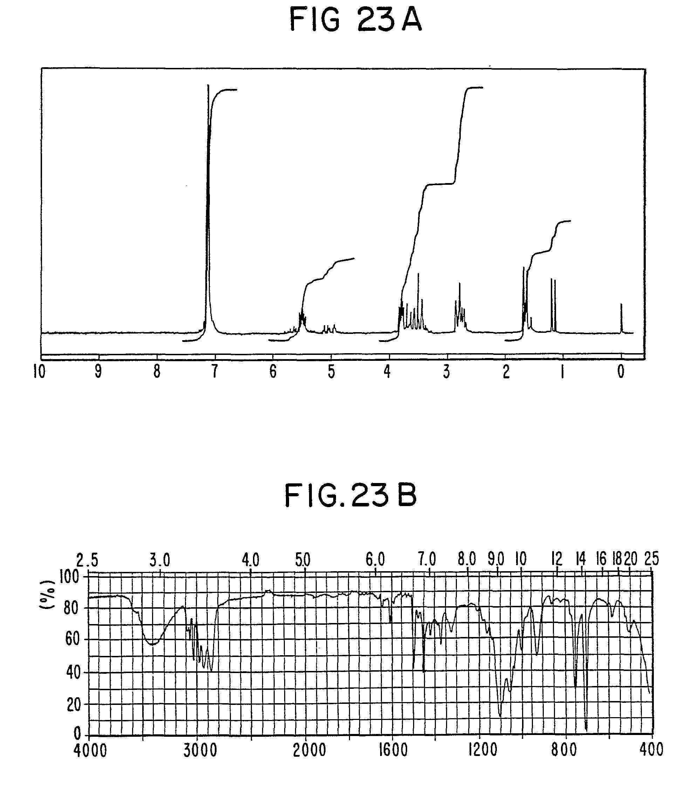

Figure 23(A0 is the NMR spectrum for peak 15 of figure 21 which is the GLC profile for the reaction product of this example containing the compounds having the structures:

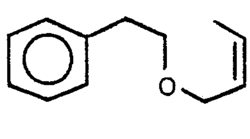 + 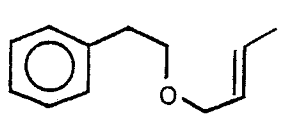

Figure 23(B) is the infra-red spectrum for peak 15 of figure 21 which is the GLC profile of the reaction product of this example containing the compounds having the structures:

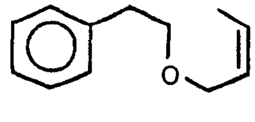

+

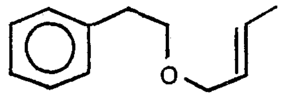

Example VII

Field tests are made each time using 100 male and 100 female *Lasioderma serricorne (F.)* cigarette beetles. The beetles were released at a certain distance from the source of attraction which was treated with cyclohexyl phenethylether prepared according to either of Example I(A) or I(B). Further, felled trees having already been contaminated with the respective beetles are positioned at both sides of the starting point. After a certain period of time, the amount of insects gathered at the source of attraction was determined thus indicating the effectiveness of the pheromonal mixture according to the invention.

Field tests with *Lasioderma serricorne (F.)* are made whereby the distance between the starting point and the source of attraction is 50 meters. Four independent field tests were made whereby 42% of the male beetles and 46% of the female beetles gathered at each catch tree. The concentration of insects at the catch tree was 55% of the male insects and 58% of the female insects. In all these tests the catch tree was impregnated with a 0.7% ethanolic solution of cyclohexyl phenethylether (7 gm cyclohexyl phenethylether per 92 gm of 95% aqueous ethanol).

Example VIII

During two consecutive days several felled oak trees surrounding a field of tobacco plants were treated with 250 mg of phenethyl cyclohexylether in 1.0% ethanolic solution. These trees were exposed in an area which was contaminated with *Lasioderma serricorne (F.)*. After 3 to 4 days, 100 beetles per square meter were observed on the logs. Other untreated logs or trees in the direct neighborhood of the treated logs or trees showed very few (about 12) insects per square meter on the average while other trees at a distance of 10 to 20 meters showed no contamination.

Example IX

In a large test field, mixtures of cyclohexyl phenethylether in admixture with different DDT preparations, fluorine-containing mixtures and arsenic-containing mixtures as well as hexachloro-cyclohexane were used. These mixtures contained also small amounts of surface-active agents and carriers. The mixtures were applied to catch trees namely logs of oak trees in an area of tobacco plants contaminated with *Lasioderma serricorne (F.)*. The distance between the catch trees was always 200 meters. After 8 days there was no contamination either in the tobacco fields or around the oak trees. About 92% of the *Lasioderma serricorne (F.)*. insects were destroyed. Surprisingly, it was found that after the fourth days, the attracting effect was not diminished in spite of dead insects being present. Furthermore, in those areas where cyclohexyl phenethylether was used alone, the average number of insects destroyed was about 80% which in itself is surprising. Thus, the cyclohexyl phenethylether not only acts as a pheromone but also as an insecticide. Furthermore, the entire area wherein the cyclohexyl phenethylether was used had a faint pleasant floral aroma covering any adverse and aesthetically displeasing aroma of any other insecticides that were used.

Example X

The following mixture is prepared:

| Ingredients | Parts by Weight |
|---|---|
| Phenylacetic acid | 70.0 |
| Coumarin | 20.0 |
| Phenylethylphenyl acetate | 100.0 |
| Phenylethyl alcohol | 5.0 |
| Benzyl benzoate | 100.0 |
| Dimethylphenylethyl carbinol | 10.0 |
| Methyl anthranilate | 5.0 |
| Beta ionone | 10.0 |
| Cyclohexyl phenethylether | 30.0 |

The cyclohexyl phenethylether prepared according to either of Example I(A) or I(B) imparts the dry green, hyacinth, rose, galbanum-like aroma to this honey fragrance while giving it a very warm undertone and imparting a very long-lasting floral top note to this fragrance.

Example XI

*Preparation of a cosmetic powder composition*

A cosmetic powder is prepared by mixing in a ball mill 100 grams of talcum powder with 0.25 grams of the perfume composition prepared according to Example X. It has an excellent floral aroma.

Example XII

*Perfumed liquid detergent*

Concentrated liquid detergents (lysine salt of n-dodecylbenzene sulfonic acid as more specifically described in U.S. Patent No. 3,948,818 issued on April 6, 1976) with floral aroma nuances and rose, galbanum and hyacinth-like top notes are prepared containing 0.10%, 0.15% and 0.20% of the fragrance prepared according to Example X. They are prepared by adding and homogeneously mixing the appropriate quantity of fragrance formulation prepared according to Example X in the liquid detergent. The detergents all possess excellent floral aromas with dry green, hyacinth, rose and galbanum nuances, the intensity increasing with greater concentrations of perfume composition prepared according to Example X.

Example XIII

*Preparation of a cologne and handkerchief perfume*

The composition prepared according to Example X is incorporated into a cologne at concentration of 2.0%, 2.5%, 3.0%, 4.0%, 4.5% and 5.0% in 80%, 85%, 90% and 95% aqueous food grade ethanol; and into handkerchief perfumes at concentrations of 15%, 20% and 30% (in 80%, 85% and 95% aqueous food grade ethanol). A distinctive and definitive dry green, hyacinth, rose, galbanum, rich and full-bodied floral aroma is imporated to the cologne and to the handkerchief perfume at all levels indicated.

Example XIV

*Preparation of soap composition*

100 grams of soap chips are mixed with 1 gram of the formulation of Example X until a homogeneous composition is obtained. The homogeneous composition is heated under three atmospheres pressure at 180°C for a period of three hours and the resulting liquid is placed into soap molds. The resulting soap cakes, on cooling, manifest excellent floral aromas with dry green, hyacinth, rose and galbanum nuances that are very long-lasting.

Example XV

*Preparation of a solid detergent composition*

A detergent is prepared from the following ingredients according to Example I of Canadian Patent No. 1,007,948:

| | Percent by Weight |
|---|---|
| "Neodol 45-11" (a $C_{14}$—$C_{15}$ alcohol ethoxylated with 11 moles of ethylene oxide) | 12 |
| Sodium carbonate | 55 |
| Sodium citrate | 20 |
| Sodium sulfate, water brighteners | q.s. |

This detergent is a "phosphate-free" detergent. A total of 100 grams of this detergent is admixed with 0.15 grams of the honey based perfume of Example X. Each of the detergent samples have an excellent honey-like, dry green, hyacinth, rose and galbanum aroma.

Example XVI

*Dryer-added fabric softener article*

Utilizing the procedure of Example I at column 15 of U.S. Patent No. 3,632,396, a non-woven cloth substrate useful as a dryer-added fabric softening article of manufacture is prepared wherein the substrate, the substrate coating and the outer coating and the perfuming material are as follows:

1. a water "dissolvable" paper ("Dissolvo Paper");
2. Adogen 448 (m.p. about 140°F) as the substrate coating; and

3. an outer coating having the following formulation (m.p. about 150°F.):
57% $C_{20-22}$ HAPS
22% isopropyl alcohol
20% antistatic agent
1% of cyclohexyl phenethylether produced according to either of Example I(A) or I(B)

A fabric softening composition prepared as set forth above having a dry green, hyacinth, rose galbanum and generally floral aroma characteristic consists of a substrate having a weight of about 3 grams per 100 square inches, a substrate coating of about 1.85 grams per 100 square inches of substrate and an outer coating of about 1.4 grams per 100 square inches of substrate, thereby providing a total aromatized substrate and outer coating weight ratio of about 1:1 by weight of the substrate. A pleasant floral aroma is imparted in a pleasant manner to the head space in the dryer on operation thereof using the said dryer-added fabric softening non-woven fabric. HAPS is 3-(N-alkyl-N,N-dimethylammonium)-2-hydroxy propane-1-sulfonate, in which the alkyl group is a mixture of from 20—22 carbon atoms (see US Patent Specification No. 3632396, column 5, lines 60 to 65).

Example XVII

A liquid detergent composition prepared according to Example IV of United Kingdom Patent No. 1,498,520 whereby the following ingredients are admixed:

| Ingredient | Weight % |
|---|---|
| Coconut alcohol ethoxylate | 30 |
| Linear alkyl benzene sulfonate, triethanolamine salt (alkyl = $C_{11.8}$ avg.) | 10 |
| Potassium chloride | 3 |
| Triethanolaine | 3 |
| Triethanolammonium citrate | 2 |
| Ethyl alcohol | 5 |
| Soil release ether "D" | 1 |
| Cyclohexyl phenethylether prepared according to either of Example I(A) or I(B) | 3 |
| Water | Balance |

The soil release either "D" is defined according to Table II on page 15 of United Kingdom Patent No. 1,498,520.

This composition is prepared by admixing all of the ingredients exclusive of soil release ether "D" and agitating the mixture until all electrolytes are dissolved.

Soil release ether "D" is then admixed wtih the solution in the form of a dry powder which passes through a 150 mesh standard sieve. The resulting composition is in the liquid state and is easily pourable. The composition is found not to redden on contact with plastic bottles, does not gel when diluted with water and has a long-lasting aroma which can be described as dry green, hyacinth, rose, galbanum and rich and rather long-lasting. Indeed, the aroma lasts for several weeks when exposed to the atmosphere.

This composition is added to an aqueous laundrying bath at a concentration of 0.20% (weight) at a temperature of 55°C, water hardness 7 grains per gallon and a pH of 10.0. Polyester and mixed polyester/cotton fabrics are laundered in the bath for a period of 10 minutes after which the fabrics are thoroughly rinsed with fresh water and dried at ambient temperatures. The fabrics are provided with a soil release finish. The head space above the fabrics has a pleasant faint aroma which can be described as hyacinth, rose and galbanum and also rather long-lasting (about 3 days).

Example XVIII

*Preparation of a cosmetic powder composition*

A cosmetic powder is prepared by admixing in a ball mill, 100 grams of talcum powder with 0.25 grams of phenethyl cyclohexylether prepared according to either of Examples I(A) or I(B). The resulting cosmetic powder has an excellent dry green, hyacinth, rose, galbanum, rich and full-bodied floral fragrance which is very long-lasting.

## Example XIX

*Preparation liquid detergent*

Concentrated liquid detergents (lysine salt of n-dodecylbenzene sulfonic acid as more specifically described in U.S. Patent No. 3,948,818 issued on April 6, 1976) with dry green, hyacinth, rose, galbanum, rich and full-bodied floral aroma are prepared containing 0.10%, 0.15%, 0.20% and 0.25% of cyclohexyl phenethylether prepared according to either of Example I(A) or I(B). They are prepared by adding and homogeneously admixing the appropriate quantity of phenethyl cyclohexylether in the liquid detergent. The detergents all possess intense, long-lasting dry green, hyacinth, rose, galbanum and generally floral aroma characteristics.

## Example XX

*Preparation of colognes and handkerchief perfumes*

Cyclohexyl phenethylether prepared according to either of Example I(A) or I(B) is incorporated into colognes at concentrations of 2.0%, 2.5%, 3.0%, 3.5%, 4.0% and 4.5% in 80%, 85%, 90% and 95% aqueous food grade ethanol; and into handkerchief perfumes at concentrations of 15%, 20%, 25% and 30% (in 80%, 90%, and 95% aqueous ethanol). Distinctive dry green, hyacinth, rose, galbanum, rich and full-bodied floral aroma nuances which are very long-lasting on dry-out (54 hours) are imparted to the colognes and to the handkerchief perfumes at the various above levels indicated.

## Example XXI

Field tests are made each time using 100 male and 100 female *Lasioderma serricorne (F.)* cigarette beetles. The beetles were released at a certain distance from the source of attraction which was treated with one of the cyclohexyl phenethylether derivatives prepared according to either Example II or III. Further, felled trees having already been contaminated with the respective beetles are positioned at both sides of the starting point. After a certain period of time, the amount of insects gathered at the source of attraction was determined thus indicating the effectiveness of the pheromonal mixture according to the invention.

Field tests with *Lasioderma serricorne (F.).* are made whereby the distance between the starting point and the source of attraction is 50 meters. Four independent field tests were made whereby 42% of the male beetles and 46% of the female beetles gathered at each catch tree. The concentration of insects at the catch tree was 55% of the male insects and 58% of the female insects. In all these tests the catch tree was impregnated with a 0.7% ethanolic solution of one of the cyclohexyl phenethylether derivatives of either Example II or III (7 grams of cyclohexyl phenethylether derivative per 92 grams of 95% aqueous ethanol).

## Example XXII

During two consecutive days, several felled oak trees surrounding a field of tobacco plants were treated with 250 mg of one of the phenethyl cyclohexylether derivatives of either Example II or III in 1.0% ethanolic solution. These trees were exposed in an area which was contaminated with *Lasioderma serricorne (F.).* After 3 to 4 days, 100 beetles per square meter were observed on the logs. Other untreated logs or trees in the direct neighborhood of the treated logs or trees showed very few (about 12) insects per square meter on the average while other trees at a distance of 10 to 20 meters showed no contamination.

## Example XXIII

In a large test field, mixtures of one of the cyclohexyl phenethylether derivatives of Example II or III in admixture with different DDT preparations, fluorine-containing mixtures and arsenic-containing mixtures as well as hexachloro-cyclohexane were used. These mixtures contained also small amounts of surface active agents and carriers. The mixtures were applied to catch trees namely logs of oak trees in an area of tobacco plants contaminated with *Lasioderma serricorne (F.).* The distance between the catch trees was always 200 meters. After 8 days there was no contamination either in the tobacco fields or around the oak trees. About 92% of the *Lasioderma serricorne (F.).* insects were destroyed. Surprisingly, it was found that after the fourth day, the attracting effect was not diminished in spite of dead insects being present. Furthermore, in those areas where one of the cyclohexyl phenethylether derivatives of Example II or III was used alone, the average number of insects destroyed was about 80% which in itself is surprising. Thus, one of the cyclohexyl phenethylether derivatives of Example II or III not only acts as a pheromone but also as an insecticide. Furthermore, the entire area wherein one of the cyclohexyl phenethylether derivatives of Example II or III was used had a faint, pleasant aroma (floral in the case of the cyclohexyl phenethylether derivative of Example II and fruity in the case of the cyclohexyl phenethylether derivative of Example III) covering any adverse and aesthetically displeasing aroma of any other insecticides that were used.

Example XXIV

The following mixture is prepared:

| Ingredient | Parts by Weight |
| --- | --- |
| Phenylacetic acid | 70.0 |
| Coumarin | 20.0 |
| Phenylethylphenyl acetate | 100.0 |
| Phenyl ethyl alcohol | 5.0 |
| Benzyl benzoate | 100.0 |
| Dimethylphenylethyl carbinol | 10.0 |
| Methyl anthranilate | 5.0 |
| Beta ionone | 10.0 |
| Cyclohexyl hydrotropyl ether produced according to Example II | 30.0 |

The cyclohexyl hydrotropyl ether prepared according to Example II imparts the floral, green, galbanum-like and fruity aroma to this honey fragrance while giving it a very warm undertone and imparting a very long-lasting floral top note to this fragrance.

Example XXV

*Preparation of a cosmetic powder composition*

A cosmetic powder is prepared by mixing in a ball mill 100 grams of talcum powder with 0.25 grams of the perfume composition prepared according to Example XXIV. It has an excellent floral aroma.

Example XXVI

*Perfumed liquid detergent*

Concentrated liquid detergents (lysine salt of n-dodecylbenzene sulfonic acid as more specifically described in U.S. Patent No. 3,948,818 issued on April 6, 1976) with floral aroma nuances and green, galbanum-like and fruity top notes are prepared containing 0.10%, 0.15% and 0.20% of the fragrance prepared according to Example XXIV. They are prepared by adding and homogeneously mixing the appropriate quantity of fragrance formulation prepared according to Example XXIV in the liquid detergent. The detergents all possess excellent floral aromas with green, galbanum-like and fruity nuances, the intensity increasing with greater concentrations of perfume composition prepared according to Example XXIV.

Example XXVII

*Preparation of a cologne and handkerchief perfume*

The composition prepared according to Example XXIV is incorporated into a cologne at concentrations of 2.0% 2.5%, 3.0%, 4.0%, 4.5% and 5.0% in 80%, 85%, 90%, and 95% aqueous food grade ethanol and into handkerchief perfumes at concentraions of 15%, 20% and 30% (in 80%, 85% and 95% aqueous good grade ethanol). A distinctive and definitive green, galbanum-like, fruity and full-bodied floral aroma is imparted to each of the colognes and to each of the handkerchief perfumes at all levels indicated.

Example XXVIII

*Preparation of soap composition*

100 grams of soap chips are mixed with 1 gram of the formulation of Example XXIV until a homogeneous composition is obtained. The homogeneous composition is heated under three atmospheres pressure at 180°C for a period of three hours and the resulting liquid is placed into soap molds. The resulting soap cakes, on cooling, manifest excellent floral aromas with green, galbanum-like and fruity nuances that are very long-lasting.

Example XXIX

*Preparation of a solid detergent composition*

A detergent is prepared from the following ingredients according to Example I of Canadian Patent No. 1,007,948:

| | Percent by Weight |
|---|---|
| "Neodol 45-11" (a $C_{14}$—$C_{15}$ alcohol ethoxylated with 11 moles of ethylene oxide) | 12 |
| Sodium carbonate | 55 |
| Sodium citrate | 20 |
| Sodium sulfate, water brighteners | q.s. |

This detergent is a "phosphate-free" detergent. A total of 100 grams of this detergent is admixed with 0.15 grams of the honey based perfume of Example XXIV. Each of the detergent samples has an excellent floral honey-like, green galbanum-like and fruity aroma.

Example XXX

*Dryer-added fabric softener article.*

Utilizing the procedure of Example I at column 15 of U.S. Patent No. 3,632,396, a non-woven cloth substrate useful as a dryer-added fabric softening article of manufacture is prepared wherein the substrate, the substrate coating and the outer coating and the perfuming material are as follows:

1. a water "dissolvable" paper ("Dissolvo Paper");
2. Adogen 448 (m.p. about 140°F.) as the substrate coating; and
3. an outer coating having the following formulation (m.p. about 150°F.):

57% $C_{20-22}$ HAPS
22% isopropyl alcohol
20% antistatic agent
1% cyclohexyl hydrotropyl ether prepared according to Example II

A fabric softening composition prepared as set forth above having a green, galbanum-like and fruity and generally floral aroma characteristic consists of a substrate having a weight of about 3 grams per 100 square inches, a substrate coating of about 1.85 grams per 100 square inches of substrate and an outer coating of about 1.4 grams per 100 square inches of substrate, thereby providing a total aromatized substrate and outer coating weight ratio of about 1:1 by weight of the substrate. A pleasant floral aroma is imparted in a pleasant manner to the head space in the dryer on operation thereof using the said dryer-added fabric softening non-woven fabric.

Example XXXI

A liquid detergent composition prepared according to Example IV of United Kingdom Patent No. 1,498,520 whereby the following ingredients are admixed:

| Ingredient | Weight % |
|---|---|
| Coconut alcohol ethoxylate | 30 |
| Linear alkyl benzene sulfonate, triethanolamine salt (alkyl = $C_{11.8}$ avg.) | 8 |
| Potassium chloride | 3 |
| Triethanolamine | 3 |
| Triethanolammonium citrate | 2 |
| Ethyl alcohol | 5 |
| Soil release ether "D" | 1 |
| Cyclohexyl hydrotropyl ether prepared according to Example II | 5 |
| Water | Balance |

The soil release ether "D" is defined according to Table II on page 15 of United Kingdom Patent No. 1,498,520.

This composition is prepared by admixing all of the ingredients exclusive of soil release ether "D" and agitating the mixture until all electrolytes are dissolved. Soil release ether "D" is then admixed with the solution in the form of a dry powder which passes through a 150 mesh standard sieve. The resulting composition is in the liquid state and is easily pourable. The composition is found not to redden on contact with plastic bottles, does not gel when diluted with water and has a long-lasting aroma which can be described as green, galbanum-like, fruity and rich floral and rather long-lasting. Indeed, the aroma lasts for several weeks when exposed to the atmosphere.

This composition is added to an aqueous laundrying bath at a concentration of 0.20% (weight) at a temperature of 55°C, water hardness 7 grains per gallon and a pH of 10.0. Polyester and mixed polyester/cotton fabrics are laundered in the bath for a period of 10 minutes after which the fabrics are thoroughly rinsed with fresh water and dried at ambient temperatures. The fabrics are provided with a soil release finish. The head space above the fabrics has a pleasant faint aroma which can be described as floral, green, galbanum-like and fruity and also rather long-lasting (about 3 days).

Example XXXII

*Preparation of a cosmetic powder composition*

A cosmetic powder is prepared by admixing in a ball mill 100 grams of talcum powder with 0.25 grams of cyclohexyl hydrotropylether prepared according to Example II. The resulting cosmetic powder has an excellent green, floral, galbanum-like and fruity aroma which is very long-lasting.

Example XXXIII

*Perfumed liquid detergent*

Concentrated liquid detergents (lysine sale of n-dodecylbenzene sulfonic acid as more specifically described in U.S. Patent No. 3,948,818 issued on April 6, 1976) with floral, green, galbanum-like and fruity aromas are prepared containing 0.10%, 0.15%, 0.20% and 0.25% of cyclohexyl hydrotropyl ether prepared according to Example II. They are prepared by adding and homogeneously admixing the appropriate quantity of cyclohexyl hydrotropyl ether in the liquid detergent. The detergents all possess intense, long-lasting floral, green, galbanum-like and fruity aroma characteristics.

Example XXXIV

*Preparation of colognes and handkerchief perfumes*

Cyclohexyl hydrotropyl ether prepared according to Example II is incorporated into colognes at concentrations of 2.0%, 2.5%, 3.0%, 3.5%, 4.0% and 4.5% in 80%, 85%, 90% and 95% aqueous food grade ethanol; and into handkerchief perfumes at concentrations of 15%, 20%, 25% and 30% (in 80%, 90% and 95% aqueous ethanol). Distinctive floral, green, galbanum-like and fruity aroma nuances which are very long-lasting on dry-out (46 hours) are imparted to the colognes and to the handkerchief perfumes at the various levels indicated above.

Example XXXV

Field tests are made each time using 100 male and 100 female *Lasioderma serricorne (F.)*. cigarette beetles. The beetles were released at a certain distance from the source of attraction which was treated with either of 1,3-dimethylbutyl phenethylether prepared according to Example IV(A), isopropyl phenethylether prepared according to Example V or the mixture of 2-butenyl phenethylether and 1-methallyl phenethylether prepared according to Example VI. Further, felled trees having already been contaminated with the respective beetles are positioned at both sides of the starting point. After a certain period of time the amount of insects gathered at the source of attraction was determined thus indicating the effectiveness of the pheromonal mixtures according to the invention.

Fields tests with *Lasioderma serricorne (F.)* are made whereby the distance between the starting point and the source of attraction is 50 meters. Four independent field tests were made whereby 42% of the male beetles and 46% of the female beetles gathered at each catch tree. The concentration of insects at the catch tree was 58% of the male insects and 59% of the female insects. In all these tests the catch tree was impregnated with 0.7% ethanolic solutions of one of the following materials:

(a) 1,3-dimethylbutyl phenethylether prepared according to Example IV(A)

(b) isopropyl phenethylether prepared according to Example V

(c) a mixture of 2-butentyl phenethylether and 1-methallyl phenethylether prepared according to Example VI

(7 grams of phenethylether derivative per 92 grams of 95% aqueous ethanol).

Example XXXVI

During two consecutive days several felled oak trees surrounding a field of tobacco plants were treated with 250 mg of:

(a) 1,3-dimethylbutyl phenethylether prepared according to Example IV(A)

(b) isopropyl phenethylether prepared according to Example V

(c) a mixture of 2-butenyl phenethylether and 1-methallyl phenethylether prepared according to Example VI

in 1.0% ethanolic solution. These trees were exposed in an area which was contaminated with *Lasioderma serricorne (F.)*. After 3 to 4 days, 115 beetles per square meter were observed on the logs. Other untreated logs or trees in the direct neighborhood of the treated logs or trees showed very few (about 12) insects per square meter on the average while other trees at a distance of 10 to 20 meters showed no contamination.

Example XXXVII

In a large test field, mixtures of either:

(a) 1,3-dimethylbutyl phenethylether prepared according to Example IV(A)

(b) isopropyl phenethylether prepared according to Example V

(c) a mixture of 2-butentyl phenethylether and 1-methallyl phenethylether prepared according to Example VI

in admixture with different DDT preparations, fluorine-containing mixture and arsenic-containing mixtures as well as hexachloro-cyclohexane were used. These mixtures contained also small amounts of surface-active agents and carriers. The mixtures were applied to catch trees namely logs of oak trees in an area of tobacco plants contaminated with *Lasioderma serricorne (F.)*. The distance between the catch trees was always 200 meters. After 8 days there was no contamination either in the tobacco fields or around the oak trees. About 93% of the *Lasioderma serricorne (F.)*. insects were destroyed. Surprisingly, it was found that after the fourth day, the attracting effect was not diminished in spite of the dead insects being present. Furthermore, in those areas where the phenethylether derivatives of either Examples IV(A), V or VI was used alone, the average number of insects destroyed was about 78% which in itself is surprising. Thus, the phenethylether derivatives of either Examples IV(A), V or VI not only act as pheromones but also as insecticides. Furthermore, the entire area wherein the phenethylether derivatives of either Example IV(A), V or VI were used had faint, pleasant, floral aromas covering any adverse and aesthetically displeasing aromas of any other insecticides that were used.

Example XXXVIII

The following mixture is prepared:

| Ingredients | Parts by Weight |
|---|---|
| Phenylacetic acid | 70.0 |
| Coumarin | 20.0 |
| Phenylethylphenyl acetate | 100.0 |
| Phenylethyl alcohol | 5.0 |
| Benzyl benzoate | 100.0 |
| Dimethylphenylethyl carbinol | 10.0 |
| Methyl anthranilate | 5.0 |
| Beta ionone | 10.0 |

either:

(a) 1,3-dimethylbutyl phenethylether prepared according to Example IV(A) or

(b) isopropyl phenethylether prepared according to Example V; or

(c) The mixture of 2-butenyl phenethylether and 1-methallyl phenethylether prepared according to Example VI 30.0

The 1,3-dimethylbutyl phenethylether prepared according to Example (IV)A) imparts the fruity, floral, hyacinth, rose aroma to this honey fragrance while giving it a very warm undertone in imparting a very long-lasting rose top note to this fragrance.

The isopropyl phenethylether prepared according to Example V imparts the strong green, floral, fruity, hyacinth aroma with a slight peppery, mushroom undertone to this honey fragrance.

The mixture of 2-butenyl phenethylether and 1-methallyl phenethylether produced according to Example VI imparts the green, floral aroma to this honey fragrance.

Example XXXIX

*Preparation of a cosmetic powder composition*

A cosmetic powder is prepared by mixing in a ball mill 100 grams of talcum powder with 0.25

grams of one of the perfume compositions prepared according to Example XXXVIII. Each of the perfume compositions has an excellent floral aroma.

Example XL

*Perfumed liquid detergent*

Concentrated liquid detergents (lysine sale of n-dodecylbenzene sulfonic acid as more specifically described in U.S. Patent No. 3,948,818 issued on April 6, 1976) with floral aroma nuances are prepared containing 0.10%, 0.15% and 0.20% of each of the fragrances prepared according to Example XXXVIII. They are prepared by adding and homogeneously admixing the appropriate quantity of one of the three fragrance formulations prepared according to Example XXXVIII in the liquid detergents. The detergents all possess excellent floral aromas, the intensity increasing with greater concentrations of perfume composition prepared according to Example XXXVIII.

Example XLI

*Preparation of a cologne and handkerchief perfume*

The compositions prepared according to Example XXXVIII are incorporated into colognes at concentrations of 2.0%, 2.5%, 3.0%, 4.0%, 4.5% and 5.0% in 80%, 85%, 90% and 95% aqueous food grade ethanol; and into handkerchief perfumes at concentrations of 15%, 20% and 30% (in 80%, 85% and 95% aqueous food grade ethanol). Distinctive and definitive strong, floral aromas are impared to the colognes and to the handkerchief perfumes at all levels indicated.

Example XLII

*Preparation of soap composition*

100 grams of soap chips are mixed with 1 gram of each of the formulations of Example XXXVIII until homogeneous compositions are obtained. The homogeneous compositions are each heated under three atmospheres pressure at 180°C for periods of 3 hours and the resulting liquids are placed into soap molds. The resulting soap cakes, on cooling, manifest excellent floral aromas that are very long-lasting.

Example XLIII

*Preparation of a solid detergent composition*

A detergent is prepared from the following ingredients according to Example I of Canadian Patent No. 1,007,948:

| | Percent by Weight |
|---|---|
| "Neodol 45—11" (a $C_{14}$—$C_{15}$ alcohol ethoxylated with 11 moles of ethylene oxide) | 12 |
| Sodium carbonate | 55 |
| Sodium citrate | 20 |
| Sodium sulfate, water brighteners | q.s. |

This detergent is a "phosphate-free" detergent. A total of 100 grams of this detergent is admixed with 0.15 gram samples of each of the three honey based perfumes of Example XXXVIII. Each of the detergent samples has excellent floral, honey-like aromas.

Example XLIV

*Dryer-added fabric softener article*

Utilizing the procedure of Example I at column 15 of U.S. Patent No. 3,632,296, a non-woven cloth substrate useful as a dryer-added fabric softening article of manufacture is prepared wherein the substrate, the substrate coating and the outer coating and the perfuming material are as follows:

1. a water "dissolvable" paper ("Dissolvo Paper");
2. Adogen 448 (m.p. about 140°F) as the substrate coating; and
3. an outer coating having the following formulation (m.p. about 150°F):

57% $C_{20-22}$ HAPS
22% isopropyl alcohol
20% antistatic agent
1% of the phenethylether substances as set forth in Table II below.

Fabric softening compositions prepared as set forth above having aroma profiles as set forth in Table II below each consist of a substrate having a weight of about 3 grams per 100 square inches, a substrate coating of about 1.85 grams per 100 square inches of substrate and an outer coating of about 1.4

grams per 100 square inches of substrate, thereby providing a total aromatized substrate and outer coating weight ratio of about 1:1. Aromas are imparted in a pleasant manner to the head space in the dryer on operation thereof using said dryer-added fabric softening non-woven fabric.

TABLE II

| Phenethylether Derivative | Organoleptic Properties |
|---|---|
| 1,3-dimethylbutyl phenethylether produced according to Example I. | A galbanum, cassis-like, rosy, hyacinth and narcissus aroma. |
| Isopropyl phenethylether prepared according to Example II. | A strong, green, floral, fruity, hyacinth aroma with a slight peppery and mushroomy undertone with honey/hyacinth top notes. |
| Mixture of 2-butenyl phenethylether and 1-methallyl phenethylether produced according to Example III. | A green, floral aroma. |

Example XLV

A liquid detergent composition prepared according to Example IV of United Kingdom Patent No. 1,498,520 whereby the following ingredients are admixed:

| Ingredient | Weight % |
|---|---|
| Coconut alcohol ethoxylate | 30 |
| Linear alkyl benzene sulfonate, triethanolamine salt (alkyl = $C_{11.8}$ avg.) | 10 |
| Potassium chloride | 3 |
| Triethanolamine | 3 |
| Triethanolammonium citrate | 2 |
| Ethyl alcohol | 5 |
| Soil release ether "D" | 1 |
| Phenethylether derivative as set forth in Table II of Example XLIV | 3 |
| Water | Balance |

The soil release ether "D" is defined according to Table II on page 15 of United Kingdom Patent No. 1,498,520.

This composition is prepared by admixing all of the ingredients exclusive of soil release ether "D" and agitating the mixture until all electrolytes are dissolved. Soil release ether "D" is then admixed with the solution in the form of a dry powder which passes through a 150 mesh standard sieve. The resulting composition is in the liquid state and is easily pourable. The composition is found not to redden on contact with plastic bottles, does not gel with water and has an aroma as set forth in Table II of Example XLIV which lasts for several weeks when exposed to the atmosphere.

The foregoing compositions are added to an aqueous laundrying bath at concentrations of 0.20% (weight) each at a temperature of 55°C, water hardness 7 grains per gallon and a pH of 10.0. Polyester and mixed polyester/cotton fabrics are laundered in the bath for a period of 10 minutes after which the fabrics are thoroughly rinsed with fresh water and dried at ambient temperatures. The fabrics are provided with a soil release finish. The head space above the fabrics have pleasant aromas as described in Table II of Example XLIV and are also rather long-lasting (about 3 days).

Example XLVI

*Preparation of cosmetic powder compositions*

Cosmetic powders are prepared by admixing in a ball mill 100 grams of talcum powder with 0.25 grams of the phenethylether derivative as set forth in Table II of Example XLIV. The resulting cosmetic powders have aromas as set forth in Table II of Example XLIV which are very long-lasting.

Example XLVII

*Perfumed liquid detergent*

Concentrated liquid detergents (lysine salt of n-dodecylbenzene sulfonic acid as more specifically described in U.S. Patent No. 3,948,818 issued on April 6, 1976) with aromas as set forth in Table II of Example XLIV are prepared containing 0.10%, 0.15%, 0.20% and 0.25% of the phenethylether derivatives as set forth in Table II of Example XLIV. They are prepared by adding and homogeneously admixing the appropriate quantities of phenethylether derivatives in the liquid detergent. The detergents all possess intense and long-lasting aromas as set forth in Table II of Example XLIV.

Example XLVIII

*Preparation of colognes and handkerchief perfumes*

Phenethylether derivatives as set forth in Table II of Example XLIV are incorporated into colognes at concentrations of 2.0%, 2.5%, 3.0%, 3.5%, 4.0% and 4.5% in 80%, 85%, 90% and 95% aqueous food grade ethanol; and into handkerchief perfumes at concentrations of 15%, 20%, 25% and 30% (in 80%, 90% and 95% aqueous ethanol solutions). Distinctive aromas as set forth in Table II of Example XLIV which are very long-lasting on dry-out (44 hours) are imparted to the colognes and to the handkerchief perfumes at the various above levels indicated.

Example XLIX

*Tobacco formulation*

A tobacco mixture is produced by admixing the following ingredients:

| Ingredients | Parts by Weight |
|---|---|
| Bright | 40.1 |
| Burley | 24.9 |
| Maryoand | 1.1 |
| Turkish | 11.6 |
| Stem (flue-cured) | 14.2 |
| Glycerine | 2.8 |
| Water | 5.3 |

Cigarettes are prepared from this tobacco.

The following flavor formulation is prepared.

| Ingredients | Parts by Weight |
|---|---|
| Ethyl butyrate | .05 |
| Ethyl valerate | .05 |
| Maltol | 2.00 |
| Cocoa extract | 26.00 |
| Coffee extract | 10.00 |
| Ethyl alcohol | 20.00 |
| Water | 41.90 |

The above stated tobacco flavor formulation is applied at the rate of 0.1% to all of the cigarettes produced using the above tobacco formulation. Half of the cigarettes are then treated with 500 or 1000 ppm of each of the phenethylether derivatives produced according to Examples I(A)—VI. The control cigarettes not containing any of the phenethylethers produced according to the processes of Examples I(A)—VI and the experimental cigarettes which contain the phenethylethers produced according to the processes of Examples I(A)—VI are evaluated by paired comparison and the results are as follows:

The experimental cigarettes are found to have more body and to be, on smoking, more tobacco-like, more aromatic, sweet, fruity and Virginia tobacco-like with sweet, rich, honey-like and fruity nuances. The tobacco of the experimental cigarettes prior to smoking has a sweet, rich, honey-like, fruity and hay tobacco-like aroma. The products of Examples I—VI, particularly the products of Example I, enhance the tobacco-like taste and aroma of the blended cigarettes imparting to them honey-like and fruity tobacco notes.

*Brief description of the drawings*

Figure 1(A) is the GLC profile for the reaction product of Example I(A) at the end of the reaction and just prior to distillation.

Figure 1(B) is the GLC profile for fraction 8 of the distillation product of the reaction of Example I(A) which contains phenethyl cyclohexylether having the structure:

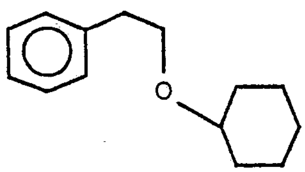

Figure 1(C) is the GLC profile for fraction 10 of the distillation product of the reaction product of Example I(A) containing the compound having the structure:

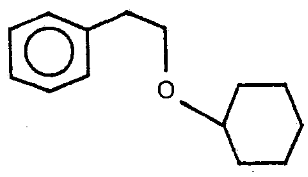

Figure 1(D) is the GLC profile for fraction 12 of the distillation product of the reaction product of Example I(A) containing the compound having the structure:

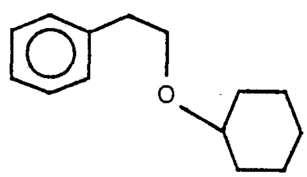

Figure 2 is the NMR spectrum for peak "A" of the GLC profile of Figure I(A) which consists of the compound having the structure:

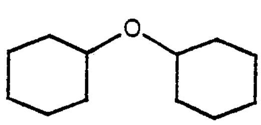

Figure 3 is the NMR spectrum for peak "B" of the GLC profile of Figure I(A) containing the compound having the structure:

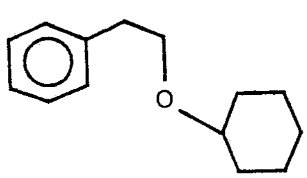

Figure 4 is the infra-red spectrum for peak "B" of the GLC profile of Figure I(A) containing the compound having the structure:

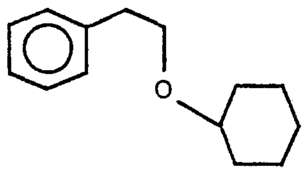

Figure 5 is the GLC profile for the reaction product of Example I(B) containing the compound having the structure:

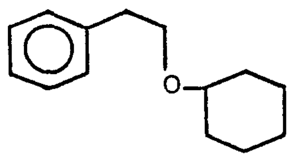

prior to distillation but subsequent to the sodium hydroxide wash of the reaction product.

Figure 6(A) is the GLC profile for fraction 7 of the distillation product of the reaction product of Example I(B) containing the compound having the structure:

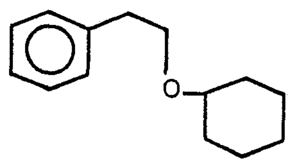

Figure 6(B) is the GLC profile for fraction 10 of the distillation product of the reaction product of Example I(B) containing the compound having the structure:

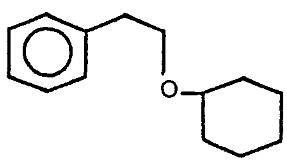

Figure 6(C) is the GLC profile for fraction 22 of the distillation product of the reaction product of Example I(B) containing the compound having the structure:

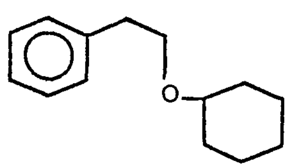

Figure 7(A) is the GLC profile of the crude reaction product prior to distillation of Example II containing the compound having the structure:

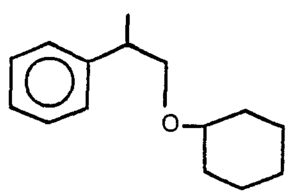

Figure 7(B) is the GLC profile for fraction 8 of the distillation product of the reaction product of Example II containing the compound having the structure:

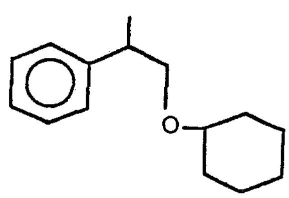

Figure 8 is the NMR spectrum for fraction 6 of the distillation product of the reaction product of Example II containing the compound having the structure:

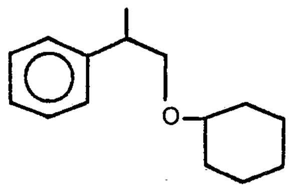

Figure 9 is the infra-red spectrum for fraction 6 of the distillation product of the reaction product of Example II containing the compound having the structure:

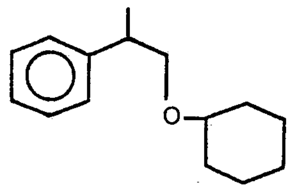

Figure 10(A) is the GLC profile for the crude reaction product prior to distillation of Example III containing the compound having the structure:

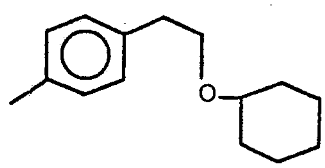

Figure 10(B) is the GLC profile for fraction 8 of the distillation product of the reaction product of Example III containing the compound having the structure:

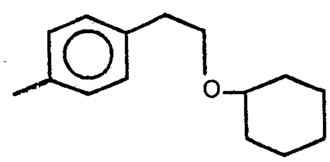

Figure 11 is the NMR spectrum for fraction 10 of the distillation product of the reaction product of Example III containing the compound having the structure:

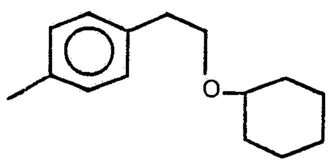

Figure 12 is the infra-red spectrum for fraction 10 of the distillation product of the reaction product of Example III containing the compound having the structure:

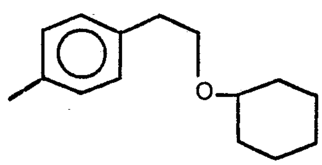

Figure 13 is the GLC profile for the crude reaction product produced according to Example IV(A) containing the compound having the structure:

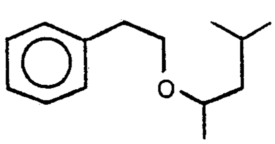

Figure 14 is the NMR spectrum for the reaction product of Example IV(A) containing the compound having the structure:

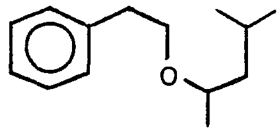

Figure 15 is the infra-red spectrum for the reaction product of Example IV(A) containing the compound having the structure:

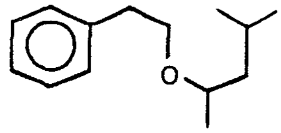

Figure 16 is the GLC profile for the crude reaction product of Example IV(B) containing the compound having the structure:

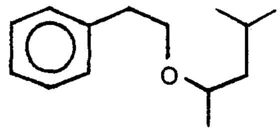

Figure 17 is the GLC profile for the crude reaction product of Example V prior to propionic anhydride reaction containing the compounds having the structures:

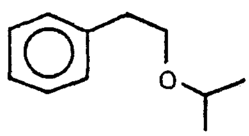

and

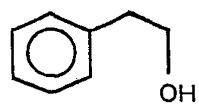

Figure 18 is the GLC profile for fraction 4 of the distillation product of the reaction product of Example V after reaction with propionic anhydride, containing the compound having the structure:

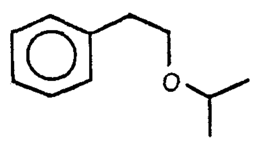

Figure 19 is the NMR spectrum for the reaction product of Example V after distillation containing the compound having the structure:

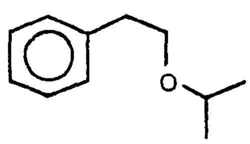

Figure 20 is the infra-red spectrum for the reaction product of Example V after distillation after reaction with propionic anhydride containing the compound having the structure:

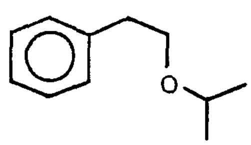

Figure 21 is the GLC profile of the reaction product of Example VI containing the compounds having the structures:

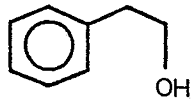 and 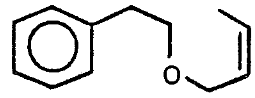 +

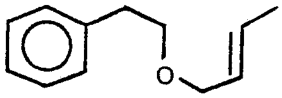 + 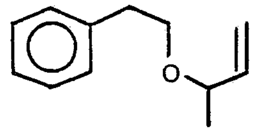

Figure 22(A) represents the NMR spectrum for peak 14 of the GLC profile of Figure 21 containing the compound having the structure:

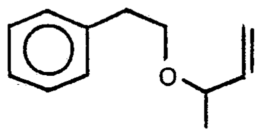

Figure 22(B) is the infra-red spectrum for peak 14 of Figure 21 which is the GLC profile for the reaction product of Example VI, containing the compound having the structure:

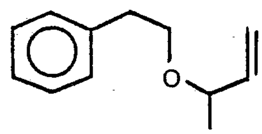

Figure 23(A) is the NMR spectrum for peak 15 of Figure 21 which is the GLC profile for the reaction product of Example VI, containing the compounds having the structures:

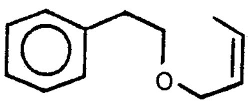 + 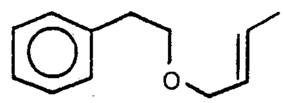

Figure 23(B) is the infra-red spectrum for peak 15 of Figure 21 which is the GLC profile for Example VI containing the compounds having the structures:

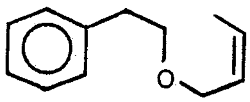 + 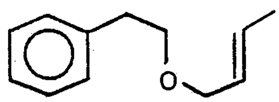

*Detailed description of Figures 5, 17 and 21*

Figure 5 is the GLC profile for the reaction product of Example I(B) resulting from the reaction of cyclohexene and phenethyl alcohol.

The peak designated by the numeral "1" is the peak indicating unreacted cyclohexene.

The peak designated by the numeral "2" in the GLC profile is the peak which represents phenylethyl alcohol.

The peak designated by the numeral "3" represents the peak for the reaction product of Example I(B), cyclohexyl phenethylether.

The conditions for this GLC profile obtention are 220°C isothermal using a 1/8″ × 10′ SE—30 column.

Figure 17 is the GLC profile for the crude reaction product of Example V of isopropanol and phenylethyl alcohol having the structure:

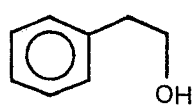

which contains the compounds phenylethyl alcohol isopropyl ether having the structure:

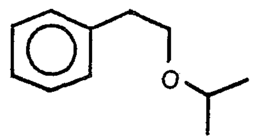

and phenylethyl alcohol having the structure:

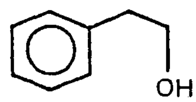

The peak indicated by the reference numeral "11" represents phenylethyl alcohol having the structure:

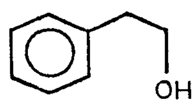

The peak indicated by the reference numeral "12" represents the phenylethyl alcohol ether having the structure:

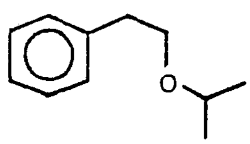

These two compounds virtually impossible to separate by distillation and require reaction with propionic anhydride followed by separation of the resultant phenylethyl alcohol isopropyl ether and phenylethyl alcohol propionate having the structure:

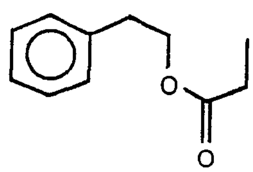

Figure 21 is the GLC profile for the reaction product of Example VI. The reaction product of Example VI contains four compounds:
phenylethyl alcohol having the structure:

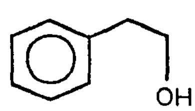

2-butenyl phenylethylether, cis-isomer and trans-isomer having the structures:

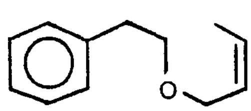 + 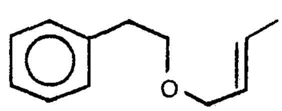

phenylethyl methallylether having the structure:

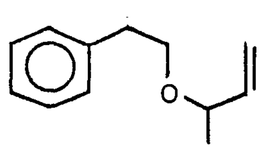

The peak on the GLC profile indicated by the reference numeral "13" is the peak for phenylethyl alcohol having the structure:

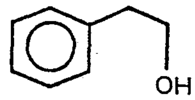

The peak on the GLC profile of Figure 21 indicated by the reference numeral "14" is the peak for the phenylethylether of methallyl alcohol having the structure:

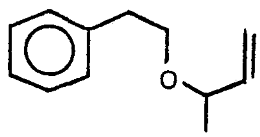

The peak on Figure 21, the GLC profile, indicated by the reference numeral "15" is the peak for the phenylethylether of 2-butenyl, both the "cis" and "trans" isomers having the structures:

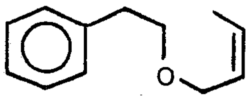

+

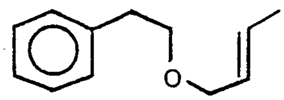

## Claims

1. A phenalkylether derivative characterized in that it is at least one phenethylether derivative having the structure:

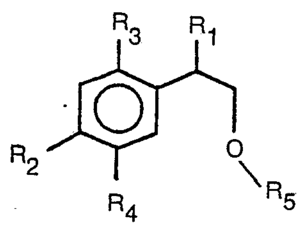

wherein $R_5$ represents $C_6$ secondary alkyl; $C_4$ alkenyl or cyclohexyl or substituted cyclohexyl defined according to the moiety:

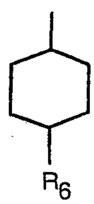

wherein $R_1$, $R_2$, $R_3$, $R_4$ and $R_6$ are the same or different and each represents methyl or hydrogen with the proviso that when one of $R_2$, $R_3$ and $R_4$ is methyl, the other two of $R_2$, $R_3$ and $R_4$ are hydrogen.

2. The phenethylether derivative of Claim 1 wherein the structure of the phenethylether is:

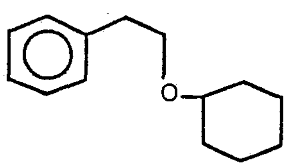

3. The phenethylether derivative of Claim 1 wherein the structure of the phenethylether is:

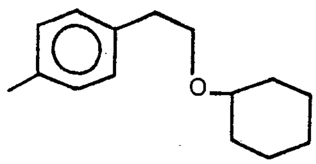

4. The process for augmenting or enhancing the aroma of a consumable material selected from the group consisting of pheromones, insecticides, perfumes, solid or liquid anionic, cationic, nonionic or zwitterionic detergents, fabric softener compositions, dryer-added fabric softener articles, cosmetic powders, and colognes comprising the step of adding to an appropriate base an aroma-augmenting or enhancing quantity of at least one compound defined according to Claim 1.

5. A method of attracting and destroying *Lasioderma serricorne (F.)* comprising applying to an area contaminated with said *Lasioderma serricorne (F.)* at least one compound defined according to Claim 1, said attractant being applied to said area in an amount sufficient to attract said *Lasioderma serricorne (F.)*.

6. The method of Claim 5 wherein the amount of said attractant applied to said area is in an amount (a) sufficient to attract said *Lasioderma serricorne (F.)* and (b) sufficient to substantially destroy said *Lasioderma serricorne (F.)* thus attracted.

7. The method of Claim 5 wherein the attractant applied to the area is in an amount (a) sufficient to attract said *Lasioderma serricorne (F.)*, (b) sufficient to destroy said *Lasioderma serricorne (F.)* substantially, and (c) sufficient to negate any adverse aromas of any other materials in said area and augment or enhance or impart a floral aroma to said area.

8. A perfume composition comprising an aroma augmenting quantity of at least one cyclohexyl phenethylether derivative defined according to the structure:

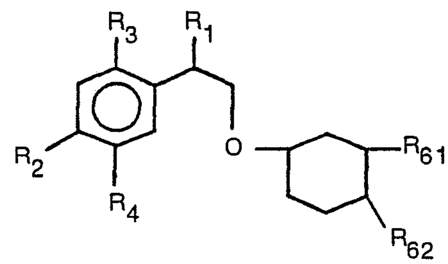

wherein $R_1$, $R_2$, $R_3$ and $R_4$ are the same or different and each represents methyl or hydrogen with the proviso that when one of $R_2$, $R_3$ and $R_4$ is methyl, the other of $R_2$, $R_3$ and $R_4$ is hydrogen and wherein $R_{61}$ and $R_{62}$ represent hydrogen or methyl with at least one of $R_{61}$ and $R_{62}$ being hydrogen; and intimately admixed therewith at least one adjuvant compatible with said cyclohexyl phenethylether derivative from an organoleptic standpoint which is selected from the group consisting of alcohols, aldehydes, ketones, nitriles, ethers other than said cyclohexyl phenethylether derivative, lactones, natural essential oils, synthetic essential oils and hydrocarbons.

9. A cologne comprising a cyclohexyl phenethylether derivative defined according to the structure:

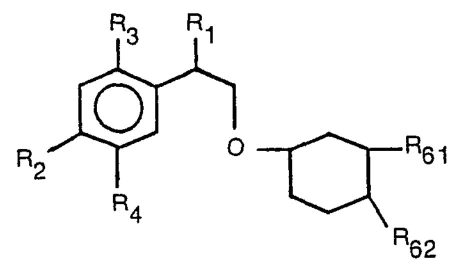

wherein $R_1$, $R_2$, $R_3$ and $R_4$ are the same or different and each represents methyl or hydrogen with the proviso that when one of $R_2$, $R_3$ and $R_4$ is methyl, the other of $R_2$, $R_3$ and $R_4$ is hydrogen and wherein $R_{61}$ and $R_{62}$ represent hydrogen or methyl with at least one of $R_{61}$ and $R_{62}$ being hydrogen and intimately admixed therewith ethanol and water.

10. A process for preparing a cyclohexyl phenethylether derivative defined according to the structure:

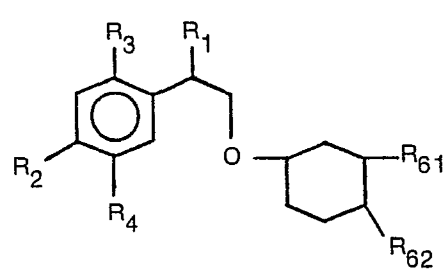

wherein $R_1$, $R_2$, $R_3$ and $R_4$ are the same or different and each represents methyl or hydrogen with the proviso that when one of $R_2$, $R_3$ and $R_4$ is methyl, the other of $R_2$, $R_3$ and $R_4$ is hydrogen and wherein $R_{61}$

and $R_{62}$ represent hydrogen or methyl with at least one of $R_{61}$ and $R_{62}$ being hydrogen, comprising the steps of intimately admixing cyclohexene having the structure:

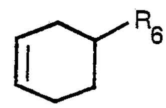

wherein $R_6$ represents $R_{61}$ or $R_{62}$, with a phenethyl alcohol derivative having the structure:

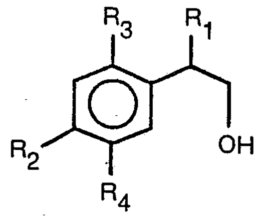

in the presence of an acid catalyst selected from the group consisting of sulfuric acid, methane sulfonic acid, paratoluene sulfonic acid, one or more acid ion exchange resins and one or more acid clays wherein the temperature of reaction is from 90°C up to 180°C; the mole ratio of phenylethyl alcohol derivative:cyclohexene is from 1:1 up to 1:2; and the weight ratio of acid catalyst:phenylethyl alcohol derivative is from 2% by weight up to 20% by weight of the reaction mass.

11. The process of Claim 10 wherein the phenylethyl alcohol derivative is phenylethyl alcohol.

12. The process of Claim 10 wherein the acid catalyst is a sulfonated copolymer of styrene and divinyl benzene ion exchange resin.

13. The process of Claim 12 wherein the phenylethyl alcohol derivative is phenylethyl alcohol.

14. The phenethylether derivative of Claim 1 wherein the phenethylether derivative has the structure:

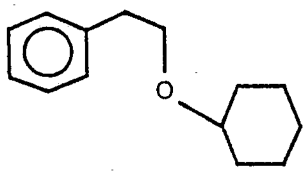

15. The process for augmenting or enhancing the aroma of a consumable material selected from the group consisting of pheromones, insecticides, perfumes, solid or liquid anionic, cationic, nonionic or zwitterionic detergents, fabric softener compositions, dryer-added fabric softener articles, cosmetic powders, and colognes comprising the step of adding to an appropriate base an aroma-augmenting or enhancing quantity of the compound of Claim 14

16. A method of attracting and destroying *Lasioderma serricorne (F.)* comprising applying to an area contaminated with said *Lasioderma serricorne (F.)* the compound of Claim 14 said attractant being applied to said area in an amount sufficient to attract said *Lasioderma serricorne (F.)*.

17. The method of Claim 16, wherein the amount of said attractant applied to said area is in an amount (a) sufficient to attract said *Lasioderma serricorne (F.)* and (b) sufficient to substantially destroy said *Lasioderma serricorne (F.)* thus attracted.

18. The method of Claim 16 wherein the attractant applied to the area is in an amount (a) sufficient to attract said *Lasioderma serricorne (F.)*, (b) sufficient to destroy said *Lasioderma serricorne (F.)* substantially, and (c) sufficient to negate any adverse aromas of any other materials in said area and augment or enhance or impart a floral aroma to said area.

19. The perfume composition comprising an aroma augmenting or enhancing quantity of cyclohexyl phenethylether having the structure:

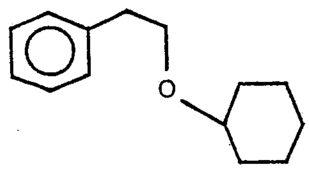

and intimately admixed therewith at least one adjuvant compatible therewith from an organoleptic standpoint which is selected from the group consisting of alcohols, aldehydes, ketones, nitriles, ethers other than said cyclohexyl phenethylether, lactones, natural essential oils, synthetic esential oils and hydrocarbons.

20. A cologne comprising cyclohexyl phenethylether having the structure:

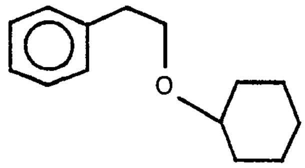

and intimately admixed therewith ethanol and water.

21. The process for augmenting or enhancing the aroma of a consumable material selected from the group consisting of pheromones, insecticides, perfumes, solid or liquid anionic, cationic, nonionic or zwitterionic detergents, fabric softener compositions, dryer-added fabric softener articles, cosmetic powders, and colognes comprising the step of adding to an appropriate base an aroma-augmenting or enhancing quantity of at least one phenethylether defined according to the structure:

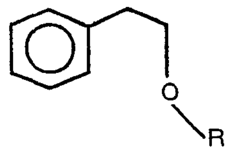

wherein R represents $C_6$ 2°-alkyl or $C_4$ alkenyl.

22. A method of attracting and destroying *Lasioderma serricorne (F.)* comprising applying to an area contaminated with said *Lasioderma serricorne (F.)* at least one phenethylether defined according to the structure:

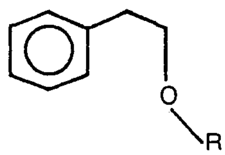

wherein R is $C_3$ or $C_6$ 2°-alkyl or $C_4$ alkenyl, said attractant being applied to said area in an amount sufficient to attract said *Lasioderma serricorne (F.)*.

23. The method of Claim 22 wherein the amount of said attractant applied to said area is in an amount (a) sufficient to attract said *Lasioderma serricorne (F.)* and (b) sufficient to substantially destroy said *Lasioderma serricorne (F.)* thus attracted.

24. The method of Claim 22 wherein the attractant applied to the area is in an amount (a) sufficient to attract said *Lasioderma serricorne (F.)*, (b) sufficient to destroy said *Lasioderma serricorne (F.)* substantially, and (c) sufficient to negate any adverse aromas of any other materials in said area and augment or enhance or impart a floral aroma to said area.

25. A perfume composition comprising an aroma augmenting or enhancing quantity of at least one phenethylether defined according to the structure:

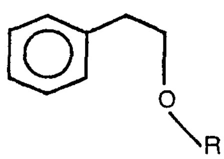

wherein R represents $C_6$ 2°-alkyl or $C_4$ alkenyl, and intimately admixed therewith at least one adjuvant compatible therewith from an organoleptic standpoint which is selected from the group consisting of alcohols, aldehydes, ketones, nitriles, ethers other than said phenethylethers, lactones, natural essential oils, synthetic essential oils and hydrocarbons.

26. A cologne comprising at least one phenethylether defined according to the structure:

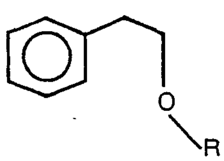

wherein R represents $C_6$ 2°-alkyl or $C_4$ alkenyl, and intimately admixed therewith ethanol and water.

27. A smoking tobacco composition comprising smoking tobacco and intimately admixed therewith at least one phenethylether derivative defined according to Claim 1.

**Revendications**

1. Un dérivé phénalkyléther, caractérisé en ce que, c'est au moins un dérivé phénéthyléther possédant la structure

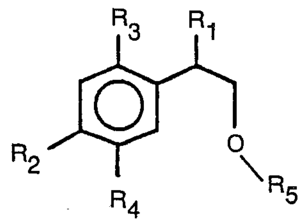

où $R_5$ représente un alkyle secondaire en $C_6$, un alcényle en $C_4$ ou un cyclohexyle ou un cyclohexyle substitué défini selon la formule:

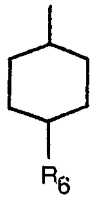

dans lequel $R_1$, $R_2$, $R_3$, $R_4$ et $R_6$ sont identiques ou différents et chacun représente un méthyle ou un hydrogène avec la réserve que, quand l'un des $R_2$, $R_3$ et $R_4$ est un méthyle, les deux autres groupes parmi $R_2$, $R_3$ et $R_4$ sont de l'hydrogène.

2. Le dérivé phénéthyléther est:

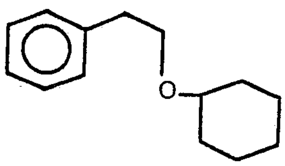

3. Le dérivé phénéthyléther dans lequel la structure du phénéthyléther est:

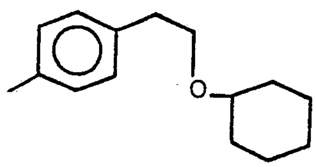

4. Procédé pour augmenter ou exalter l'arôme d'un bien consommable choisi dans le groupe constitué des phéromones, insecticides, parfums, détergents solide ou liquide, anionique, cationique, non ionique ou amphotère, compositions d'adoucissant textile, articles adoucissants textiles ajoutés au séchage, poudres cosmétiques et eaux de cologne, comprenant l'étape d'addition à une base appropriée d'une quantité d'au moins l'un des composés définis selon la revendication 1, augmentant ou exaltant l'arôme.

5. Méthode pour attirer ou détruire le *Lasioderma serricorne (F)* comprenant l'application à une aire contaminée par ledit *Lasioderma serricorne (F)* d'au moins un composé défini selon la revendication 1, ledit agent attirant étant appliqué à ladite aire en quantité suffisante pour attirer ledit *Lasioderma serricorne (F).*

6. Méthode selon la revendication 5, dans laquelle la quantité de l'agent attirant appliqué à ladite aire est en quantité (a) suffisante pour attirer ledit *Lasioderma serricorne (F)* et (b) suffisante pour sensiblement détruite ledit *Lasioderma serricorne* (F) ainsi attiré.

7. Méthode selon la revendication 5 dans laquelle l'agent attirant appliqué dans l'aire est en quantité (a) suffisante pour attirer ledit *Lasioderma serricorne (F),* (b) suffisante pour détruire ledit *Lasioderma serricorne (F)* sensiblement, et (c) suffisante pour annuler tout autre arôme adverse de n'importe quel autre matériau dans ladite aire et pour augmenter ou exalter ou donner un arôme floral à ladite aire.

8. Composition parfumée comprenant une quantité augmentant l'arôme d'au moins un dérivé cyclohexylphénéthyléther défini selon la structure:

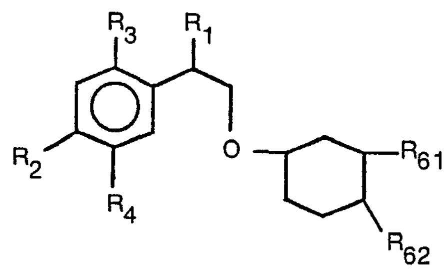

dans laquelle $R_1$, $R_2$, $R_3$ et $R_4$ sont les mêmes ou différents et chacun représente un méthyle ou un hydrogène avec la réserve que quand l'un des $R_2$, $R_3$ et $R_4$ est un méthyle, l'autre parmi $R_2$, $R_3$ et $R_4$ est l'hydrogène et dans laquelle $R_{61}$ et $R_{62}$ représentent un hydrogène ou un méthyle avec au moins l'un des deux $R_{61}$ et $R_{62}$ étant l'hydrogène; et intimement mélangé avec au moins un adjuvant compatible avec ledit dérivé cyclohexylphénéthyléther d'un point de vue organoleptique qui est choisi dans le groupe constitué des alcools, aldéhydes, cétones, nitriles, éthers autres que ledit dérivé cyclohexylphénéthyléther, lactones, huiles essentielles naturelles, huiles essentielles synthétiques et hydrocarbures.

9. Une eau de cologne comprenant un dérivé cyclohexylphénéthyléther défini selon la structure:

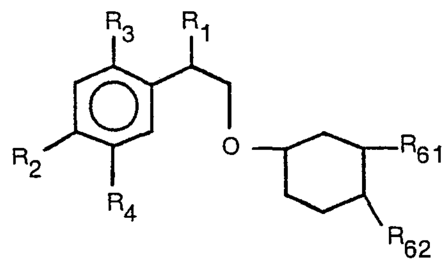

dans laquelle $R_1$, $R_2$, $R_3$ et $R_4$ sont les mêmes ou différents et chacun représente un méthyle ou un hydrogène avec la réserve que quand l'un des $R_2$, $R_3$ et $R_4$ est un méthyle, l'autre parmi $R_2$, $R_3$ et $R_4$ est l'hydrogène et dans laquelle $R_{61}$ et $R_{62}$ représentent un hydrogène ou un méthyle avec au moins l'un des deux $R_{61}$ et $R_{62}$ étant l'hydrogène et intimement mélangé avec de l'éthanol et de l'eau.

10. Procédé de préparation d'un dérivé cyclohexylphénéthyléther défini selon la structure:

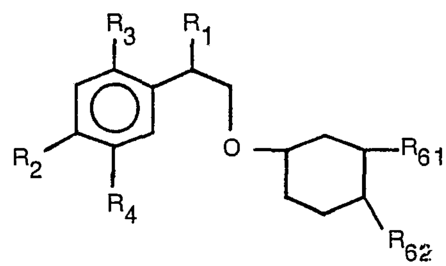

dans laquelle $R_1$, $R_2$, $R_3$ et $R_4$ sont les mêmes ou différents et chacun représente un méthyle ou un hydrogène avec la réserve que quand l'un des $R_2$, $R_3$ et $R_4$ est un méthyle, l'autre parmi $R_2$, $R_3$ et $R_4$ est l'hydrogène et dans laquelle $R_{61}$ et $R_{62}$ représentent un hydrogène ou un méthyle avec au moins l'un des deux $R_{61}$ et $R_{62}$ étant l'hydrogène comprenant l'étape d'intimement mélanger à un cyclohexène qui a la structure:

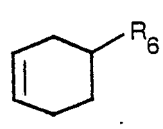

dans laquelle $R_6$ représente $R_{61}$ ou $R_{62}$, avec un dérivé du phényl qui a la structure:

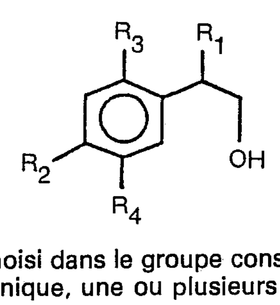

en présence d'un catalyseur acide choisi dans le groupe constitué de l'acide sulfurique, l'acide méthane sulfonique, l'acide paratoluène sulfonique, une ou plusieurs résines acides échangeuses d'ions et une

ou plusieurs argiles acides où la température de réaction est comprise entre 90°C et 180°C; le rapport molaire du dérivé phényléthanol: cyclohexène varie entre 1:1 et 1:2; et le rapport pondéral du catalyseur acide: dérivé phényléthanol varie entre 2% en poids et 20% en poids de la masse réactionnelle.

11. Procédé selon la revendication 10 dans lequel le dérivé du phényléthanol est le phényléthanol.

12. Procédé selon la revendication 10 dans lequel le catalyseur acide est une résine échangeuse d'ions copolymère sulfoné du styrène et du divinylbenzène.

13. Procédé selon la revendication 12 dans lequel le dérivé phényléthanol est le phényléthanol.

14. Dérivé phénéthyléther selon la revendication 1 dans lequel le dérivé phénéthyléther a la structure:

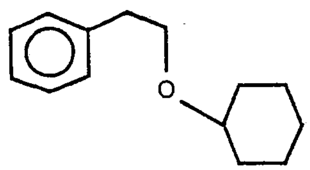

15. Procédé pour augmenter ou exalter l'arôme d'un bien consommable choisi dans le groupe constitué des phéromones, insecticides, parfums, détergents solide ou liquide, anionique, cationique, non ionique ou amphotère, compositions d'adoucissant textile, articles adoucissants, textiles ajoutés au séchage, poudres cosmétiques et eaux de cologne, comprenant l'étape d'addition à une base appropriée d'une quantité du composé défini selon la revendication 14 augmentant ou exaltant l'arôme.

16. Méthode pour attirer ou détruire le *Lasioderma serricorne (F)* comprenant l'application à une aire contaminée par ledit *Lasioderma serricorne (F)* du composé défini selon la revendication 14, ledit agent attirant étant appliqué à ladite aire en quantité suffisante pour attirer ledit *Lasioderma serricorne (F)*.

17. Méthode selon la revendication 16 dans laquelle la quantité de l'agent attirant appliqué à ladite aire est en quantité (a) suffisante pour attirer ledit *Lasioderma serricorne (F)* et (b) suffisante pour sensiblement détruire ledit *Lasioderma serricorne (F)* ainsi attiré.

18. Méthode selon la revendication 16 dans laquelle l'agent attirant appliqué dans l'aire est en quantité (a) suffisante pour attirer ledit *Lasioderma serricorne (F)*, (b) suffisante pour détruire ledit *Lasioderma serricorne (F)* sensiblement et, (c) suffisante pour annuler tout autre arôme adverse de n'importe quel autre matériau dans ladite aire et pour augmenter ou exalter ou donner un arôme floral à ladite aire.

19. Composition parfumée comprenant une quantité augmentant ou exaltant l'arôme de cyclohexylphénéthyléther défini selon la structure:

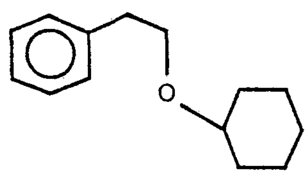

et intimement mélangé avec au moins un adjuvant compatible avec lui d'un point de vue organoleptique qui est choisi dans le groupe constitué des alcools, aldéhydes, cétones, nitriles, éthers autres que ledit cyclohexylphénéthyléther, lactones, huiles essentielles naturelles, huiles essentielles synthétiques et hydrocarbures.

20. Une eau de cologne comprenant du cyclohexylphénéthyléther ayant la structure:

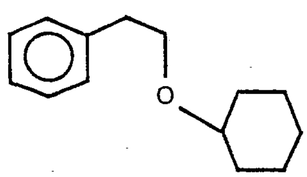

intimement mélangé à de l'éthanol et de l'eau.

21. Procédé pour augmenter ou exalter l'arôme d'un bien consommable choisi dans le groupe constitué des phéromones, insecticides, parfums, détergents solide ou liquide, anionique, cationique, non ionique ou amphotère, compositions d'adoucissant textile, articles adoucissants textiles ajoutés au séchage, poudres cosmétiques et eaux de cologne, comprenant l'étape d'addition à une base appropriée d'une quantité d'au moins un phénéthyléther augmentant ou exaltant l'arôme, défini selon la structure:

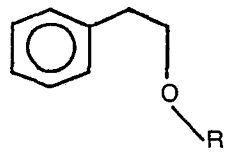

dans laquelle R représente un alkyle secondaire en $C_6$ ou un alcényle en $C_4$.

22. Méthode pour attirer ou détruire le *Lasioderma serricorne (F)* comprenant l'application à une aire contaminée par ledit *Lasioderma serricorne (F)* d'au moins un phénéthyléther selon la structure:

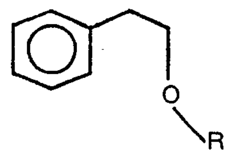

dans laquelle R est un alkyle secondaire en $C_6$ ou alcényle en $C_4$, ledit agent attirant étant appliqué à ladite aire en quantité suffisante pour attirer ledit *Lasioderma serricorne (F).*

23. Méthode selon la revendication 22 dans laquelle la quantité de l'agent attirant appliqué à ladire aire est en quantité (a) suffisante pour attirer ledit *Lasioderma serricorne (F)* et (b) suffisante pour sensiblement détruite ledit *Lasioderma serricorne (F)* ainsi attiré.

24. Méthode selon la revendication 22 dans laquelle l'agent attirant appliqué dans l'aire est en quantité (a) suffisante pour attirer ledit *Lasioderma serricorne (F),* (b) suffisante pour détruire ledit *Lasioderma serricorne (F)* sensiblement, et (c) suffisante pour annuler tout autre arôme adverse de n'importe quel autre matériau dans ladite aire et pour augmenter ou exalter ou donner un arôme floral à ladite aire.

25. Composition parfumée comprenant une quantité augmentant l'arôme d'au moins un phénéthyléther défini selon la structure:

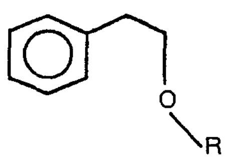

dans laquelle R représente un alkyle secondaire en $C_6$ ou un alcényle en $C_4$ ou un alcényle en $C_4$ et est intimement mélangé avec au moins un adjuvant compatible avec lui d'un point de vue organoleptique qui est choisi dans le groupe constitué des alcools, aldéhydes, cétones, nitriles, éthers autres que lesdits phényéthyléthers, lactones, huiles essentielles naturelles, huiles essentielles synthétiques et hydrocarbures.

26. Eau de cologne comprenant au moins un phénéthyléther défini selon la structure:

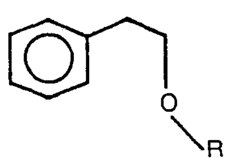

dans laquelle R représente un alkyle secondaire en $C_6$ ou un alcényle en $C_4$, et dans laquelle il est intimement mélangé à de l'éthanol et de l'eau.

27. Composition de tabac à fumer comprenant un tabac à fumer et intimement mélangé avec lui au moins un dérivé phénéthyléther défini selon la revendication 1.

**Patentansprüche**

1. Phenylalkyletherderivat, dadurch gekennzeichnet, daß es mindestens ein Phenyletherderivat mit der Struktur ist:

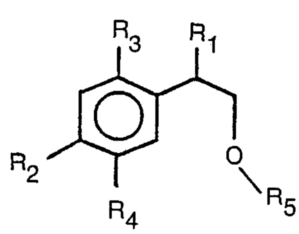

wobei $R_5$ einen sekundären $C_6$-Alkylrest; $C_4$ einen Alkenyl oder Cyclohexyl oder substituierten Cyclohexylrest, definiert durch folgendes:

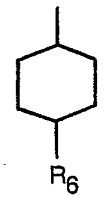

repräsentiert, wobei $R_1$, $R_2$, $R_3$, $R_4$ und $R_6$ gleich oder unterschiedlich sind und jeweils Methyl oder Wasserstoff repräsentieren, wobei dann, wenn eine der $R_2$, $R_3$ und $R_4$ Methyl ist, die beiden anderen Reste aus $R_2$, $R_3$ und $R_4$ Wasserstoff sind.

2. Phenethyletherderivat nach Anspruch 1, wobei die Phenethylether-Struktur folgende ist:

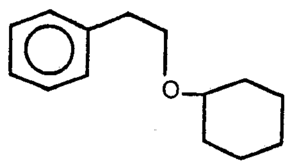

3. Phenethyletherderivat gemäß Anspruch 1, wobei die Strukturformel des Phenethylethers wie folgt ist:

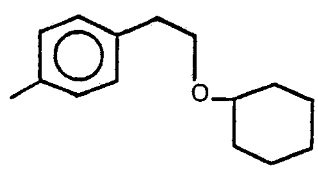

4. Verfahren zum Verbessern oder Verstärken des Aromas eines verzehrbaren Materials, ausgewählt aus der Gruppe bestehend aus Pheromonen, Insektiziden, Parfums, festen oder flüssigen anionischen, kationischen, nicht-ionischen oder zwitterionischen Detergenzien, Gewebeweichmacherzusammensetzungen, in Trocknern einsetzbare Gewebeweichmacherartikel, kosmetischen Pudern, und Eau de Cologne, welches den Schritt der Zugabe einer das Aroma verbessernden oder verstärkenden Menge mindestens einer der gemäß Anspruch 1 definierten Verbindungen aufweist.

5. Verfahren zum Anlocken und Vernichten von Lasioderma serricorne (F.), welches das Anwenden in einem mit der Lasioderma serricorne (F.) befallenen Bereich von mindestens einer Verbindung, wie sie in Anspruch 1 definiert ist, wobei der Lockstoff in diesem Bereich in einer derartigen Menge angewendet wird, die hinreichend ist, um die Lasioderma serricorne (F.) anzulocken, aufweist.

6. Verfahren nach Anspruch 5, wobei die in dem Bereich angewandte Menge Lockstoff in einer Menge vorhanden ist, die a) hinreichend ist, um die Lasioderma serricorne (F.) anzulocken und b) hinreichend ist, um im wesentlichen die derart angelockten Lasioderma serricorne (F.) zu vernichten.

7. Verfahren nach Anspruch 5, wobei der auf dem Bereich aufgebrachte Lockstoff in einer Menge vorliegt, die a) hinreichend ist, um Lasioderma serricorne (F.) anzulocken, b) hinreichend ist, um im wesentlichen die Lasioderma serricorne (F.) zu vernichten, und c) hinreichend, um unerwünschte Aromen anderer Materialien in dem Bereich zu übertönen und diesem Bereich ein florales Aroma mitzuteilen, dieses zu verstärken oder zu verbessern.

8. Parfumzusammensetzung, welche eine das Aroma verbessernde Menge mindestens eines Cyclohexylphenethyletherderivats, definiert durch die Struktur:

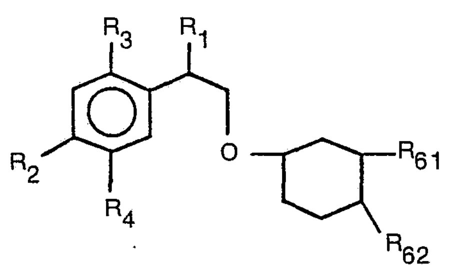

aufweist, wobei $R_1$, $R_2$, $R_3$ und $R_4$ gleich oder unterschiedlich sind und jedes Methyl oder Wasserstoff repräsentiert, vorausgesetzt, daß dann, wenn eine Rest aus $R_2$, $R_3$ und $R_4$ Methyl ist, der andere aus $R_2$, $R_3$ und $R_4$ Wasserstoff ist, und wobei $R_{61}$ und $R_{62}$ Wasserstoff oder Methyl repräsentieren, wobei mindestens ein Rest aus $R_{61}$ und $R_{62}$ Wasserstoff ist; wobei innig damit mindestens ein mit dem Cyclohexylphenethyletherderivat organoleptischen Standpunkt aus kompatibler Hilfsstoff vermischt ist, der ausgewählt ist aus der Gruppe bestehend aus Alkoholen, Aldehyden, Ketonen, Nitrilen, anderen Ethern als Cyclohexylphenethyletherderivaten, Laktonen, natürlichen ätherischen, Ölen, synthetischen ätherischen-Ölen und Kohlenwasserstoffen.

9. Eau de Cologne, welches ein Cyclohexylphenethyletherderivat, definiert durch folgende Struktur aufweist:

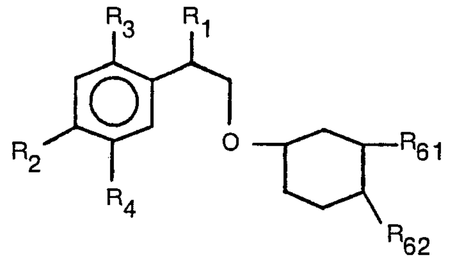

wobei $R_1$, $R_2$, $R_3$ und $R_4$ gleich oder unterschiedlich sind und jedes Methyl oder Wasserstoff repräsentiert, vorausgesetzt, daß dann, wenn ein Rest aus $R_2$, $R_3$ und $R_4$ Methyl ist, der andere aus $R_2$, $R_3$ und $R_4$ Wasserstoff ist, und wobei $R_{61}$ und $R_{62}$ für Wasserstoff oder Methyl steht, wobei mindestens ein Rest aus $R_{61}$ und $R_{62}$ Wasserstoff ist und dieses innig mit Ethanol oder Wasser vermischt wird.

10. Verfahren zum Herstellen eines Cyclohexylphenethyletherderivats, welches durch die Struktur definiert ist:

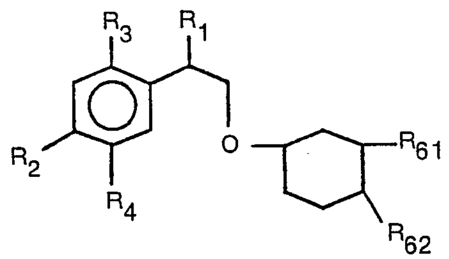

wobei $R_1$, $R_2$, $R_3$ und $R_4$ gleich oder unterschiedlich sind und jedes für Methyl oder Wasserstoff steht, vorausgesetzt, daß wenn ein Rest aus $R_2$, $R_3$ und $R_4$ Methyl ist, der andere aus $R_2$, $R_3$ und $R_4$ Wasserstoff ist, und wobei $R_{61}$ und $R_{62}$ für Wasserstoff oder Methyl stehen, wobei mindestens einer aus $R_{61}$ und $R_{62}$ Wasserstoff ist; welches die Schritte des innigen Vermischens von Cyclohexan mit der Struktur:

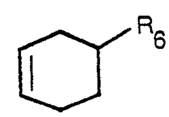

wobei $R_6$ für $R_{61}$ oder $R_{62}$ steht, mit einem Phenethylalkoholderivat folgender Struktur:

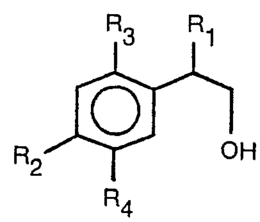

in Gegenwart eines Säurekatalysators, ausgewählt aus der Gruppe bestehend aus Schwefelsäure, Methansulfonsäure, Paratoluolsulfonsäure, einem oder mehreren Säurenionenaustauscherharzen und einem oder mehreren sauren Tone, wobei die Temperatur der Reakton zwischen 90°C bis zu 180°C liegt; das Molverhältnis von Phenylethylalkoholderivat zu Cyclohexen sich von 1:1 bis zu 1:2 erstreckt; und das Gewichtsverhältnis des sauren Katalysators zu Phenylethylalkoholderivat sich zwischen 2 Gew.-% bis zu 20 Gew.-% der Reaktionsmasse bewegt, aufweist.

11. Verfahren nach Anspruch 10, wobei das Phenethylalkoholderivat Phenylethylalkohol ist.

12. Verfahren nach Anspruch 10, wobei der Säurekatalysator ein sulfoniertes Copolymer von Styrol und Divinylbenzolionenaustauscherharz ist.

13. Verfahren nach Anspruch 12, wobei das Phenethylalkoholderivat Phenylethylalkohol ist.

14. Phenethyletherderivat gemäß Anspruch 1, wobei das Phenethyletherderivat die Struktur besitzt:

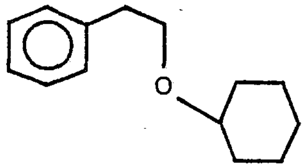

15. Verfahren zum Verstärken oder Verbessern des Aromas eines verzehrbaren Materials ausgewählt aus der Gruppe bestehend aus Pheromonen, Insektiziden, Parfums, festen oder flüssigen anionischen, kationischen, nicht-ionischen oder zwitterionischen Detergenzien, Gewebeweichmacherzusammensetzungen, in Trocknern einsetzbare Gewebeweichmacherartikel, kosmetischen Pudern, und Eau de Cologne, welches den Schritt der Zugabe einer das Aroma verbessernden oder verstärkenden Menge der Verbindung des Anspruches 14 zu einer geeigneten Basis aufweist.

16. Verfahren zum Anlocken und Vernichten von Lasioderma serricorne (F.), welches das Aufbringen der Verbindung gemäß Anspruch 14 in einem von Lasioderma serricorne (F.) befallenen Bereich aufweist, wobei der Lockstoff auf dem Bereich in einer hinreichenden Menge aufgebracht wird, um Lasioderma serricorne (F.) anzulocken.

17. Verfahren nach Anspruch 16, wobei die auf die Menge aufgebrachte Menge Lockstoff eine Menge ist, die a) hinreichend ist, um die Lasioderma serricorne (F.) anzulocken und b) hinreichend ist, um im wesentlichen die derart angelockte Lasioderma serricorne (F.) zu vernichten.

18. Verfahren nach Anspruch 16, wobei der in dem Bereich angewandte Lockstoff in einer Menge vorliegt, die a) hinreichend ist, um Lasioderma serricorne (F.) anzulocken, b) hinreichend ist, um die Lasioderma serricorne (F.) im wesentlichen zu vernichten, und c) hinreichend ist, um unerwünschte Aromen anderer Materialien in dem Bereich zu übertönen und diesem Bereich ein florales Aroma mitzuteilen, dieses zu verstärken oder zu verbessern.

19. Parfumzusammensetzung, welche eine das Aroma verbessernde oder verstärkende Menge von Cyclohexylphenethylether folgenden Struktur aufweist:

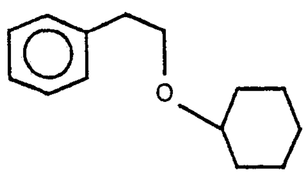

wobei innig damit ein mit diesem vom organoleptischen Standpunkt aus verträglicher Hilfsstoff vermischt ist, der ausgewählt ist aus der Gruppe bestehend aus Alkoholen, Aldehyden, Ketonen, Nitrilen, anderen Ethern als dem Cyclohexylphenethylether, Laktonen, natürlichen ätherischen Ölen, synthetischen ätherischen Ölen und Kohlenwasserstoff.

20. Eau de Cologne, welches Cyclohexylphenethylether folgender Struktur aufweist:

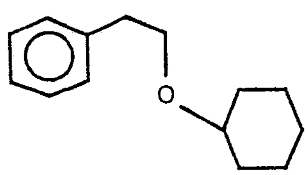

dem innig mit Ethanol und Wasser vermischt ist.

21. Verfahren zum Verbessern oder Verstärken des Aromas eines verzehrbaren Materials, ausgewählt aus der Gruppe bestehend aus Pheromonen, Insektiziden, Parfums, flüssigen oder festen anionischen, kationischen, nicht-ionischen oder zwitterionischen Detergenzien, Gewebeweichmacherzusammensetzungen, in Trocknern einsetzbare Gewebeweichmacherartikeln, kosmetischen Pudern, und Eau de Colognes, welches den Schritt der Zugabe einer das Aroma verbessernden oder verstärkenden Menge mindestens eines Phenethylethers, definiert durch folgende Struktur:

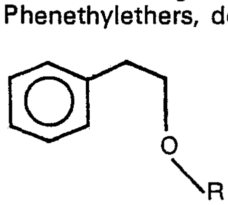

wobei R eine sekundäre $C_6$ Alkylgruppe oder $C_4$-Alkenylgruppe repräsentiert, zu einer geeigneten Basis aufweist.

22. Verfahren zum Anlocken und Vernichten von Lasioderma serricorne (F.), welches das Aufbringen mindestens eines durch die Struktur definierten Phenethylethers:

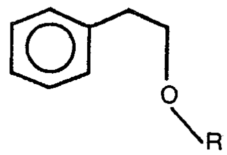

in einem mit der Lasioderma serricorne (F.) befallenen Bereich aufweist, wobei R eine sekundäre $C_3$ oder $C_6$ Alkylgruppe oder eine $C_4$ Alkenylgruppe ist, wobei der Lockstoff auf die Fläche in einer hinreichenden Menge aufgebracht wird, um Lasioderma serricorne (F.) anzulocken.

23. Verfahren nach Anspruch 22, wobei die auf die Fläche aufgebrachte Menge Lockstoff in einer Menge vorliegt, die a) hinreichend ist, um Lasioderma serricorne (F.) anzulocken und b) hinreichend ist, um im wesentlichen derart angelockte Lasioderma serricorne (F.) zu vernichten.

24. Verfahren nach Anspruch 22, wobei der in dem Bereich angewandte Lockstoff in einer Menge vorliegt, die a) hinreichend ist, um die Lasioderma serricorne (F.) anzulocken, b) hinreichend ist, um die Lasioderma serricorne (F.) im wesentlichen zu vernichten, und c) hinreichend ist, um irgendwelche unerwünschten Aromen anderer Materialien in dem Bereich zu übertönen und dem Bereich ein florales Aroma mitzuteilen, dieses zu verstärken oder zu verbessern.

25. Parfumzusammensetzung, welche eine das Aroma verbessernde oder verstärkende Menge mindestens eines Phenethylethers, definiert durch die Struktur:

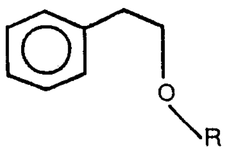

aufweist, wobei R einen sekundären $C_6$ Alkylrest oder einen $C_4$ Alkenylrest repräsentiert und mit diesem mindestens ein vom organoleptischen Standpunkt aus verträglicher Hilfsstoff innig vermischt ist, der ausgewählt ist aus der Gruppe bestehend aus Alkoholen, Aldehyden, Ketonen, Nitrilen, anderen Ethern als den Phenethylethern, Laktonen, natürlichen ätherischen Ölen, synthetischen ätherischen Ölen und Kohlenwasserstoffen.

26. Eau de Cologne, welches mindestens einen Phenethylether, definiert durch die Struktur:

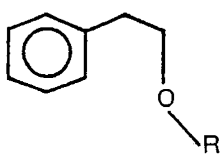

aufweist, wobei R einen sekundären $C_6$ Alkylrest oder einen $C_4$ Alkenylrest bedeutet, und dieser innig mit Ethanol und Wasser vermischt ist.

27. Rauchtabakzusammensetzung, welche einen Rauchtabak und mindestens ein gemäß Anspruch 1 definiertes Phenethyletherderivat, innig mit diesem vermischt, aufweist.

FIG.1A

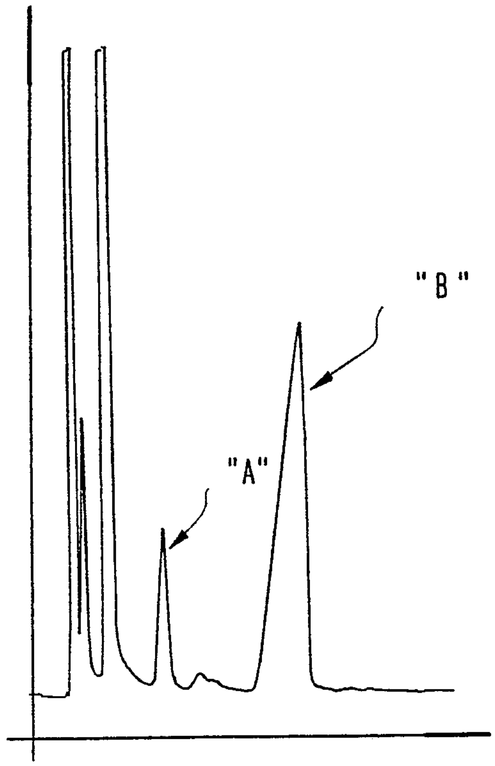


FIG.1B

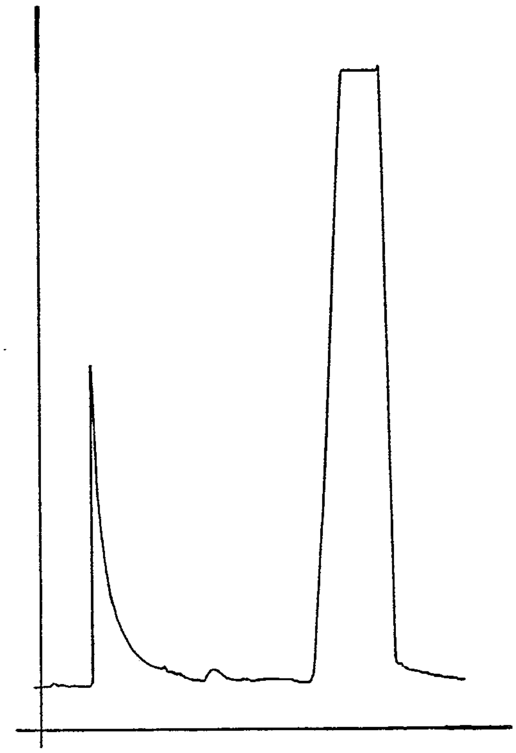

FIG.1C

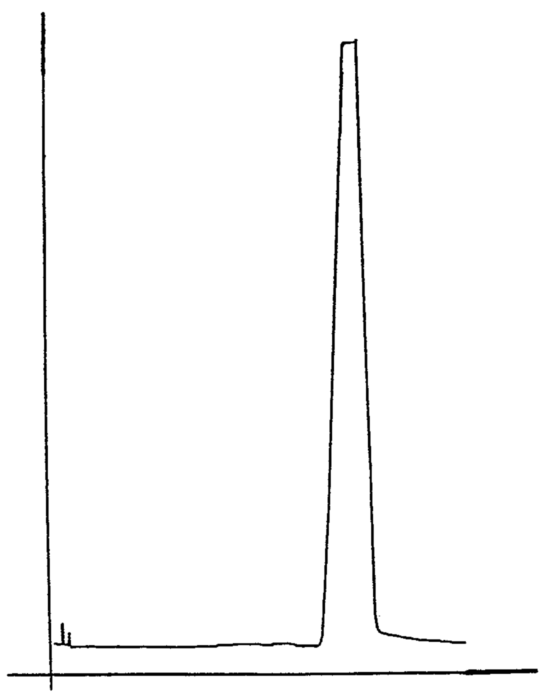

FIG.1D

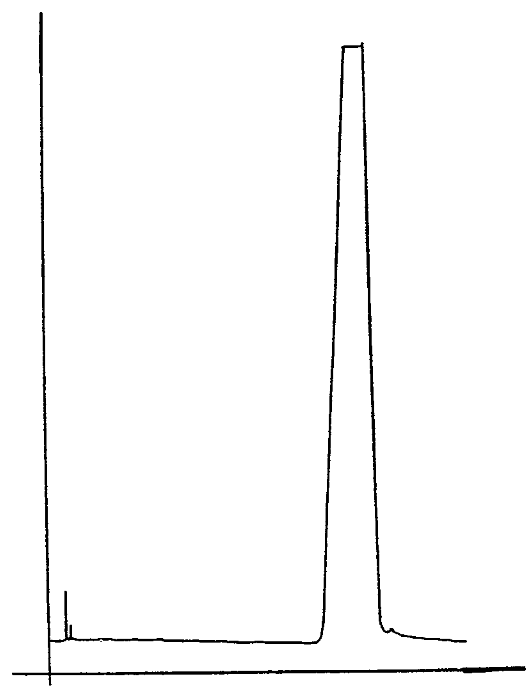

FIG. 2

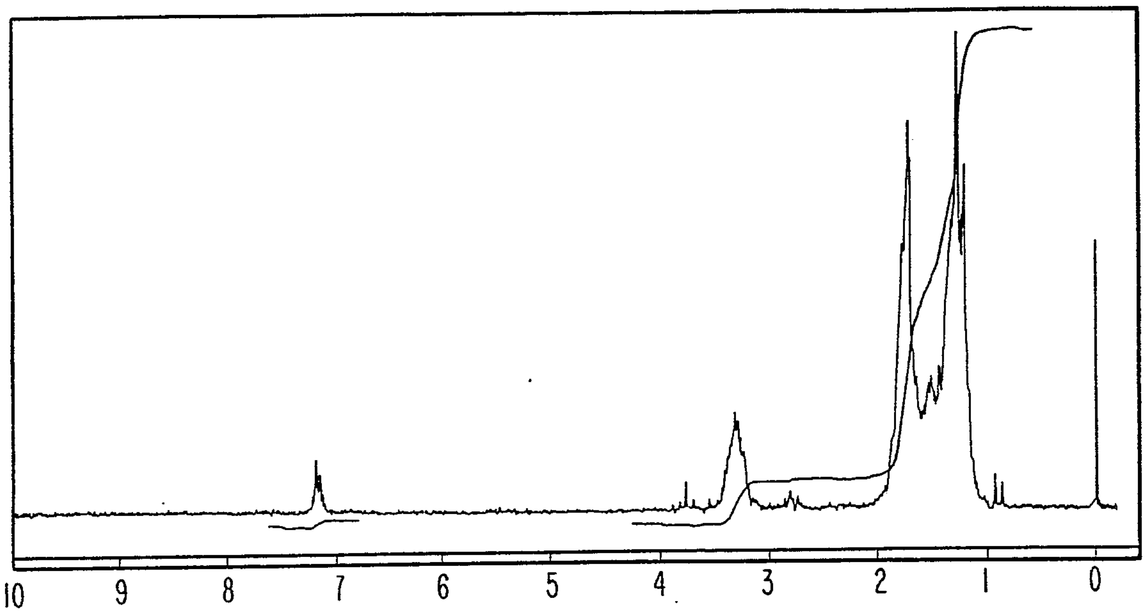


FIG. 3

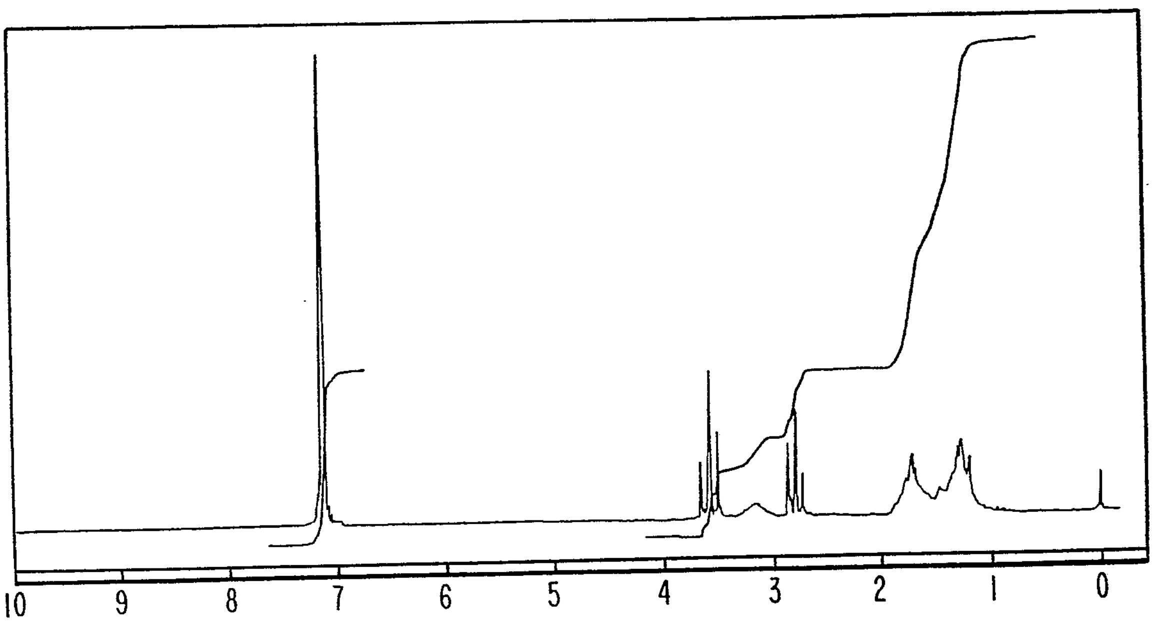

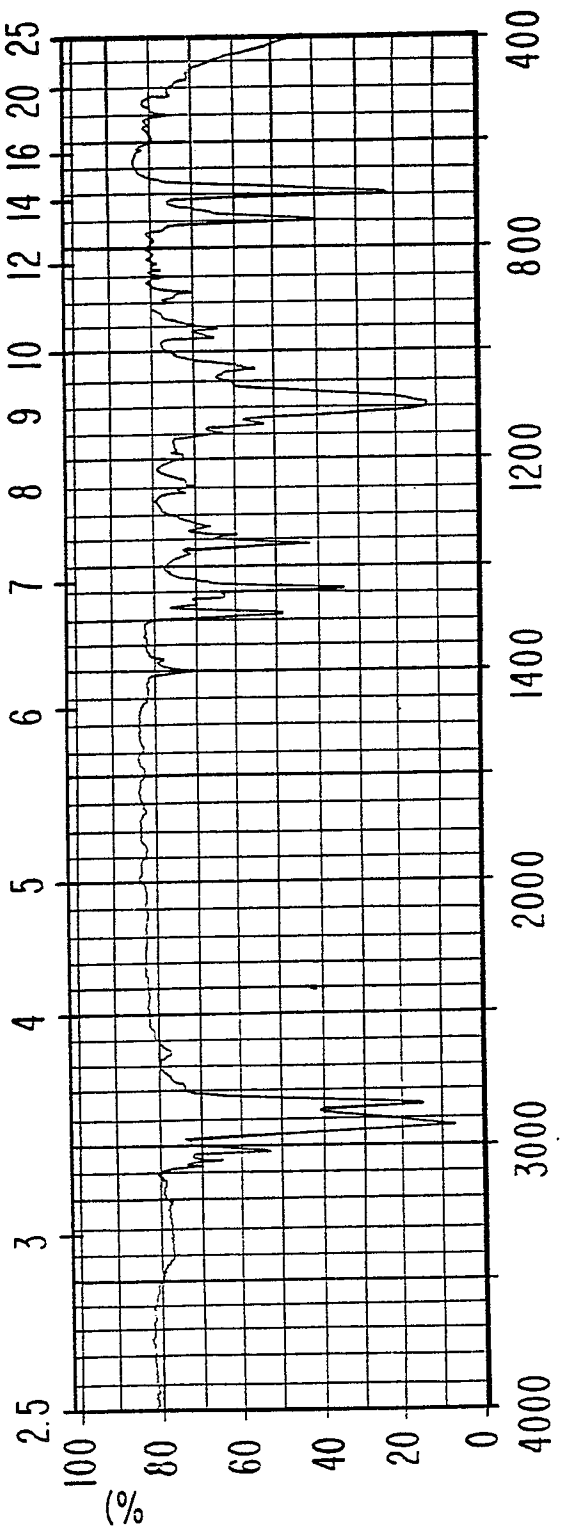
2.5
3
4
5
6
7
8
9
10
12
14
16
20
25
4000
3000
2000
1400
1200
800
400
100
80
60
40
20
0
(%

FIG. 4

FIG.5

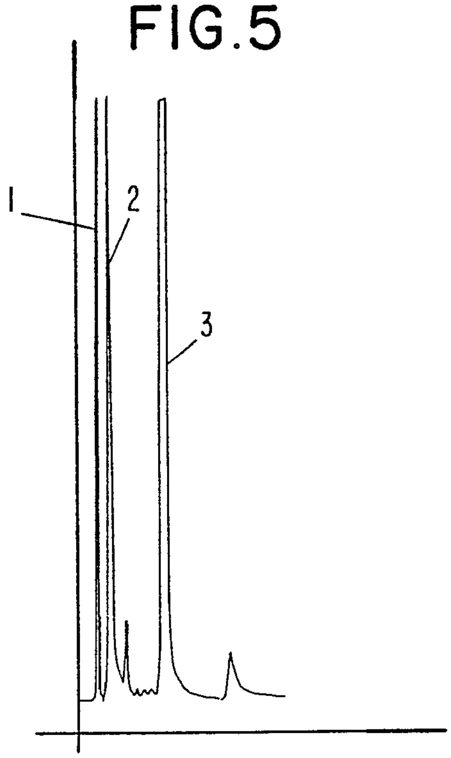


FIG.6A

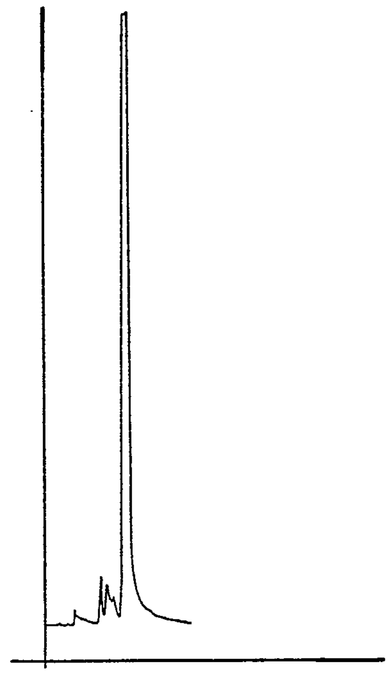

FIG.6B

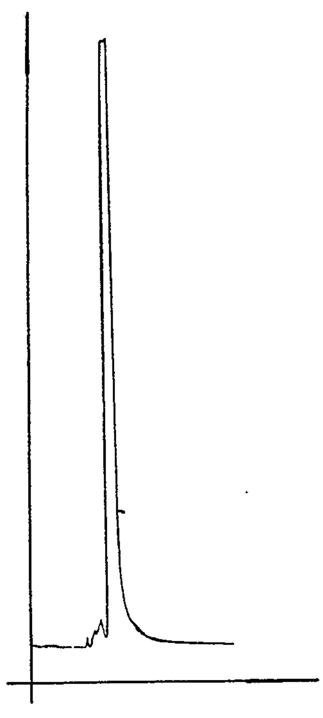

FIG.6C

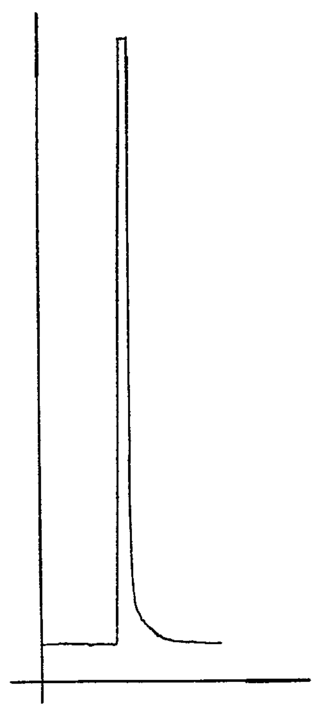

FIG.7B

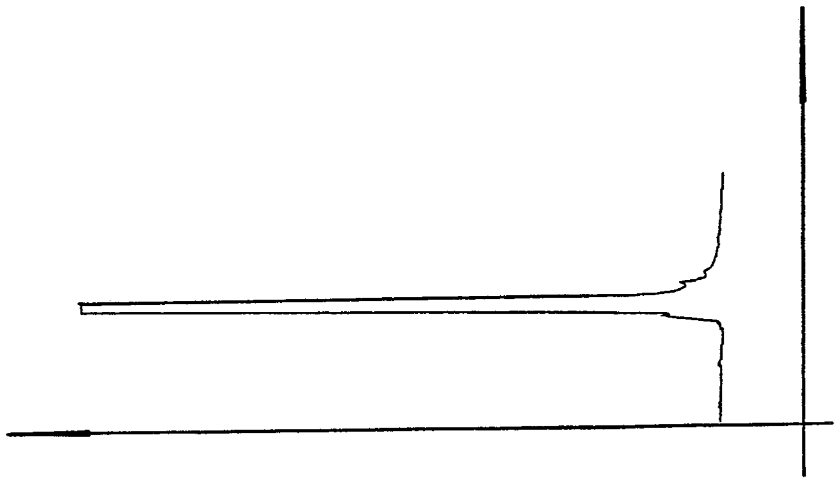

FIG.7A

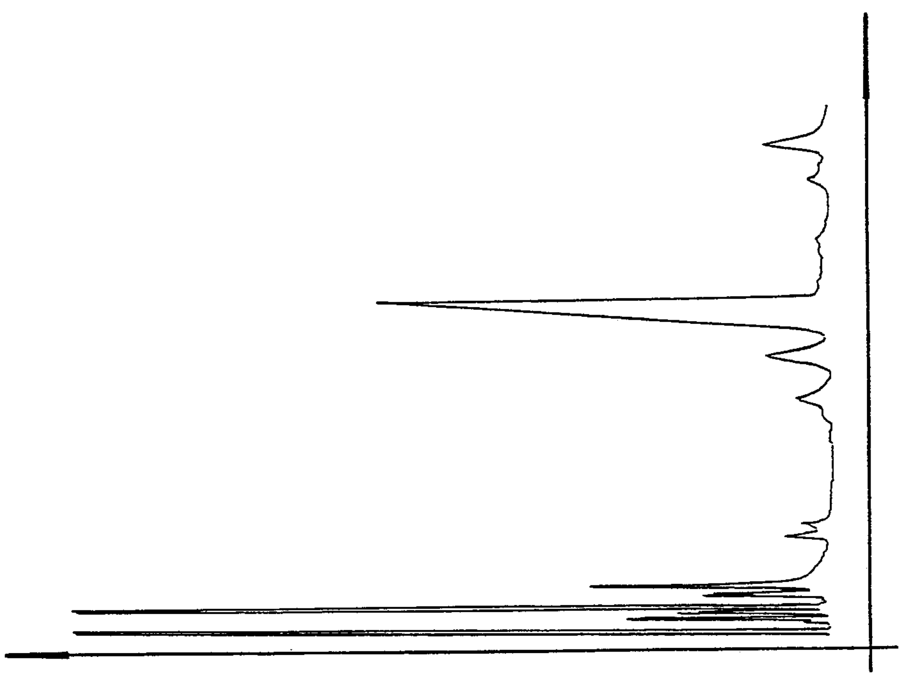

FIG. 8

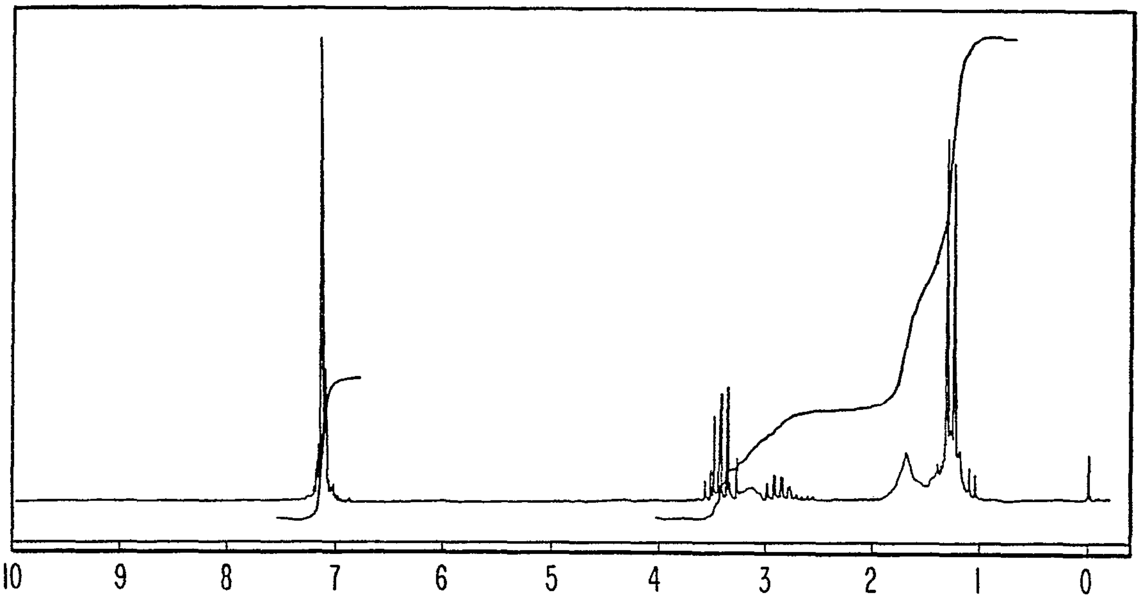
10
9
8
7
6
5
4
3
2
1
0

FIG. 9

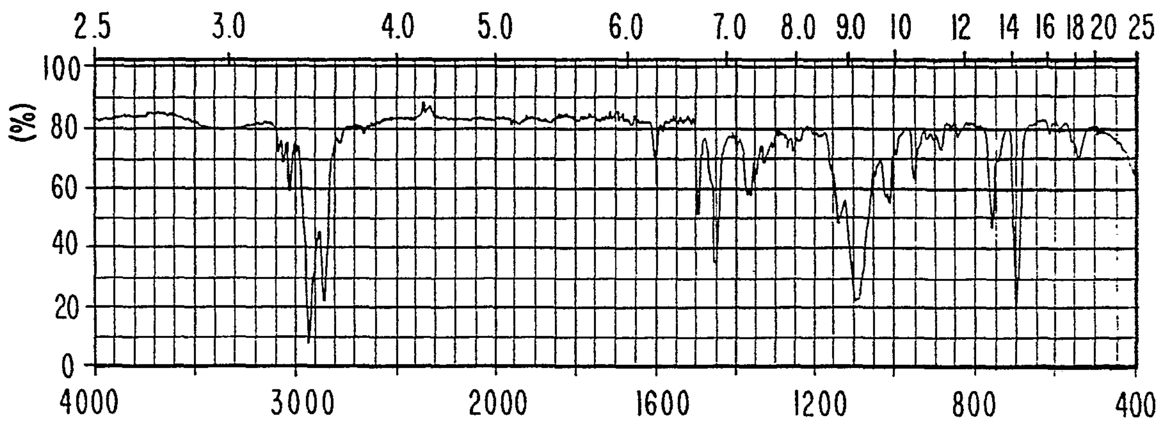
2.5
3.0
4.0
5.0
6.0
7.0
8.0
9.0
10
12
14
16
18
20
25
(%)
100
80
60
40
20
0
4000
3000
2000
1600
1200
800
400

FIG.10B

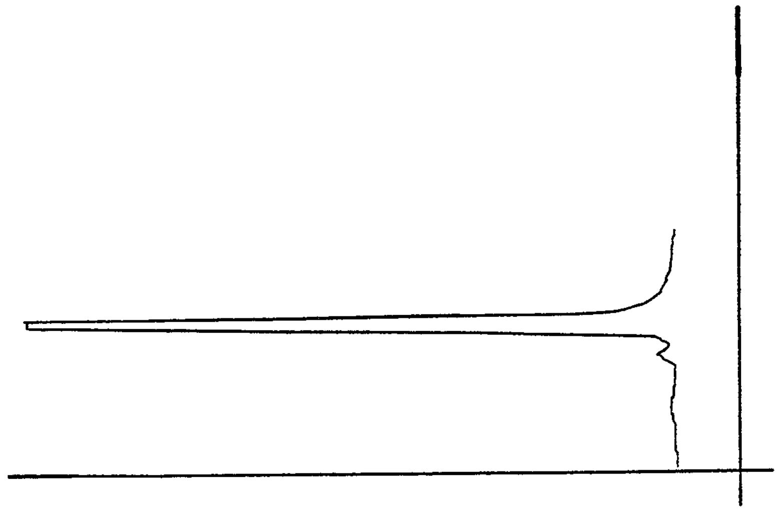

FIG.10A

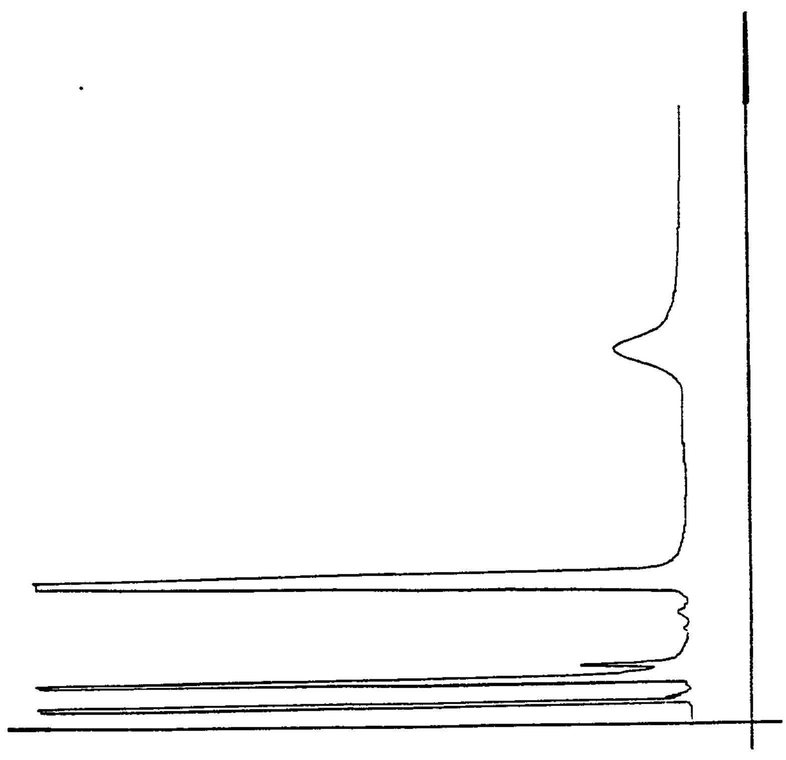

FIG.11

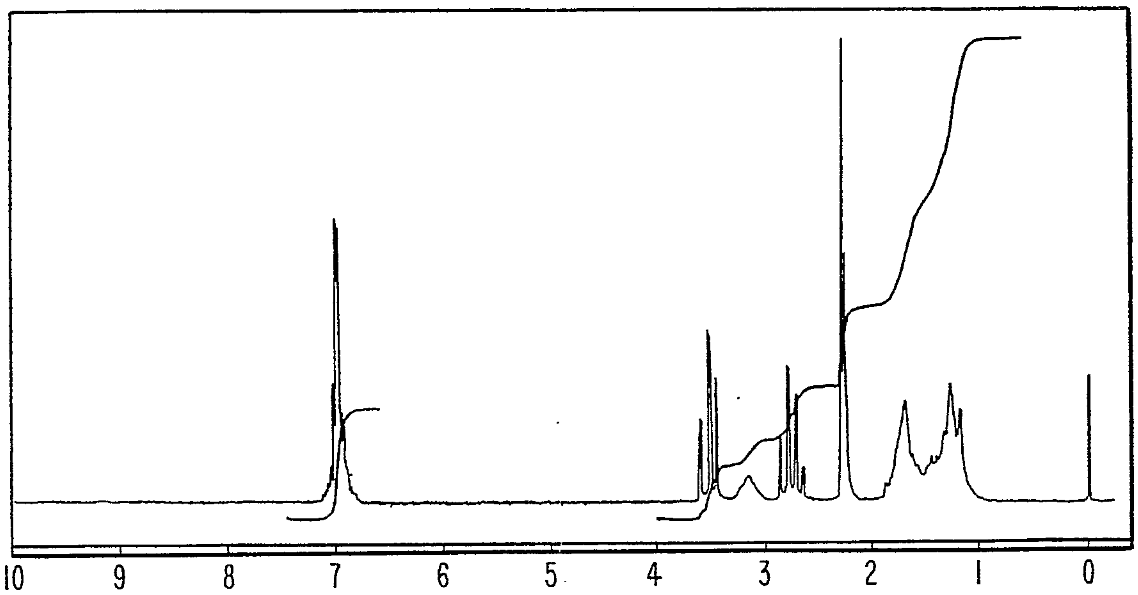
10
9
8
7
6
5
4
3
2
1
0

FIG.12

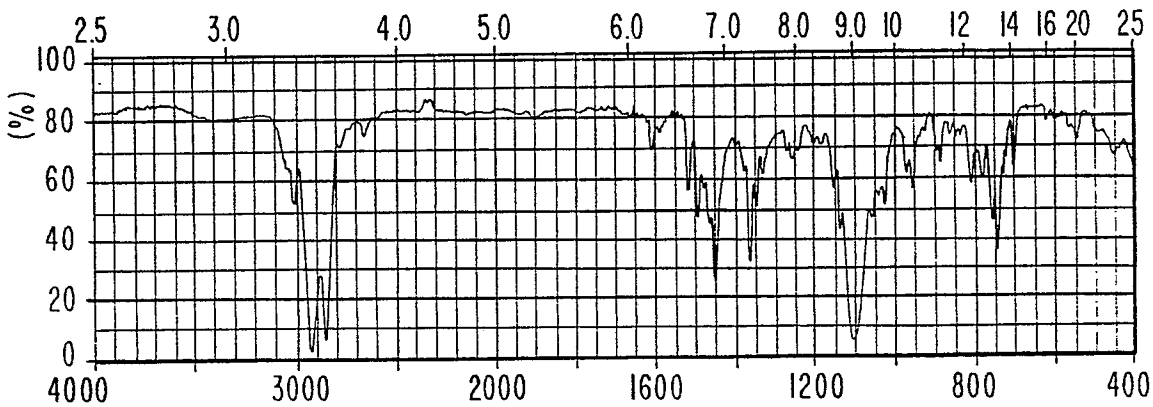
2.5
3.0
4.0
5.0
6.0
7.0
8.0
9.0
10
12
14
16
20
25
100
80
60
40
20
0
(%)
4000
3000
2000
1600
1200
800
400

FIG.16

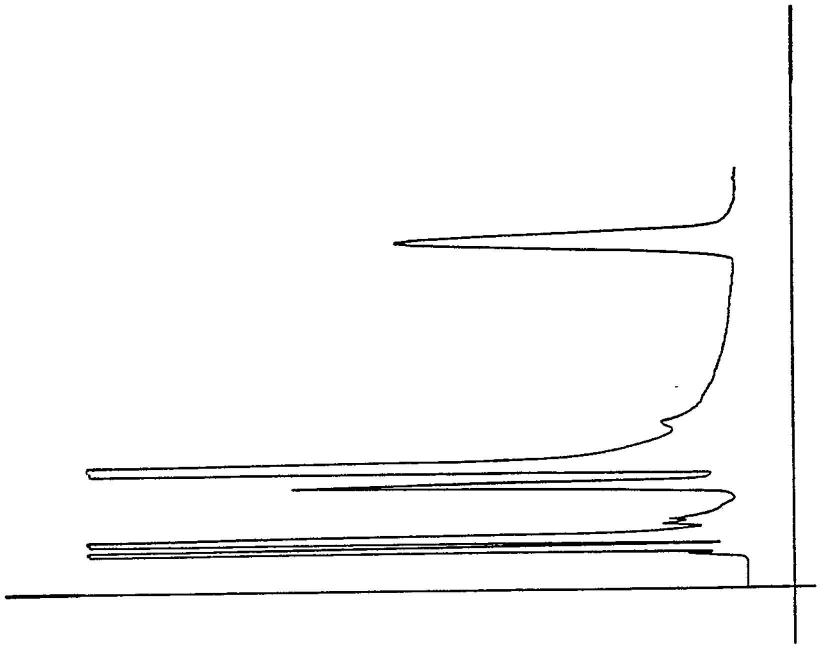

FIG.13

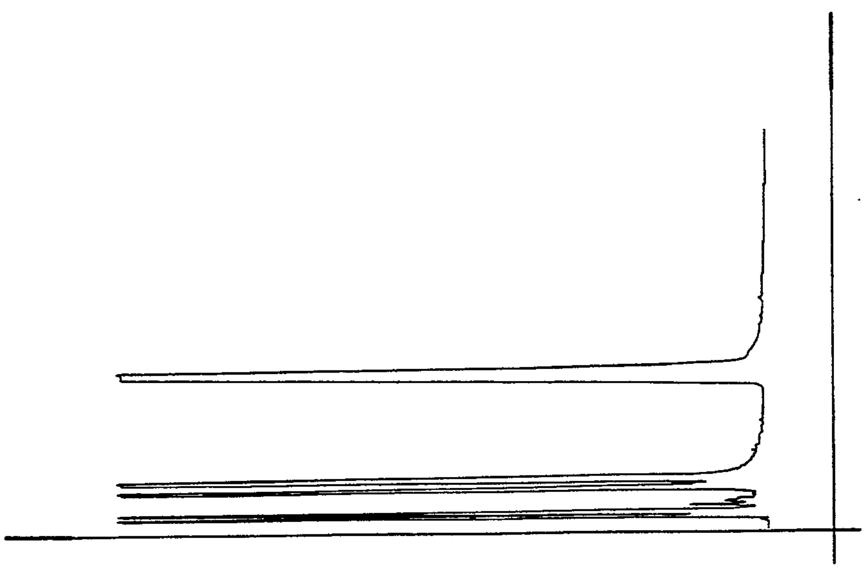

FIG. 14

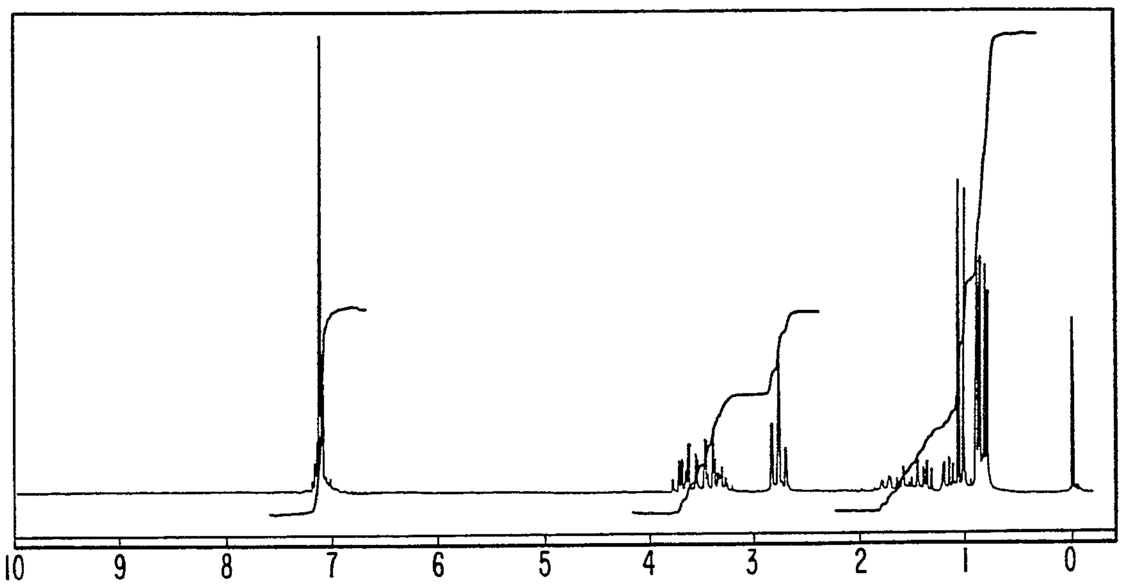
10
9
8
7
6
5
4
3
2
1
0

FIG. 15

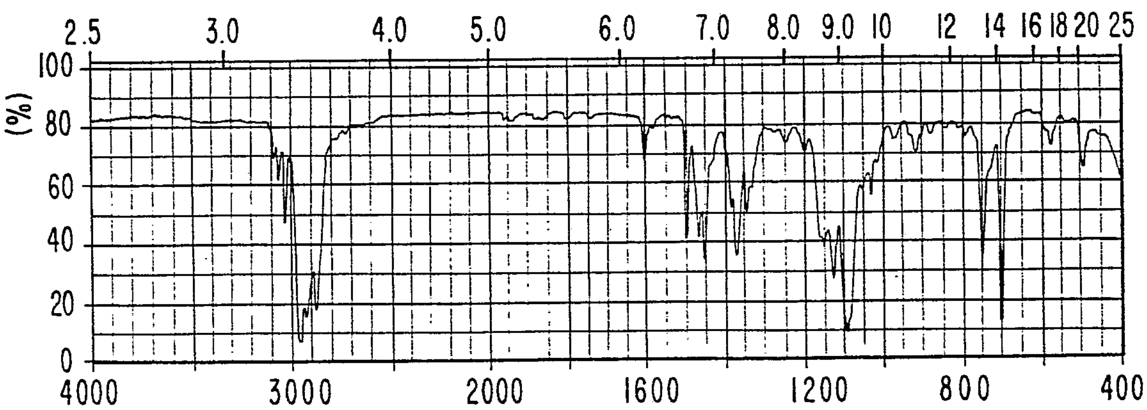
2.5
3.0
4.0
5.0
6.0
7.0
8.0
9.0
10
12
14
16
18
20
25
(%)
100
80
60
40
20
0
4000
3000
2000
1600
1200
800
400

FIG.18

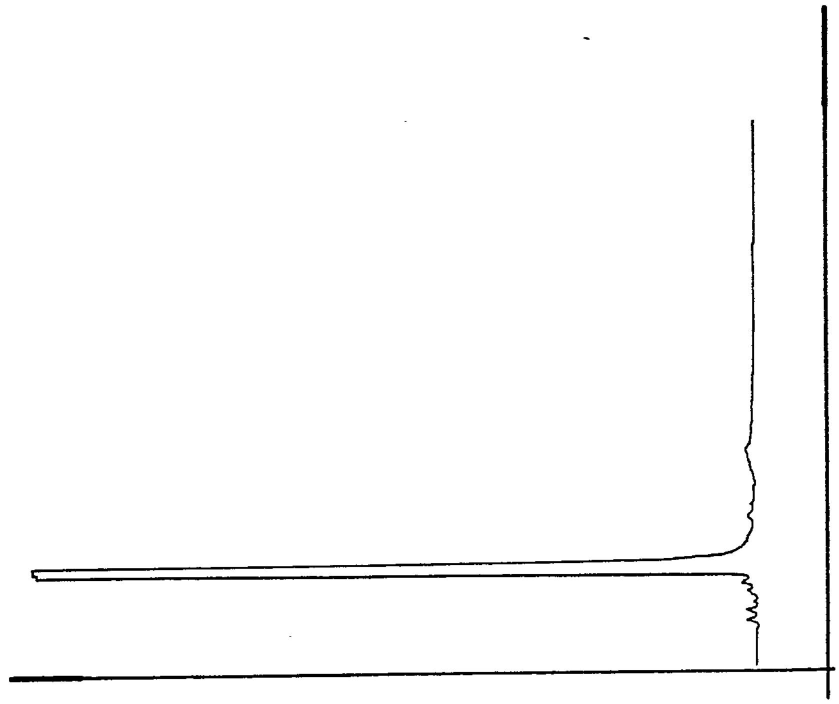

FIG. 17

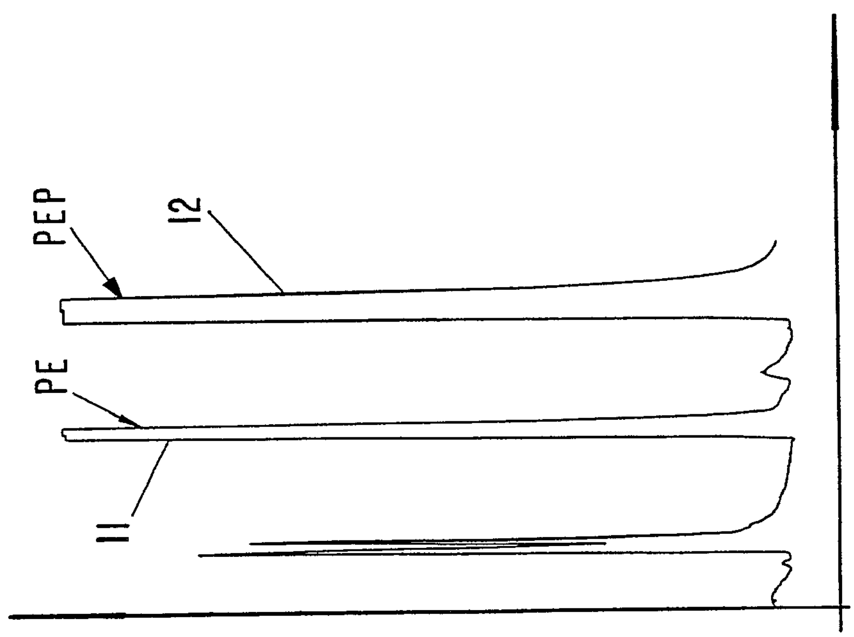

FIG.19

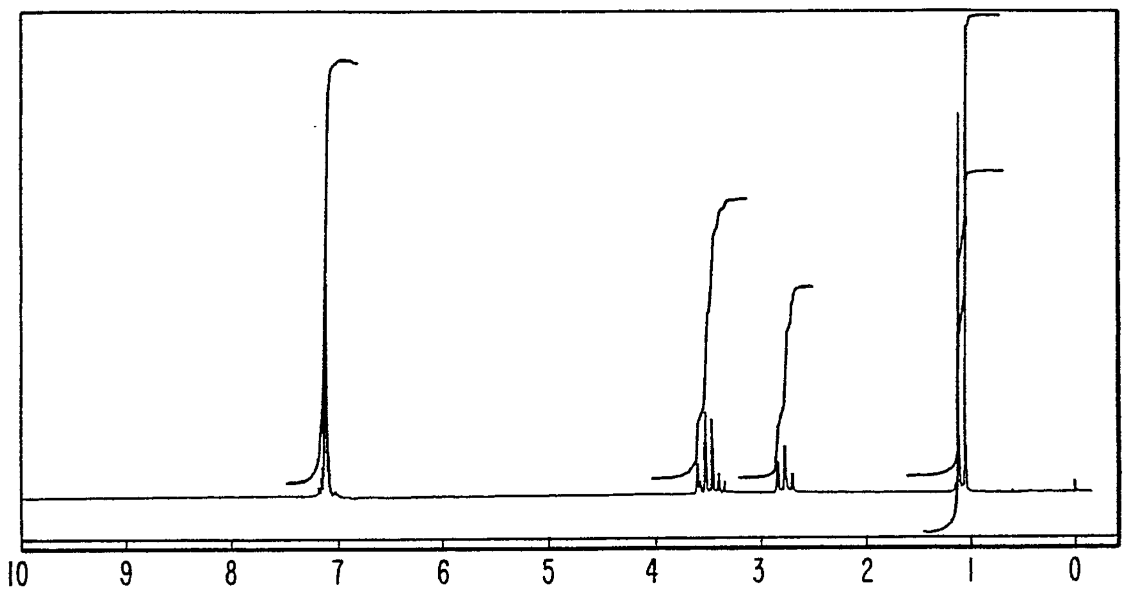
10
9
8
7
6
5
4
3
2
1
0

FIG.20

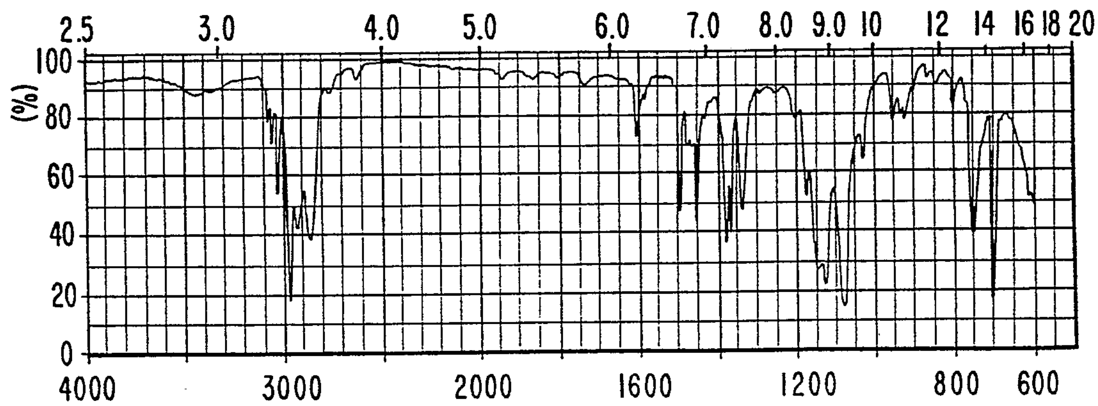
2.5
3.0
4.0
5.0
6.0
7.0
8.0
9.0
10
12
14
16
18
20
100
(%)
80
60
40
20
0
4000
3000
2000
1600
1200
800
600

FIG. 21

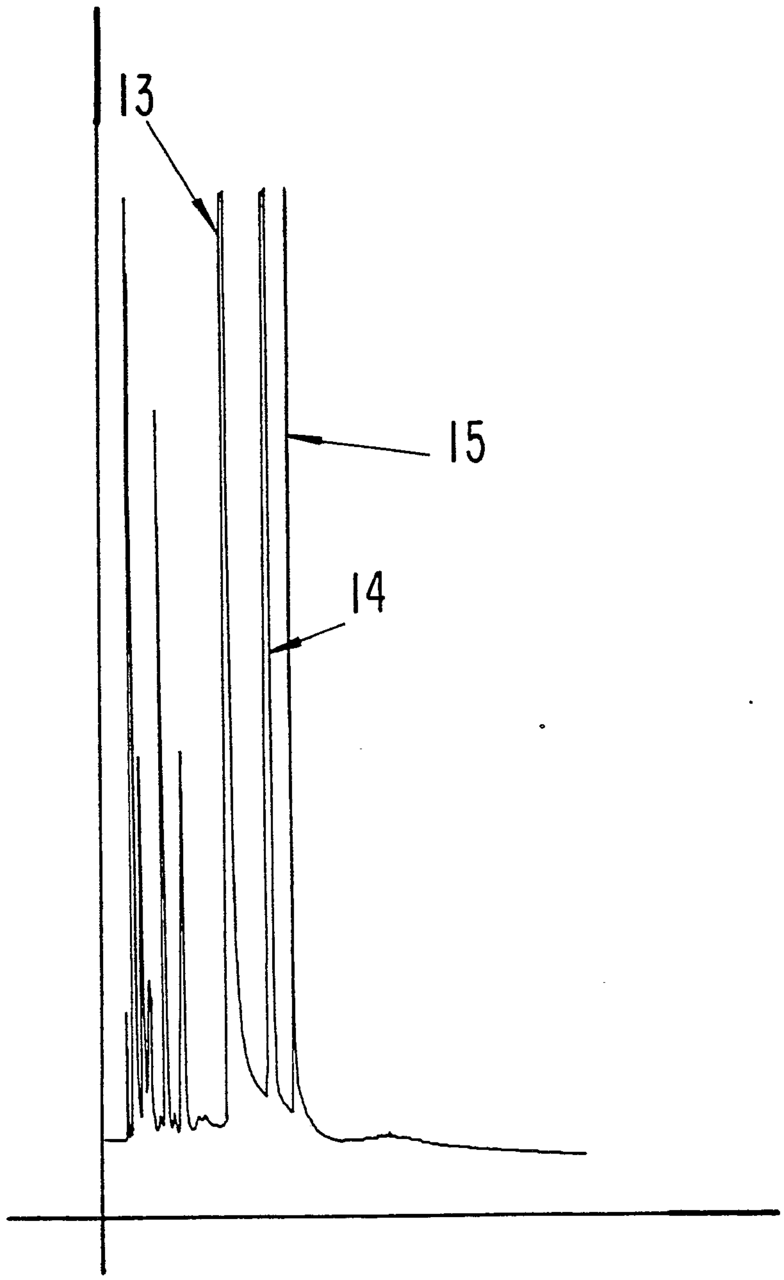

FIG.22A

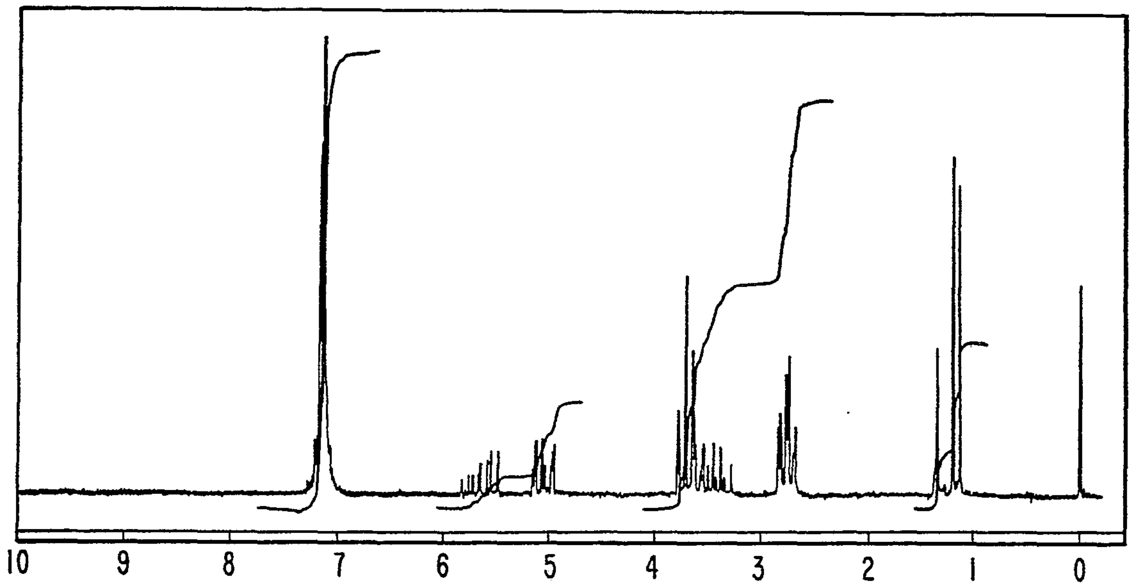


FIG.22B

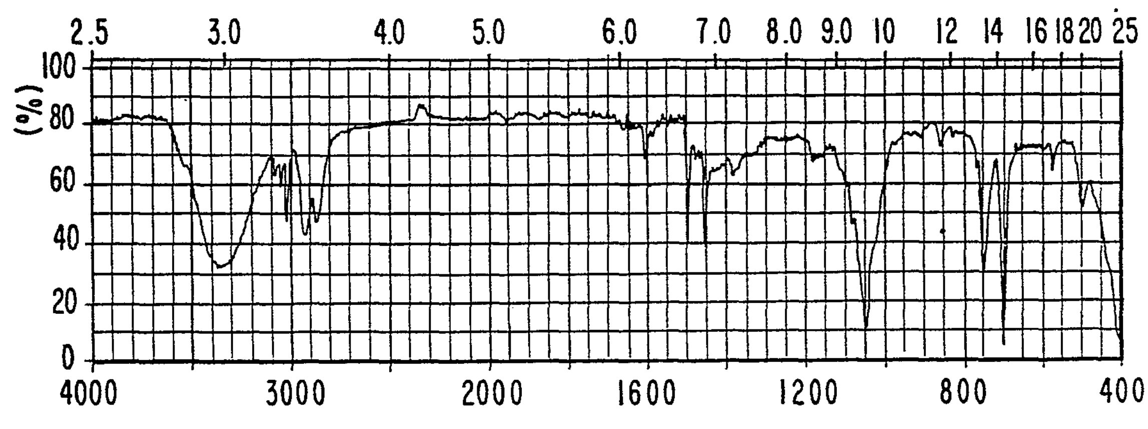

FIG 23A

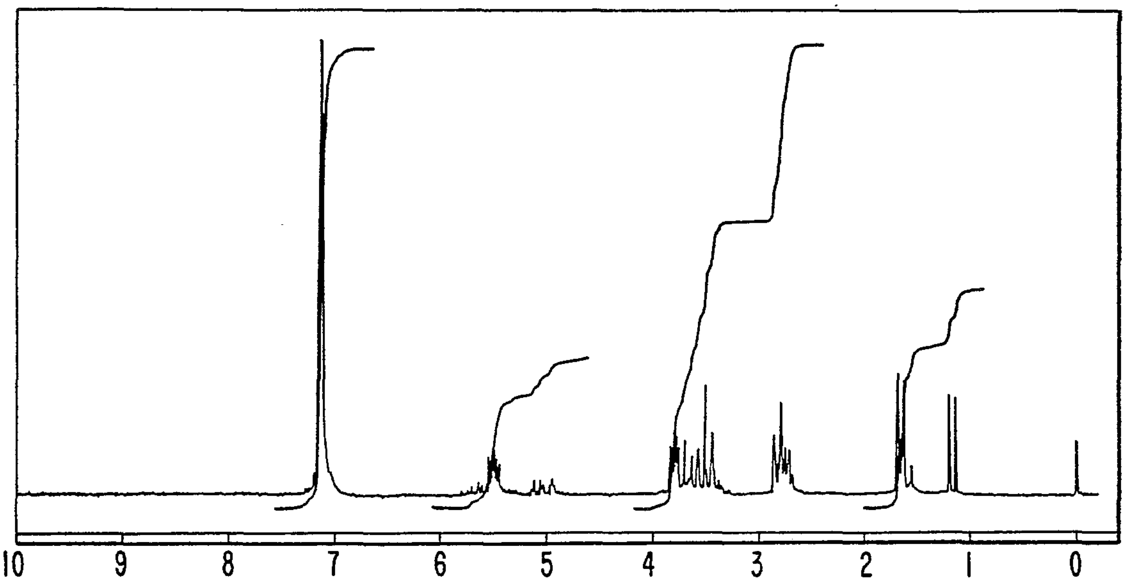


FIG. 23B